# EUROPEAN PATENT SPECIFICATION

(11) **EP 3 329 018 B1**
(45) Date of publication and mention of the grant of the patent: **31.03.2021**
(21) Application number: 16833574.3
(22) Date of filing: 28.07.2016
(51) Int. Cl.: C12Q 1/6883, C12Q 1/6886, A61K 39/395

(54) **IL-6 ANTAGONISTS FOR USE IN TREATING HEPCIDIN-MEDIATED DISORDERS**
IL-6 ANTAGONISTEN ZUR VERWENDUNG IN DER BEHANDLUNG VON HEPCIDIN-VERMITTELTEN KRANKHEITEN
ANTAGONISTES DE IL-6 POUR L'UTILISATION DANS LE TRAITEMENT DE TROUBLES MÉDIÉS PAR L'HEPCIDINE

(30) Priority: 31.07.2015 US 201562199434 P; 17.12.2015 US 201562268788 P
(43) Date of publication of application: 06.06.2018
(60) Divisional application: 21157575.8
(73) Proprietor: Medimmune Limited, Cambridge, Cambridgeshire CB21 6GH (GB)
(72) Inventor: KAKKAR, Rahul, Waltham, MA 02451 (US); DEVALARAJA, Madhav, N., Waltham, MA 02451 (US); ESCOTT, Katherine, Jane, Waltham, MA 02451 (US)
(74) Representative: J A Kemp LLP
(86) International application number: PCT/US2016/044528
(87) International publication number: WO 2017/023699

(56) References cited:
- US-A1- 2016 017 056
- MISATO HASHIZUME ET AL: "Tocilizumab, a humanized anti-interleukin-6 receptor antibody, improved anemia in monkey arthritis by suppressing IL-6-induced hepcidin production", RHEUMATOLOGY INTERNATIONAL ; CLINICAL AND EXPERIMENTAL INVESTIGATIONS, SPRINGER, BERLIN, DE, vol. 30, no. 7, 29 July 2009 (2009-07-29), pages 917-923, XP019801014, ISSN: 1437-160X
- E. FUNG ET AL: "Manipulation of the hepcidin pathway for therapeutic purposes", HAEMATOLOGICA, vol. 98, no. 11, 1 November 2013 (2013-11-01), pages 1667-1676, XP055170100, ISSN: 0390-6078, DOI: 10.3324/haematol.2013.084624
- SONG ET AL.: 'Down-regulation of hepcidin resulting from long-term treatment with an anti- IL -6 receptor · antibody (tocilizumab) improves anemia of inflammation in multicentric Castleman disease' BLOOD. vol. 116, no. 18, 2010, pages 3627 - 3634, XP055362309
- NAI ET AL.: 'TMPRSS6 rs855791 modulates hepcidin transcription in vitro and serum hepcidin levels in I normal individuals' BLOOD vol. 118, no. 16, 2011, pages 4459 - 4462, XP055362310

## Description

### 2. BACKGROUND

The peptide hormone, hepcidin, plays a central role in systemic iron homeostasis. Hentze et al., Cell 142:24-38 (2010). Hepcidin expression is known to be influenced by the product of the *TMPRSS6* gene, matriptase-2, a type II transmembrane serine protease. Common variants in the *TMPRSS6* gene have been shown to correlate with iron status, Benyamin et al., Nature Genetics 41(11):1173-1175 (2009), with the rs855791 SNP (2321G→A; A736V) having been shown to correlate with naturally occurring variations in hepcidin expression and blood hemoglobin levels.

Hepcidin expression has also been implicated in human iron disorders, Pietrangelo, J. Hepatology 54:173-181 (2011), and in anemias of chronic disease (ACD) (also known as anemia of inflammation (AI)). ACD is prevalent in patients with chronic infection, autoimmune disease, cancer, and chronic kidney disease (CKD). Sun et al., Am. J. Hematol. 87(4):392-400 (2012) Hashizume et al, Rheumatology International, Clinical and Experimental Investigations, 30(7): 917-923 (2009) discloses that Tocilizumab, a humanised anti-interleukin 6 antibody, improves anemia in monkey arthritis by suppressing IL-6 induced hepcidin production.

There is a need in the art for methods of treating hepcidin-mediated disorders.

### 3. SUMMARY

The scope of the invention is defined in the appended claims.

We have demonstrated that reducing IL-6 signaling provides clinical benefit in patients with a hepcidin-mediated disorder, including anemia of chronic disease and hepcidin-mediated cellular toxicities, but only in those patients having at least one copy of the *TMPRSS6* rs855791 major allele, with greatest effect in patients with elevated levels of IL-6.

In the following, references to aspects and embodiments concerns aspects and embodiments of the present disclosure which are not necessarily within the scope of the claimed invention. Accordingly, in a first aspect, methods of treating a hepcidin-mediated disorder are provided. The methods comprise administering a therapeutically effective amount of an IL-6 antagonist to a patient with a hepcidin-mediated disorder who has been determined to have at least one copy of the major allele at the *TMPRSS6* rs855791 SNP. In a first series of embodiments, the patient has previously been determined to have at least one copy of the *TMPRSS6* rs855791 major allele. In another series of embodiments, the method further comprises the earlier step of determining that the patient has at least one copy of the *TMPRSS6* rs855791 major allele. Typically, the patient has elevated pre-treatment serum levels of IL-6. In some embodiments, the patient has elevated pre-treatment serum levels of CRP.

In various embodiments, the hepcidin-mediated disorder is an anemia of chronic disease.

In some anemia embodiments, the patient is male and has a pre-treatment hemoglobin (Hb) level of less than 14 g/dl; pre-treatment Hb level of less than 13 g/dl; pre-treatment Hb level of less than 12 g/dl; or pre-treatment Hb level of less than 11 g/dl. In some anemia embodiments, the patient is female and has a pre-treatment Hb level of less than 12 g/dl; pre-treatment Hb level of less than 11 g/dl; pre-treatment Hb level of less than 10 g/dl; or pre-treatment Hb level of less than 9 g/dl.

In some anemia embodiments, the patient is male and has a pre-treatment hematocrit of less than 40%, less than 35%, or 30-34%. In some embodiments, the patient is female and has a pre-treatment hematocrit of less than 36%, less than 35%, less than 34%, less than 33%, less than 32%, or less than 31%. In some embodiments, the female patient has a pre-treatment hematocrit of 26 - 29%.

In various anemia embodiments, the patient has received at least one pre-treatment administration of an erythropoiesis stimulating agent (ESA). In certain embodiments, the patient has received at least one pre-treatment administration of an ESA and has a normal Hb level or normal hematocrit. In various embodiments, the patient has received at least one pre-treatment administration of an iron supplement. In certain embodiments, the patient has received at least one pre-treatment administration of an iron supplement and has a normal Hb level or normal hematocrit. In various embodiments, the patient has received at least one pre-treatment transfusion of blood or packed red blood cells. In certain embodiments, the patient has received at least one pre-treatment transfusion of blood or packed red blood cells and has a normal Hb level or normal hematocrit.

In a variety of anemia embodiments, the IL-6 antagonist is administered at a dose, on a schedule, and for a period sufficient to increase the patient's Hb levels above pre-treatment levels. In various embodiments, the IL-6 antagonist is administered at a dose, on a schedule, and for a period sufficient to increase the patient's hematocrit above pre-treatment levels. In some embodiments, the IL-6 antagonist is administered at a dose, on a schedule, and for a period sufficient to allow reduction in the patient's dose of ESA without reduction in the patient's Hb levels below levels present immediately pre-treatment. In certain embodiments, the IL-6 antagonist is administered at a dose, on a schedule, and for a period sufficient to allow reduction in the patient's dose of ESA without reduction in the patient's hematocrit below levels present immediately pre-treatment.

In various embodiments, the IL-6 antagonist is administered at a dose, on a schedule, and for a period sufficient to allow at least a 10% reduction in the patient's dose of ESA as compared to pre-treatment ESA dose, at least a 20% reduction in the patient's dose of ESA as compared to pre-treatment ESA dose, at least a 30% reduction in the patient's dose of ESA as compared to pre-treatment ESA dose, at least a 40% reduction in the patient's dose of ESA as compared to pre-treatment ESA dose, or at least a 50% reduction in the patient's dose of ESA as compared to pre-treatment ESA dose.

In some embodiments, the IL-6 antagonist is administered at a dose, on a schedule, and for a period sufficient to reverse functional iron deficiency.

In a series of embodiments, the hepcidin-mediated disorder is an anemia of chronic disease wherein the chronic disease is chronic kidney disease (CKD).

In some CKD embodiments, the patient has KDOQI stage 1 chronic kidney disease, KDOQI stage 2 chronic kidney disease, KDOQI stage 3 chronic kidney disease, KDOQI stage 4 chronic kidney disease, or KDOQI stage 5 chronic kidney disease. In particular embodiments, the patient has KDOQI stage 5 chronic kidney disease.

In some CKD embodiments, the patient has cardiorenal syndrome (CRS). In particular embodiments, the patient has CRS Type 4. In certain embodiments, the patient has received at least one pre-treatment dialysis treatment.

In some CKD embodiments, the IL-6 antagonist is administered at a dose, on a schedule, and for a period sufficient to reduce cardiovascular (CV) mortality as compared to age-matched and disease-matched historical controls.

In various embodiments, the hepcidin-mediated disorder is an anemia of chronic disease wherein the chronic disease is a chronic inflammatory disease.

In some embodiments, the chronic inflammatory disease is rheumatoid arthritis (RA). In certain embodiments, the patient has a pre-treatment DAS28 score of greater than 5.1. In some embodiments, the patient has a pre-treatment DAS28 score of 3.2 to 5.1. In particular embodiments, the patient has a pre-treatment DAS28 score of less than 2.6. In selected embodiments, the patient's pre-treatment RA is moderately active to severely active.

In some RA embodiments, the patient has received at least one pre-treatment administration of methotrexate. In some embodiments, the patient has received at least one pre-treatment administration of a TNFα antagonist. In select embodiments, the TNFα antagonist is selected from the group consisting of etanercept, adalimumab, infliximab, certolizumab, and golimumab.

In some RA embodiments, the patient has received at least one pre-treatment administration of an IL-6 antagonist. In certain embodiments, the pre-treatment IL-6 antagonist is tocilizumab or tofacitinib.

In a preferred series of embodiments, the treatment IL-6 antagonist is MEDI5117.

In various embodiments, the hepcidin-mediated disorder is an anemia of chronic disease wherein the chronic disease is selected from the group consisting of juvenile idiopathic arthritis, ankylosing spondylitis, plaque psoriasis, psoriatic arthritis, inflammatory bowel disease, Crohn's disease, and ulcerative colitis.

In some embodiments, the hepcidin-mediated disorder is an anemia of chronic disease wherein the chronic disease is cancer. In certain embodiments, the cancer is selected from the group consisting of: solid tumors, small cell lung cancer, non-small cell lung cancer, hematological cancer, multiple myeloma, leukemias, chronic lymphocytic leukemia (CLL), chronic myeloid leukemia (CML), lymphomas, Hodgkin's lymphoma and hepatic adenoma.

In some embodiments, the hepcidin-mediated disorder is an anemia of chronic disease wherein the chronic disease is the chronic disease is a chronic infection.

In some embodiments, the hepcidin-mediated disorder is an anemia of chronic disease wherein the chronic disease is the chronic disease is congestive heart failure (CHF).

In some embodiments, the hepcidin-mediated disorder is iron-refractory iron-deficiency anemia (IRIDA).

In some embodiments, the hepcidin-mediated disorder is acute coronary syndrome. In particular embodiments, the patient has suffered a myocardial infarction (MI) within the 60 days preceding the first administration of an IL-6 antagonist, the 30 days preceding the first administration of an IL-6 antagonist, within the 48 hours preceding the first administration of an IL-6 antagonist, or within the 24 hours preceding the first administration of an IL-6 antagonist.

In some acute coronary syndrome embodiments, the IL-6 antagonist is administered at a dose, on a schedule, and for a period sufficient to improve myocardial contractility as compared to pre-treatment levels. In some acute coronary syndrome embodiments, the IL-6 antagonist is administered at a dose, on a schedule, and for a period sufficient to improve cardiac ejection fraction as compared to pre-treatment levels. In some acute coronary syndrome embodiments, the IL-6 antagonist is administered at a dose, on a schedule, and for a period sufficient to reduce cardiac fibrosis as compared to pre-treatment levels.

In some embodiments, the hepcidin-mediated disorder is Castleman's Disease.

In another aspect, methods are provided for improving treatment of a hepcidin-mediated disorder. The method comprises discontinuing administration of an IL-6 antagonist to a patient with a hepcidin-mediated disorder, wherein the patient has been determined to be homozygous for the TMPRSS6 rs855791 minor allele.

In another aspect, methods are provided for improving treatment of a hepcidin-mediated disorder by discontinuing therapy that is ineffective, thereby reducing side effects and reducing cost without loss of treatment efficacy. The methods comprise discontinuing administration of an IL-6 antagonist to a patient with a hepcidin-mediated disorder, wherein the patient has been determined to be homozygous for the *TMPRSS6* rs855791 minor allele. In one series of embodiments, the patient has previously been determined to be homozygous for the *TMPRSS6* rs855791 minor allele. In another series of embodiments, the method further comprises the earlier step of determining that the patient is homozygous for the *TMPRSS6* rs855791 minor allele. In typical embodiments, the patient has elevated pre-treatment serum levels of IL-6. In various embodiments, the patient has elevated pre-treatment serum levels of CRP. In various embodiments, the patient has a hepcidin-mediated disorder selected from those described in Section 5.2.1 herein. In certain embodiments, the patient has anemia of chronic disease.

The data presented in Examples 2, 3 and 5 below demonstrate that IL-6 antagonists provide therapeutic benefit in subjects having elevated pre-treatment IL-6 levels and at least one copy of the *TMPRSS6* major allele, even in the absence of anemia. Accordingly, in another aspect, methods are provided for treating IL-6 mediated inflammatory disorders in patients without anemia of chronic inflammation. The methods comprise administering a therapeutically effective amount of an IL-6 antagonist to a subject, typically a human patient, who has an IL-6 mediated inflammatory disorder, wherein the patient does not have anemia, and wherein the subject has been determined to have at least one copy of the *TMPRSS6* rs855791 major allele. In a first series of embodiments, the subject has previously been determined to have at least one copy of the *TMPRSS6* rs855791 major allele. In another series of embodiments, the method further comprises the earlier step of determining that the subject has at least one copy of the *TMPRSS6* rs855791 major allele. Typically, the methods affirmatively exclude treatment of subjects who are homozygous for the *TMPRSS6* rs855791 minor allele. Typically, the patient has elevated pre-treatment serum levels of IL-6.

In particular embodiments of any of the treatment methods, the patient has elevated pre-treatment serum levels of IL-6. In certain embodiments, the patient has a pre-treatment serum IL-6 level of greater than 2.5 pg/ml, greater than 5 pg/ml, greater than 7.5 pg/ml, greater than 10 pg/ml, or greater than 12.5 pg/ml.

In various embodiments, the IL-6 antagonist is administered at a dose, on a schedule, and for a period sufficient to reduce the free IL-6 levels in the patient's serum below pre-treatment levels. In particular embodiments, the IL-6 antagonist is administered at a dose, on a schedule, and for a period sufficient to reduce the free IL-6 levels by at least 10% as compared to pre-treatment levels, by at least 20% as compared to pre-treatment levels, or by at least 50% as compared to pre-treatment levels.

In particular embodiments of any of the treatment methods, the patient has elevated pre-treatment levels of C-reactive protein (CRP). In certain embodiments, the patient has a pre-treatment CRP level greater than 2 mg/L, greater than 3 mg/L, greater than 5 mg/L, greater than 7.5 mg/L, or even greater than 10 mg/L.

In various embodiments, the IL-6 antagonist is administered at a dose, on a schedule, and for a period sufficient to reduce the patient's CRP levels below pre-treatment levels. In particular embodiments, the IL-6 antagonist is administered at a dose, on a schedule, and for a period sufficient to reduce the patient's CRP levels by at least 50% as compared to pre-treatment levels.

In particular embodiments of any of the treatment methods, the patient has been determined to have at least one copy of the TMPRSS6 rs855791 major allele using a TaqMan® real-time PCR assay.

In embodiments of any of the treatment methods, the IL-6 antagonist is an anti-IL-6 antibody, or antigen-binding fragment or derivative thereof.

In certain embodiments, the anti-IL-6 antibody or antigen-binding fragment or derivative has a K_{D} for binding human IL-6 of less than 100 nM, less than 50 nM, less than 10 nM, or less than 1 nM. In certain embodiments, the anti-IL-6 antibody or antigen-binding fragment or derivative has an elimination half-life following intravenous administration of at least 7 days, of at least 14 days, of at least 21 days, or at least 30 days.

In various antibody embodiments, the IL-6 antagonist is a full-length monoclonal anti-IL-6 antibody, such as an IgG1 or IgG4 antibody.

In select embodiments, the anti-IL-6 antibody or antigen-binding fragment or derivative is fully human. In some embodiments, the anti-IL-6 antibody or antigen-binding fragment or derivative is humanized.

In currently preferred embodiments, the anti-IL-6 antibody or antigen-binding fragment or derivative comprises all six variable region CDRs of MED5117. In some of these these embodiments, the antibody comprises the VH and VL of MED5117. And in particular embodiments, the antibody is MED5117.

In various embodiments, the anti-IL-6 antibody or antigen-binding fragment or derivative comprises all six variable region CDRs of an antibody selected from the group consisting of siltuximab, gerilimzumab, sirukumab, clazakizumab, olokizumab, elsilimomab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

In some embodiments, the anti-IL-6 antibody or antigen-binding fragment or derivative comprises the heavy chain V region and light chain V region from an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb). In particular embodiments, the anti-IL-6 antibody is an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

In some embodiments, the anti-IL-6 antibody or antigen-binding fragment or derivative is an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb). In particular embodiments, the anti-IL-6 antibody is an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

In various embodiments, the IL-6 antagonist is a single domain antibody, a VHH Nanobody, an Fab, or a scFv.

In a variety of embodiments, the IL-6 antagonist is an anti-IL-6R antibody, or antigen-binding fragment or derivative thereof In certain embodiments, the anti-IL-6R antibody, antigen-binding fragment, or derivative is tocilizumab or vobarilizumab.

In a variety of embodiments, the IL-6 antagonist is a JAK inhibitor. In particular embodiments, the JAK inhibitor is selected from the group consisting of tofacitinib (Xeljanz), decemotinib, ruxolitinib, upadacitinib, baricitinib, filgotinib, lestaurtinib, pacritinib, peficitinib, INCB-039110, ABT-494, INCB-047986 and AC-410.

In various embodiments, the IL-6 antagonist is a STAT3 inhibitor.

In some embodiments in which the IL-6 antagonist is an antibody or antigen-binding fragment or derivative, the IL-6 antagonist is administered parenterally. In particular embodiments, the IL-6 antagonist is administered subcutaneously.

In some embodiments in which the IL-6 antagonist is a JAK inhibitor or a STAT3 inhibitor, wherein the IL-6 antagonist is administered orally.

### 4. BRIEF DESCRIPTION OF THE DRAWINGS

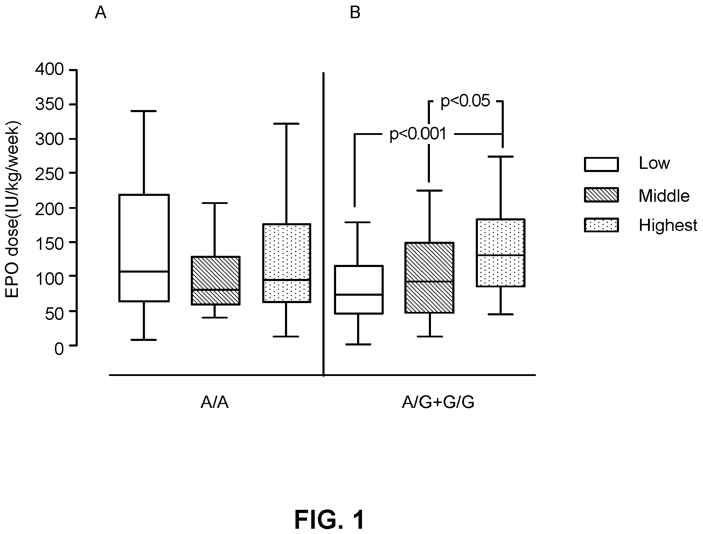

**FIGS. 1A** and **1B** provide boxplots showing that increased amounts of erythropoietin ("EPO") were required for treatment in chronic kidney disease patients (CKD stage 5 dialysis subjects) who had elevated levels of serum IL-6 and at least one copy of the major allele at a known SNP in the *TMPRSS6* gene, rs855791 (G or C at nucleotide position 2321, encoding a *TMPRSS6* polypeptide comprising an alanine at amino acid position 736; 736A), but not in chronic kidney disease patients who had elevated levels of IL-6 and were homozygous for the rs855791 *TMPRSS6* minor allele (T or A at nucleotide position 2321, encoding a *TMPRSS6* polypeptide having a valine at position 736; 736V). Data from patients homozygous for the minor allele (A/A) are shown in FIG. 1A; data from patients having at least one copy of the major allele (homozygous G/G and heterozygous G/A) were pooled and are shown in FIG. 1B. Each of the two patient populations was further stratified into groups based on tertiles of serum IL-6 level: "Low" tertile (IL-6 < 5 pg/ml); "Middle" tertile (IL-6 = 5-15 pg/ml); "Highest" tertile (IL-6 > 15pg/ml). Boxplots with error bars are overlaid over raw data. Each boxplot represents a patient group based on both IL-6 level and genotype. Details are provided in Example 1.

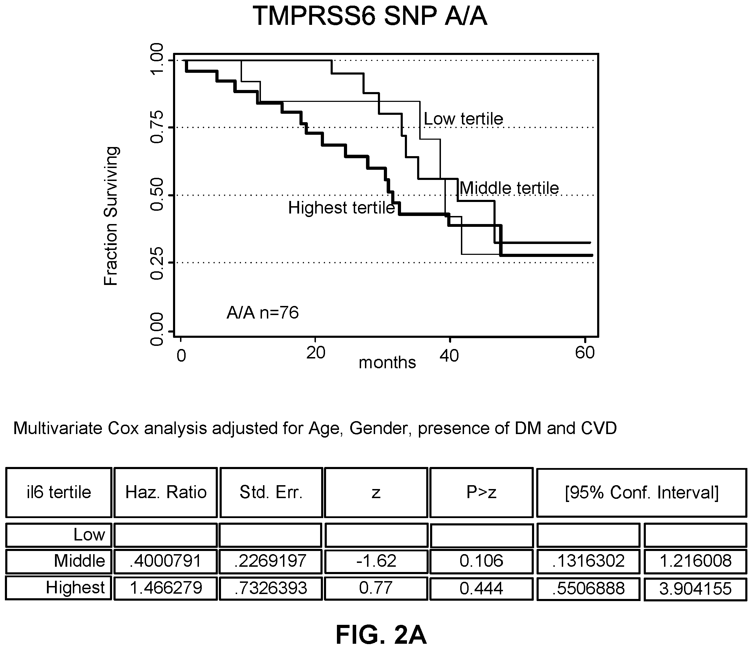

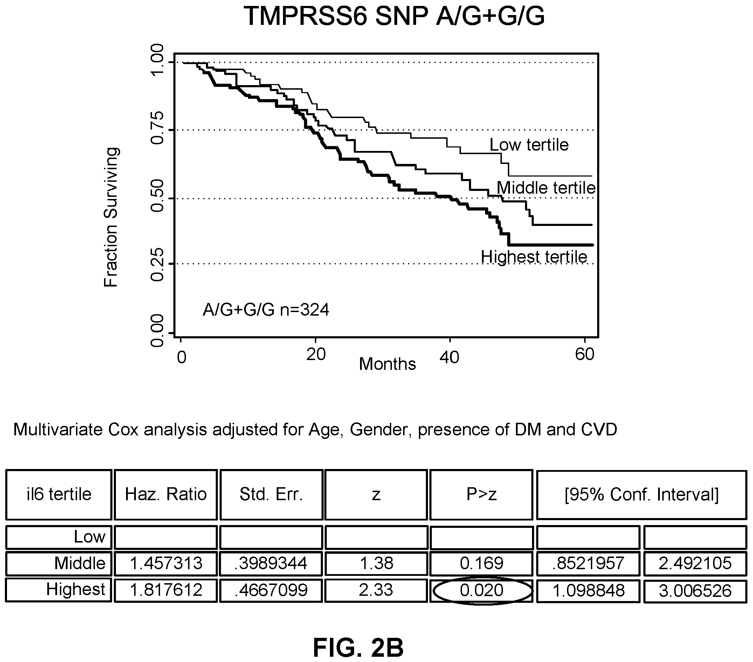

**FIGS. 2A** and **2B** provide survival curves that demonstrate that the *TMPRSS6* rs855791 major allele confers a higher all-cause mortality in response to elevated IL-6 levels in chronic kidney disease stage 5 dialysis subjects. FIG. 2A shows data from patients homozygous for the minor allele (A/A). FIG. 2B shows data from patients having at least one copy of the major allele (homozygous G/G and heterozygous G/A). Each group was separated into tertiles of serum IL-6 level using the IL-6 levels used in FIG. 1. Details are provided in Example 1.

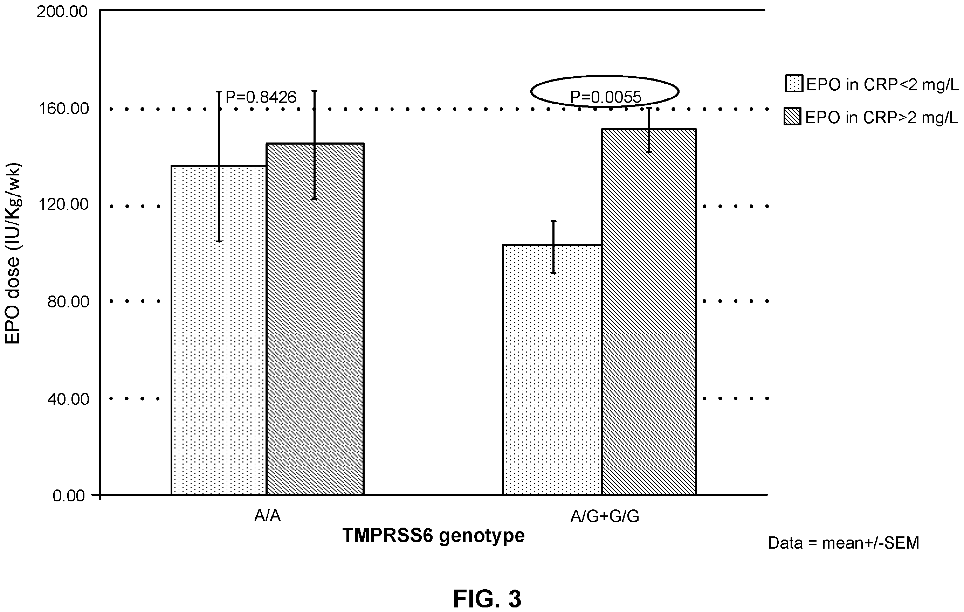

**FIG. 3** is a graph showing that increased amounts of EPO were required for therapy in chronic kidney disease patients (CKD stage 5 dialysis subjects) who had elevated serum levels of the acute phase reactant CRP and who had at least one copy of the *TMPRSS6* rs855791 major allele, but not in chronic kidney disease patients who had elevated serum levels of the acute phase reactant CRP and who were homozygous for the rs855791 minor allele. Each genotype group was separated into serum CRP levels < 2 mg/L vs >2 mg/L. Details are provided in Example 1.

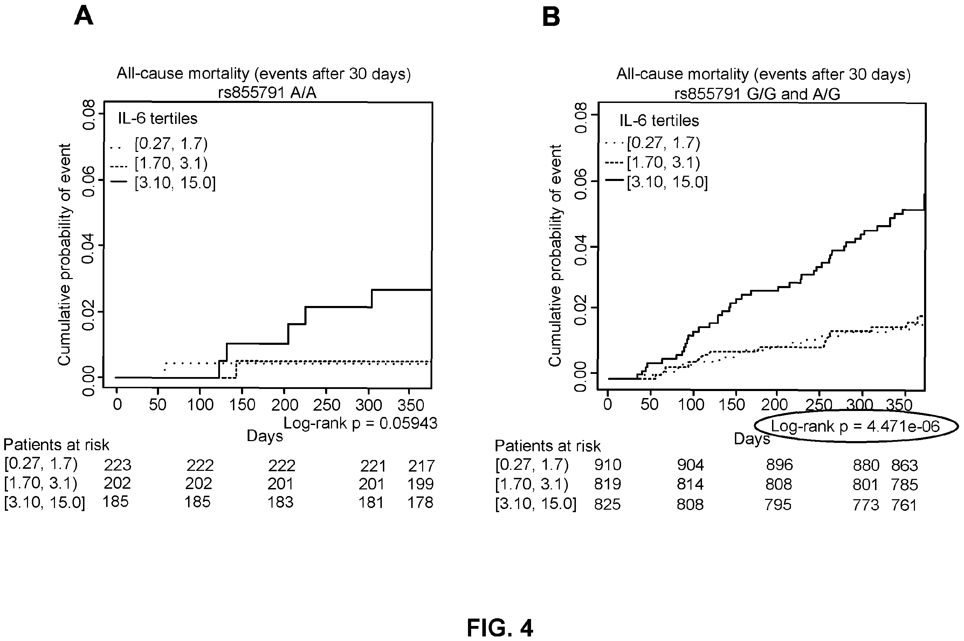

**FIGS. 4A** and **4B** provide graphs that demonstrate that the *TMPRSS6 rs855791* major allele confers higher all-cause mortality in response to elevated IL-6 levels in patients after myocardial infarction ("MI"). FIG. 4A plots the cumulative probability of mortality event over time (y-axis) versus days following MI (x-axis) for the population homozygous for the *TMPRSS6* rs855791 minor allele. FIG. 4B plots the cumulative probability of mortality event over time for the population having at least one copy of the *TMPRSS6* rs855791 major allele. Each group was separated into tertiles of serum IL-6 level as indicated. IL-6 level was measured one month after myocardial infarction. Mortality was measured one to twelve months following myocardial infarction. Details are provided in Example 2.

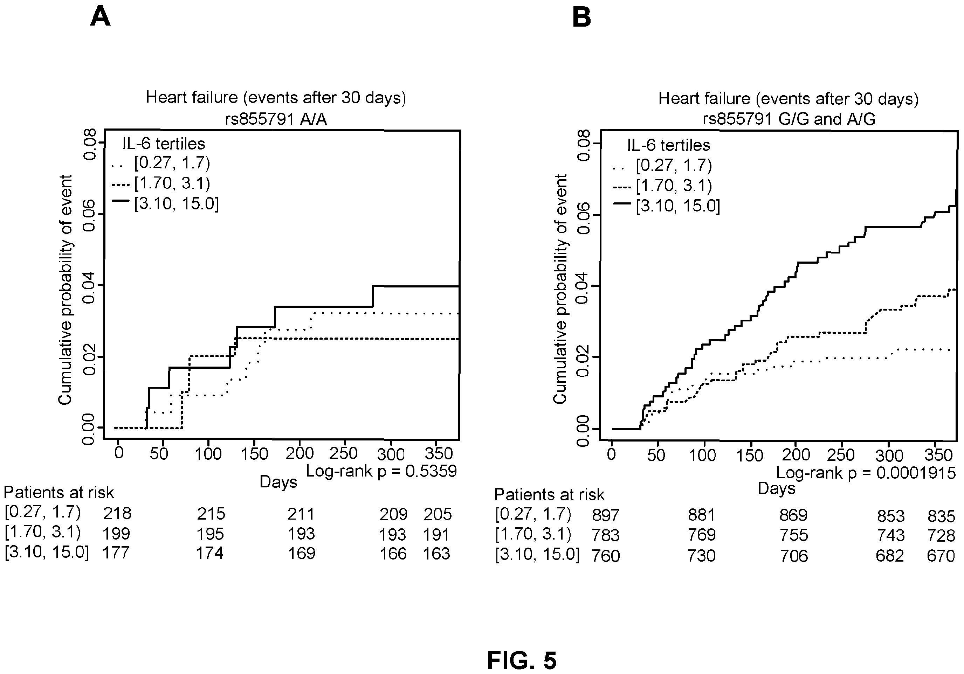

**FIGS. 5A** and **5B** provide graphs that demonstrate that the *TMPRSS6* rs855791 major allele confers higher risk of heart failure ("HF") in response to elevated IL-6 levels in patients after MI. FIG. 5A plots the cumulative probability of HF over time (y-axis) versus days following MI (x-axis) for the population homozygous for the *TMPRSS6* rs855791 minor allele. FIG. 5B plots the cumulative probability of HF event over time for the population having at least one copy of the TMRPSS6 rs855791 major allele. Each group was separated into tertiles of serum IL-6 level as indicated. IL-6 level was measured one month after myocardial infarction. HF was measured one to twelve months following myocardial infarction. Details are provided in Example 2.

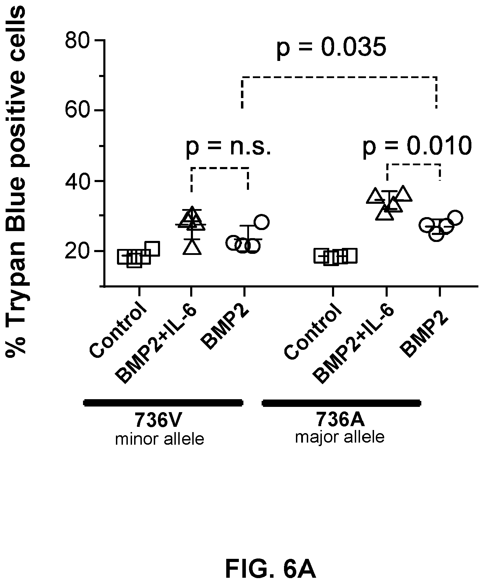

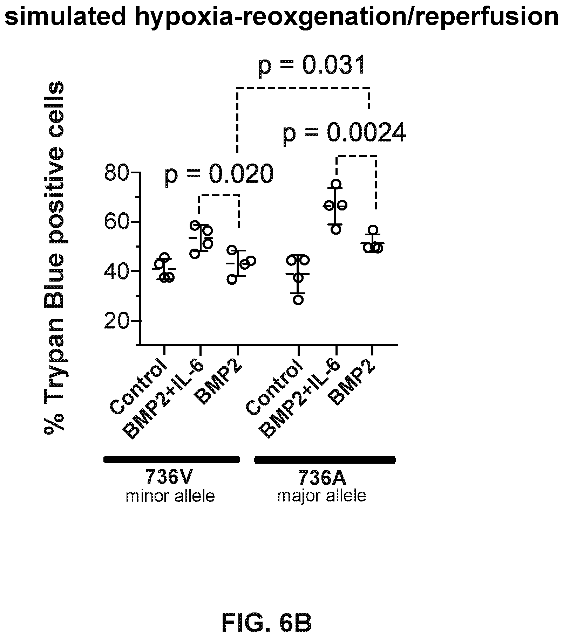

**FIGS. 6A** and **6B** show results from assays of human iPS cells that had been transfected with constructs constitutively expressing either the *TMPRSS6* rs855791 minor allele or major allele and differentiated into cardiomyocytes, upon exposure *in vitro* to BMP2+IL-6, or BMP2 alone, demonstrating that the *TMPRSS6* rs855791 major allele confers higher risk of cell death (Trypan Blue positive) in response to IL-6. FIG. 6A shows results in a normoxic environment. FIG. 6B shows results after exposure to hypoxic conditions and reoxygenation. The data imply that reducing IL-6 exposure should improve survival of cardiomyocytes in patients with the *TMPRSS6* rs855791 major allele, but not in patients with the *TMPRSS6* rs855791 minor allele. Details are provided in Example 3.

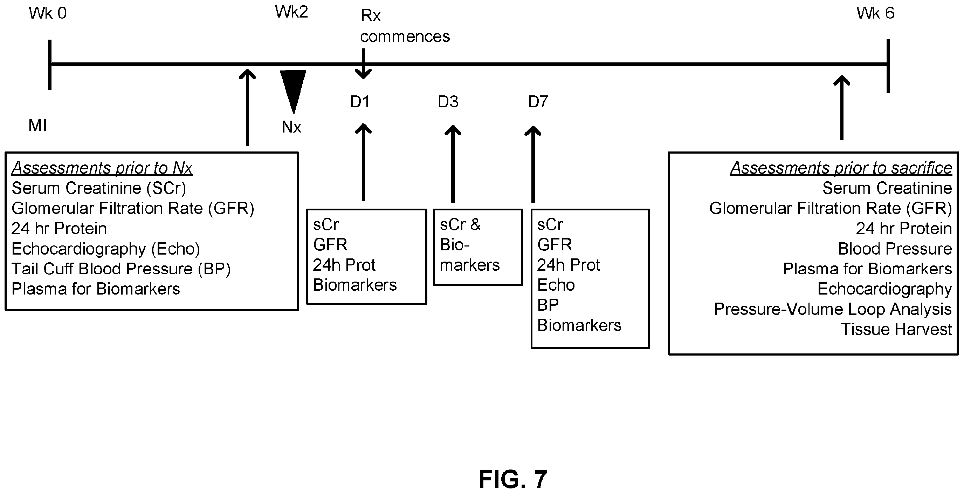

**FIG.** 7 is a diagram showing the experimental design of a cardiorenal syndrome study described in Example 4. CRS4 was induced in rats genotypically analogous to human beings homozygous for the *TMPRSS6* rs855791 major allele. The diagram shows various events in the study along a timeline. In the study, myocardial infarction ("MI") was induced in rats at week 0. At week 2, a single nephrectomy ("Nx") was performed in each subject. Anti-IL-6 antibody (ab9770, Abcam Plc, UK) (Rx) or isotype control antibody ("IgG"; ab171516, Abcam Plc, UK) was administered once every 3 days starting at 1 day (D1) after the nephrectomy until the end of the study. The standard of care therapy (ACE inhibitor - perindopril) was administered daily from 1 day after Nx until the end of the study. At week 6, the rodents were sacrificed. MI and Nx were not performed in the control group 'sham' subject group. Various assessments of the rodents were made at the time points indicated by arrows.

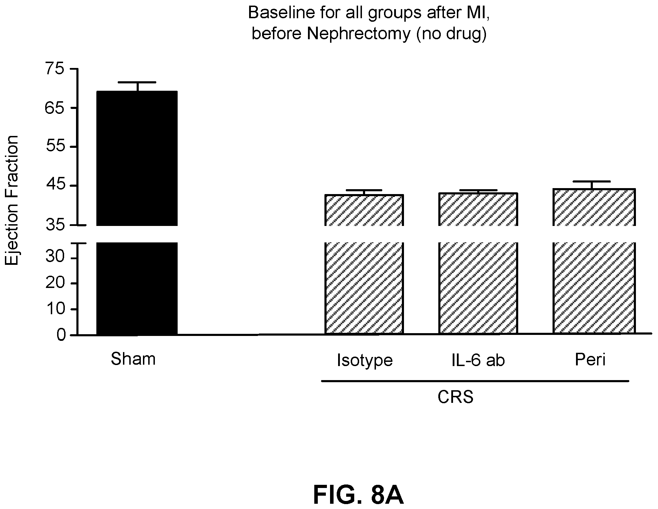

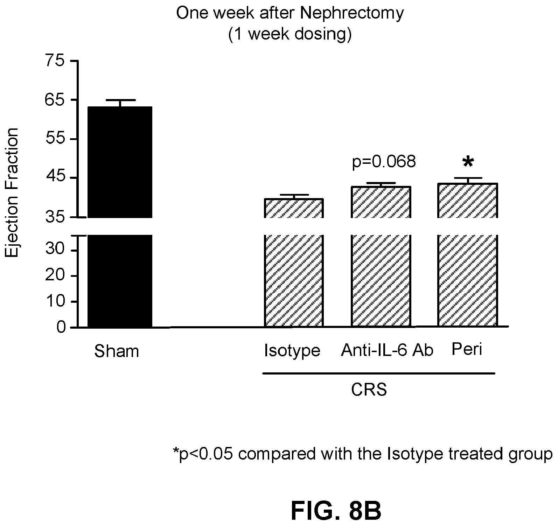

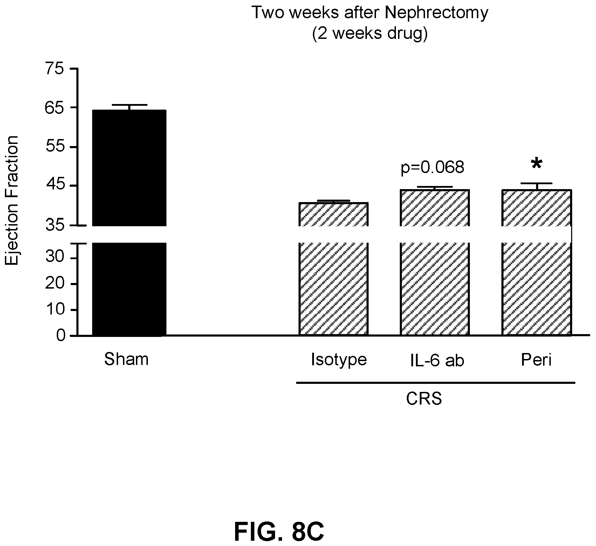

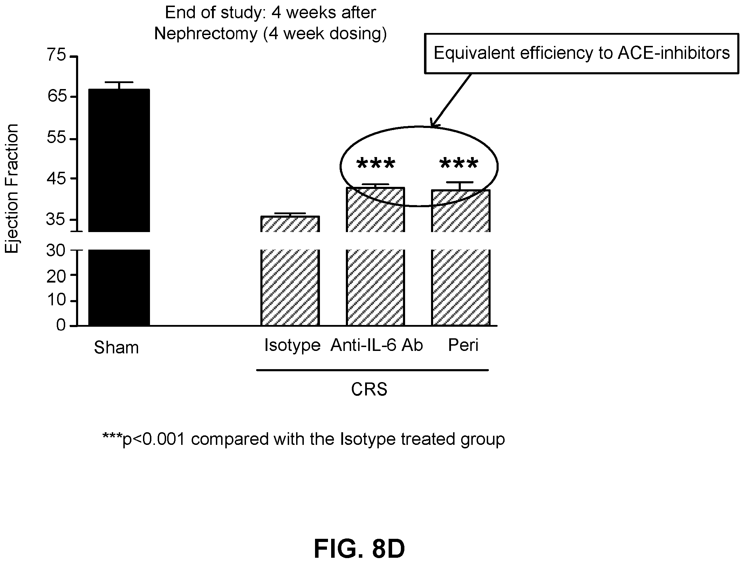

**FIGS. 8A-8D** shows the cardiac ejection fraction of rats treated with anti-IL-6 antibody ("IL-6 ab"), standard of care ACE inhibitor (perindopril or "Peri"), versus control ("isotype") treated group and sham operated animals in the cardiorenal syndrome model summarized in FIG. 7 and described in detail in Example 4. Figure 8A is a plot showing baseline ejection fraction levels for all groups two weeks after myocardial infarction, but before nephrectomy. Figure 8B is a plot showing ejection fraction levels for all groups one week after nephrectomy, after 1 week of treatment. Figure 8C is a plot showing ejection fraction levels for all groups two weeks after nephrectomy, after 2 weeks of treatment. FIG. 8D is a plot showing ejection fraction levels for all groups four weeks after nephrectomy, after 4 weeks of treatment. Results are expressed as mean +/-SEM, and demonstrate that anti-IL-6 therapy had therapeutic efficacy in the cardiorenal syndrome model equivalent to standard of care therapy, as measured by changes in cardiac ejection fraction.

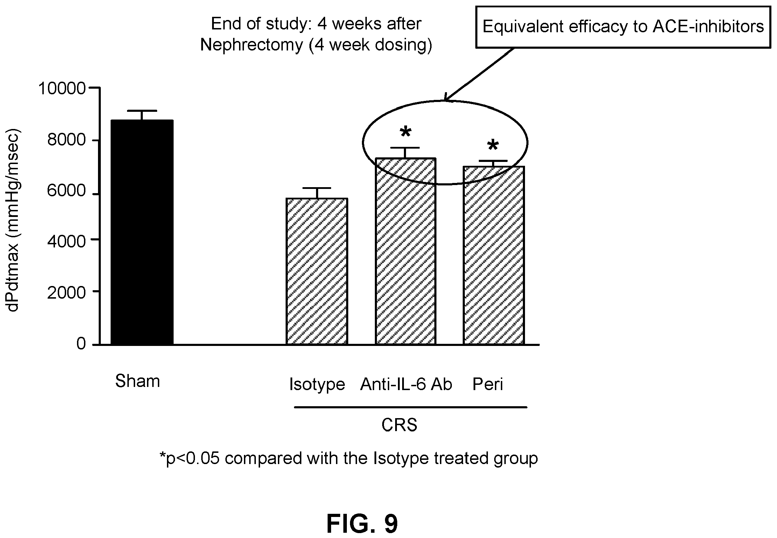

**FIG. 9** depicts a plot showing the cardiac contractility of rats treated with anti-IL-6 antibody ("IL-6 ab"), standard of care (perindopril or "Peri"), versus control ("isotype") treated group in the cardiorenal syndrome model summarized in FIG. 7 and described in detail in Example 4. Cardiac contractility was assessed at the end of the study by measuring dP/dtmax (mmHb/msec), which is a measure of pressure within the heart. Measurements are shown for all groups four weeks after nephrectomy, after 4 weeks of treatment. Results are expressed as mean +/- SEM, and demonstrate that anti-IL-6 therapy had therapeutic effect equivalent to standard of care therapy, as shown by increased cardiac contractility in rodent groups treated with anti-IL-6.

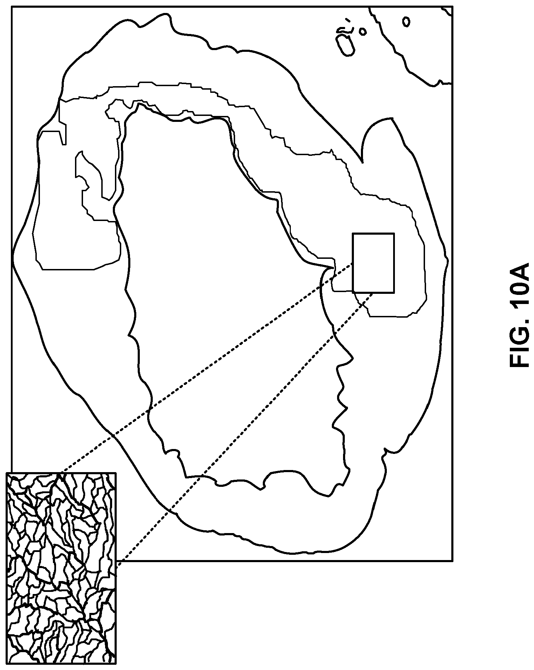

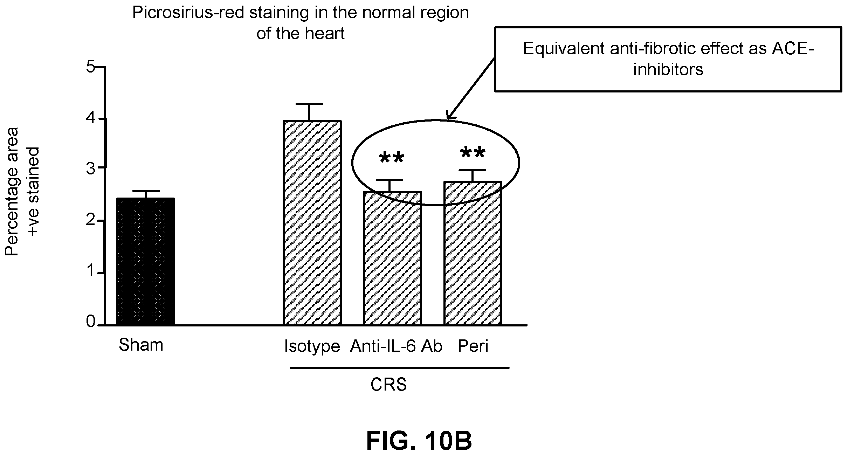

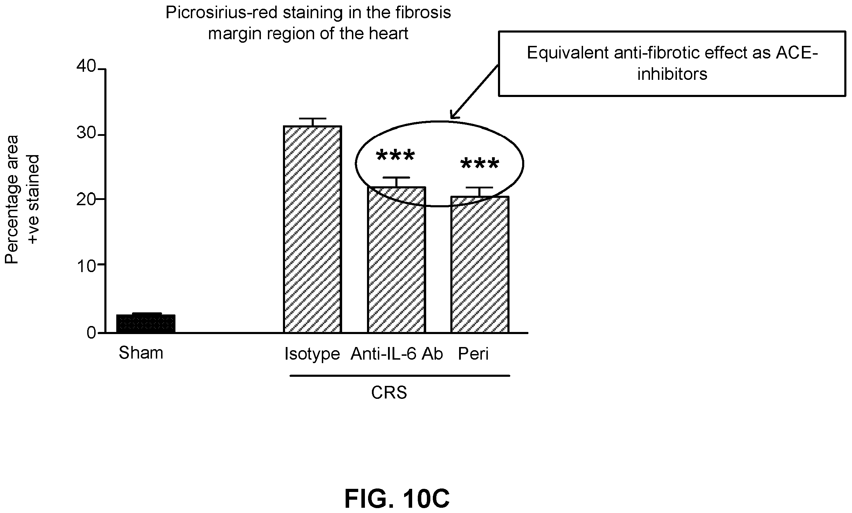

**FIGS. 10A** - **10C** show that anti-IL-6 therapy had an anti-cardiorenal syndrome effect equivalent to standard of care therapy, as measured by levels of fibrosis in heart tissue from rodent groups treated with anti-IL-6 ("IL-6 Ab"), standard of care (perindopril or "Peri"), and a control ("IgG"). Figure 10A is a micrograph showing a histological section of heart tissue stained with picrosirius-red. Two regions of the tissue were analyzed: a "Normal" region and a "Fibrosis Margin" region. An example "Normal" region is indicated by the delineated portion of the tissue slice. The inset in the micrograph shows a magnified view of the "Normal" region, showing that small portions of the "Normal" region has fibrotic tissue. The "Fibrosis Margin" region is a region of tissue in the "Normal" region peripheral to the fibrotic tissue. Figure 10B is a plot showing percentages of the area of the "Normal" region indicated as fibrotic tissue (i.e., stained/dark regions) in tissue samples from all groups. Figure 10C is a plot showing percentages of the area of the "Fibrosis Margin" region indicated as fibrotic tissue in tissue samples from all groups. Results are expressed as mean +/-SEM. Details are provided in Example 4.

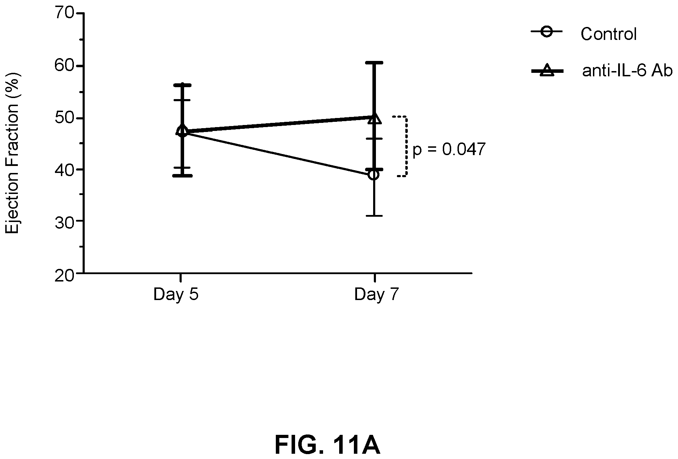

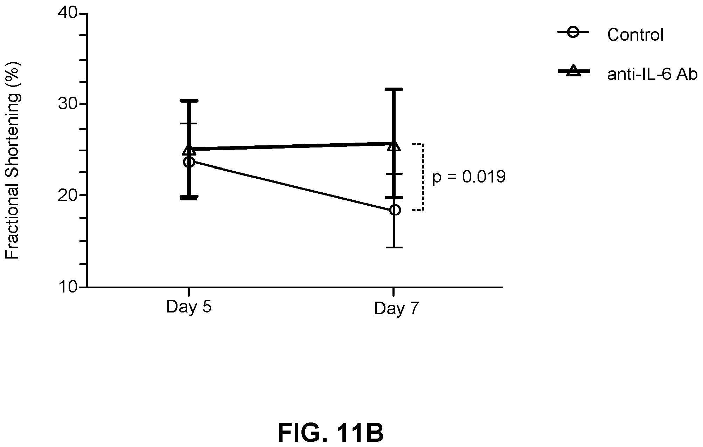

**FIGS. 11A** **and** **11B** show data from an *in vivo* model in which myocardial infarction was induced in mice genotypically analogous to human beings homozygous for the *TMPRSS6* rs855791 major allele. The control group received no therapy. The experimental group were treated with an anti-murine-IL-6 antibody. FIG. 11A shows that treatment with anti-IL-6 provide statistically significant improvement in ejection fraction. FIG. 11B shows that treatment with anti-IL-6 provides statistically significant improvement in contractility, measured as cardiac left ventricular fractional shortening. The data demonstrate that anti-IL-6 therapy given immediately after myocardial infarction improves functional recovery of the left ventricle in rodents that mimic human patients having the *TMPRSS6* rs855791 major allele. Details are provided in Example 5.

The figures depict various embodiments of the present invention for purposes of illustration only. One skilled in the art will readily recognize from the following discussion that alternative embodiments of the structures and methods illustrated herein may be employed.

### 5. DETAILED DESCRIPTION

### 5.1. Overview of experimental results

The peptide hormone, hepcidin, plays a central role in systemic iron homeostasis. Hentze et al., Cell 142:24-38 (2010). Hepcidin expression is known to be influenced by the product of the *TMPRSS6* gene, matriptase-2, a type II transmembrane serine protease. Common variants in the *TMPRSS6* gene have been shown to correlate with iron status, Benyamin et al., Nature Genetics 41(11):1173-1175 (2009), with the rs855791 SNP (2321G→A; A736V) having been shown to correlate with naturally occurring variations in hepcidin expression and blood hemoglobin levels. Hepcidin expression has also been implicated in human iron disorders, Pietrangelo, J. Hepatology 54:173-181 (2011), and in anemias of chronic disease (ACD) (also known as anemia of inflammation (AI)). ACD is prevalent in patients with chronic infection, autoimmune disease, cancer, and chronic kidney disease (CKD). Sun et al., Am. J. Hematol. 87(4):392-400 (2012).

To determine whether the genotype at the *TMPRSS6* rs855791 SNP predicts extent of anemia in end stage renal disease, data previously collected in clinical studies of patients with chronic kidney disease were analyzed in conjunction with newly determined SNP genotyping. Because hepcidin expression is also regulated by IL-6, Casanovas et al., PLOS Computational Biol. 10(1):e1003421 (2014), the data were further analyzed to determine whether serum IL-6 levels could predict extent of anemia in end stage renal disease.

As described in Example 1 and shown in FIG. 1, the extent of underlying anemia - measured as the clinically-titrated EPO dose - correlated with IL-6 levels only in patients having at least one copy of the major allele at the *TMPRSS6* rs855791 SNP. In these patients, higher serum IL-6 levels correlated with higher required EPO dose (FIG. 1B). In contrast, the degree of anemia in patients having two copies of the minor allele was not correlated with serum IL-6 levels (FIG. 1A).

Analogously, overall survival correlated with IL-6 levels only in patients with at least one copy of the major allele at the *TMPRSS6* SNP rs855791. In subjects having at least one copy of the *TMPRSS6* rs855791 major allele, survival was inversely correlated with serum IL-6 level, with patients in the highest tertile of serum IL-6 levels having statistically significantly worse survival than those in the lowest tertile of IL-6 levels (FIG. 2B). In contrast, the overall survival of patients homozygous for the minor allele at rs855791 was unaffected by IL-6 levels (FIG. 2A).

Without intending to be bound by theory, in patients having at least one copy of the *TMPRSS6* major allele, increases in serum IL-6 may drive increased hepcidin expression, thereby increasing anemia. The increased mortality risk is a consequence of the dysregulated iron metabolism, the resulting anemia, and/or the increased dose of erythropoiesis stimulating agent, such as EPO, administered for treatment. These correlations raise the possibility that reducing IL-6 levels or IL-6 signaling could reduce anemia, reduce required EPO dose, and increase survival in patients with chronic kidney disease, but only in those patients having at least one copy of the *TMPRSS6* rs855791 major allele, and with greatest effect in those patients having elevated serum levels of IL-6.

To determine whether *TMPRSS6* rs855791 genotype affected IL-6 sensitivity in patients with acute, rather than chronic, disease, in Example 2 we analyzed data previously collected in clinical studies of patients hospitalized for acute coronary syndrome in conjunction with newly determined SNP genotyping.

The mortality of subjects homozygous for the *TMPRSS6* rs855791 SNP minor allele (A) did not correlate with variations in IL-6 (FIG. 4A). However, one or two copies of the major allele (G) conferred a higher all-cause mortality in response to elevated IL-6 levels in subjects following myocardial infarction (FIG. 4B). Thus, *TMPRSS6* modulated IL-6 mediated risk of death following myocardial infarction.

The effect of *TMPRSS6* genotype on IL-6 mediated risk of heart failure was also assessed. Heart failure in subjects homozygous for the minor allele (A) did not correlate with variations in IL-6 (FIG. 5A). However, the G allele of *TMPRSS6* conferred a higher heart failure rate in response to elevated IL-6 levels in subjects following a myocardial infarction (FIG. 5B). Thus, *TMPRSS6* modulated IL-6 mediated risk of heart failure following myocardial infarction.

The data from Example 2 demonstrate that the correlation between *TMPRSS6* genotype, IL-6 levels, and adverse clinical outcomes is not limited to patients with chronic kidney disease. Without intending to be bound by theory, in patients having at least one copy of the *TMPRSS6* major allele, increases in serum IL-6 may drive increased hepcidin expression, with consequent increased sequestration of iron in cardiomyocytes, followed by iron-mediated cellular toxicity. These correlations raise the possibility that reducing IL-6 levels or IL-6 signaling could reduce heart failure and mortality in patients with acute coronary syndrome, but only in those patients having at least one copy of the *TMPRSS6* rs855791 major allele, and with greatest effect in those patients with elevated serum levels of IL-6.

Although the correlations observed in Examples 1 and 2 strongly suggest that reducing IL-6 mediated signaling should provide clinical benefit in patients having at least one copy of the *TMPRSS6* rs855791 major allele, elevated levels of IL-6, and either anemia or a hepcidin-mediated cellular toxicity, the observed correlations fall short of proving a causal relationship. Accordingly, in Example 3, human induced pluripotent stem (iPS) cell cardiomyocytes were engineered to express only the *TMPRSS6* rs855791 major allele or minor allele, and tested *in vitro.*

Hepcidin expression is regulated by both the BMP6/SMAD and IL-6/STAT signaling pathways, with both BMP and IL-6 acting through their respective receptors to drive increased hepcidin expression. Casanovas et al., PLOS Comp. Biol. 10(1):e1003421 (2014). The major allele and minor allele iPS cardiomyocytes were treated *in vitro* with agonists of both signaling pathways - recombinant BMP2 and IL-6 - or with BMP2 alone to model clinical interventions in which IL-6 levels (or signaling) are reduced. Control iPS cells were not treated with either agonist. Cell mortality was measured under normal oxygen tension (normoxia), and also under conditions that simulate hypoxia followed by reoxygenation (reperfusion).

FIG. 6A shows the results when the cells were treated under normal oxygen levels. iPS cardiomyocytes expressing only the *TMPRSS6* rs855791 minor allele ("736V minor allele") are not significantly affected ("n.s.") by elimination of IL-6 signaling: cell mortality measured as percent Trypan Blue positive cells not significantly reduced when the cells are treated with BMP2 alone as compared to treatment with BMP2+IL-6. In contrast, iPS cardiomyocytes expressing the *TMPRSS6* rs855791 major allele show statistically significantly lower cell death when IL-6 signaling is eliminated.

FIG. 6B shows the results when the cells were subjected to hypoxia followed by reoxygenation. As compared to normoxic conditions, hypoxia/reoxygenation is toxic to the iPS cardiomyocytes, with about 40 percent of major and minor allele control cells killed, as compared to about 20% of the control cells killed under normoxic conditions (compare to FIG. 6A). Against this increased background toxicity, minor allele iPS cardiomyocytes are not significantly affected by elimination of IL-6 signaling: cell mortality is not significantly reduced when the cells are treated with BMP2 alone, as compared to treatment with BMP2+IL-6. In contrast, the iPS cardiomyocytes expressing the *TMPRSS6* rs855791 major allele show statistically significantly lower cell death when IL-6 signaling is eliminated.

These data strengthen the inferences drawn from the post hoc analysis of clinical trial data in Example 1 and Example 2: reduction in IL-6 signaling is effective to reduce IL-6 mediated toxicity in cardiomyocytes expressing the *TMPRSS6* rs855791 major allele, but not in cardiomyocytes expressing only the minor allele. Without intending to be bound by theory, the IL-6 driven increase in toxicity in the major allele iPS cardiomyocytes may result from IL-6 mediated increase in hepcidin expression, with consequent increased sequestration of iron in the cells, followed by iron-mediated cellular toxicity.

Patients with chronic kidney disease, such as those enrolled in the MIMICK studies analyzed in Example 1, often develop impaired cardiac function, which is a major contributor to overall mortality. This secondary cardiac injury following primary chronic kidney disease is termed cardiorenal syndrome type 4 (CRS type 4). To test directly whether anti-IL-6 therapy would be effective as a treatment in CRS4 patients having at least one copy of the *TMPRSS6* rs855791 major allele, as suggested by the data in Examples 1 and 3, we used a model of CRS4 in rats that are genotypically analogous to human beings homozygous for the *TMPRSS6* rs855791 major allele.

After 4 weeks of treatment, both of the treatment groups - the group treated with an anti-IL-6 antibody and the group treated with the standard of care ACE inhibitor therapy, perindopril - showed statistically significantly increased ejection fraction levels compared to the isotype control group (FIG. 8D) (p<0.001). Similar ejection fraction levels in the anti-IL-6 and standard of care groups measured after week 4 of treatment showed that anti-IL-6 therapy had equivalent efficacy to the ACE inhibitor. FIG. 9 shows that anti-IL-6 therapy was also equally effective as an ACE inhibitor in preserving cardiac contractility. FIGS. 10A - 10C demonstrate that anti-IL-6 therapy was equally effective in reducing cardiac fibrosis.

These data demonstrate that treatment with an anti-IL-6 agent is effective to reduce cardiac injury and restore function in an *in vivo* model of cardiorenal syndrome in animals that are genotypically analogous to human beings homozygous for the *TMPRSS6* rs855791 major allele.

Analogously, the data in Examples 2 and 3 suggested that reducing IL-6 levels or IL-6 signaling could reduce heart failure and mortality in patients with acute coronary syndrome, but only in those patients having at least one copy of the *TMPRSS6* rs855791 major allele, and with greatest effect in those patients with elevated serum levels of IL-6.

A study was performed to determine the effect of anti-IL-6 therapy after acute myocardial infarction in mice that are genotypically analogous to human beings homozygous for the *TMPRSS6* rs855791 major allele.

FIGS. 11A and 11B show data from an *in vivo* model in which myocardial infarction was induced in mice genotypically analogous to human beings homozygous for the *TMPRSS6* rs855791 major allele. The control group received no therapy. The experimental group was treated with an anti-murine-IL-6 antibody. FIG. 11A shows that treatment with anti-IL-6 provides statistically significant improvement in ejection fraction as compared to controls. FIG. 11B shows that treatment with anti-IL-6 provides statistically significant improvement in contractility, measured as cardiac fractional shortening, as compared to controls. These data demonstrate that anti-IL-6 therapy given immediately after myocardial infarction improves functional recovery of the left ventricle in rodents that are genotypically analogous to human patients having the *TMPRSS6* rs855791 major allele.

Collectively, the experimental data demonstrate that therapeutic interventions that reduce IL-6 signaling will provide clinical benefit in patients with a hepcidin-mediated disorder, such as anemia or a hepcidin-mediated cellular toxicity, but only in those who have at least one copy of the *TMPRSS6* rs855791 major allele, with greatest effect in patients with elevated levels of IL-6.

Accordingly, as further described below, in a first aspect methods of treating a hepcidin-mediated disorder are provided. The methods comprise administering a therapeutically effective amount of an IL-6 antagonist to a patient with a hepcidin-mediated disorder who has been determined to have at least one copy of the major allele at the *TMPRSS6* rs855791 SNP. In a second aspect, methods are provided for improving treatment of hepcidin-mediated disorders, the method comprising discontinuing administration of an IL-6 antagonist to a patient with a hepcidin-mediated disorder, wherein the patient has been determined to be homozygous for the *TMPRSS6* rs855791 minor allele. Treatment is improved by discontinuing therapy that is ineffective, thereby reducing side effects and reducing cost without loss of treatment efficacy. In a further aspect, methods are provided for treating IL-6 mediated inflammatory disorders in patients without anemia of chronic inflammation, the methods comprising administering a therapeutically effective amount of an IL-6 antagonist to a patient who has an IL-6 mediated inflammatory disorder, does not have anemia, and the subject has been determined to have at least one copy of the *TMPRSS6* rs855791 major allele.

### 5.2. Definitions

Unless defined otherwise, all technical and scientific terms used herein have the meaning commonly understood by a person skilled in the art to which this invention belongs. As used herein, the following terms have the meanings ascribed to them below.

By "**hepcidin**" is meant a polypeptide having at least about 85% or greater amino acid identity to the amino acid sequence provided at NCBI Accession No. NP_066998 ("hepcidin preprotein"), or biologically active fragment thereof. Exemplary hepcidin biological activities include binding and reducing the levels of the iron export channel ferroportin, inhibiting iron transport, inhibiting intestinal iron absorption, and inhibiting iron release from macrophages and the liver. An exemplary hepcidin preprotein amino acid sequence is provided below: 
1 MALSSQIWAA CLLLLLLLAS LTSGSVFPQQ TGQLAELQPQ DRAGARASWM PMFQRRRRRD
61 THFPICIFCC GCCHRSKCGM CCKT (SEQ ID NO:1)
 With reference to the sequence above, hepcidin exists in various forms, including as a preprohormone (amino acids 25-84), prohormone (amino acids 25-84), and mature forms termed hepcidin-25 (amino acids 60-84), hepcidin-22 (amino acids 63-84), and hepcidin-20 (amino acids 65-84).

A "**hepcidin-mediated disorder**" is any disorder in which hepcidin expression contributes to the etiology of the disorder or any of its symptoms. The contribution of hepcidin to the etiology may be known, may be suspected, or may inferred from an observation that administration of an IL-6 antagonist provides greater therapeutic benefit in patients with the disorder who have at least one copy of the *TMPRSS6* rs855791 SNP maj or allele as compared to patients with the disorder who are homozygous for the *TMPRSS6* rs855791 SNP minor allele. Hepcidin-mediated disorders are further described below in Section 5.2.1.

By "**transmembrane protease serine 6 (*TMPRSS6*) polypeptide**" is meant a polypeptide or fragment thereof having at least about 85% or greater amino acid identity to the amino acid sequence provided at NCBI Accession No. NP_001275929 and having serine proteinase activity. The *TMPRSS6* polypeptide, also known as Matriptase-2 (MT2), cleaves hemojuvelin and inhibits bone morphogenetic protein signaling. An exemplary *TMPRSS6* amino acid sequence having an alanine at position 736 (736A) is provided below: 
1 MPVAEAPQVA GGQGDGGDGE EAEPEGMFKA CEDSKRKARG YLRLVPLFVL LALLVLASAG
61 VLLWYFLGYK AEVMVSQVYS GSLRVLNRHF SQDLTRRESS AFRSETAKAQ KMLKELITST
121 RLGTYYNSSS VYSFGEGPLT CFFWFILQIP EHRRLMLSPE VVQALLVEEL LSTVNSSAAV
181 PYRAEYEVDP EGLVILEASV KDIAALNSTL GCYRYSYVGQ GQVLRLKGPD HLASSCLWHL
241 QGPKDLMLKL RLEWTLAECR DRLAMYDVAG PLEKRLITSV YGCSRQEPVV EVLASGAIMA
301 VVWKKGLHSY YDPFVLSVQP VVFQACEVNL TLDNRLDSQG VLSTPYFPSY YSPQTHCSWH
361 LTVPSLDYGL ALWFDAYALR RQKYDLPCTQ GQWTIQNRRL CGLRILQPYA ERIPVVATAG
421 ITINFTSQIS LTGPGVRVHY GLYNQSDPCP GEFLCSVNGL CVPACDGVKD CPNGLDERNC
481 VCRATFQCKE DSTCISLPKV CDGQPDCLNG SDEEQCQEGV PCGTFTFQCE DRSCVKKPNP
541 QCDGRPDCRD GSDEEHCDCG LQGPSSRIVG GAVSSEGEWP WQASLQVRGR HICGGALIAD
601 RWVITAAHCF QEDSMASTVL WTVFLGKVWQ NSRWPGEVSF KVSRLLLHPY HEEDSHDYDV
661 ALLQLDHPVV RSAAVRPVCL PARSHFFEPG LHCWITGWGA LREGALRADA VALFYGWRNQ
721 GSETCCCPIS NALQKADVQL IPQDLCSEVY RYQVTPRMLC AGYRKGKKDA CQGDSGGPLV
781 CKALSGRWFL AGLVSWGLGC GRPNYFGVYT RITGVISWIQ QVVT (SEQ ID NO:2)
 An exemplary *TMPRSS6* amino acid sequence having a valine at position 736 (736V) is provided below:

1 

61 
121 
181 
24 
30 
36 
42 
48 
54 
60 
66 
72 
78  (SEQ ID NO:3)

By *"****TMPRSS6* nucleic acid molecule"** is meant a polynucleotide encoding an *TMPRSS6* polypeptide (Matriptase-2; MT2). An exemplary *TMPRSS6* nucleic acid molecule sequence is provided at NCBI Accession No. NM_001289000. A *TMPRSS6* nucleic acid sequence having a G at nucleotide position 2321 ("G allele"; "major allele") is provided below: 
1 GGACAAACAG AGGCTCCTGA GGCCTGTGTG CAGGCCCGGC ACCTATCTGC CGCTCCCAAA
61 GGATGCCCGT GGCCGAGGCC CCCCAGGTGG CTGGCGGGCA GGGGGACGGA GGTGATGGCG
121 AGGAAGCGGA GCCGGAGGGG ATGTTCAAGG CCTGTGAGGA CTCCAAGAGA AAAGCCCGGG
181 GCTACCTCCG CCTGGTGCCC CTGTTTGTGC TGCTGGCCCT GCTCGTGCTG GCTTCGGCGG
241 GGGTGCTACT CTGGTATTTC CTAGGGTACA AGGCGGAGGT GATGGTCAGC CAGGTGTACT
301 CAGGCAGTCT GCGTGTACTC AATCGCCACT TCTCCCAGGA TCTTACCCGC CGGGAATCTA
361 GTGCCTTCCG CAGTGAAACC GCCAAAGCCC AGAAGATGCT CAAGGAGCTC ATCACCAGCA
421 CCCGCCTGGG AACTTACTAC AACTCCAGCT CCGTCTATTC CTTTGGGGAG GGACCCCTCA
481 CCTGCTTCTT CTGGTTCATT CTCCAAATCC CCGAGCACCG CCGGCTGATG CTGAGCCCCG
541 AGGTGGTGCA GGCACTGCTG GTGGAGGAGC TGCTGTCCAC AGTCAACAGC TCGGCTGCCG
601 TCCCCTACAG GGCCGAGTAC GAAGTGGACC CCGAGGGCCT AGTGATCCTG GAAGCCAGTG
661 TGAAAGACAT AGCTGCATTG AATTCCACGC TGGGTTGTTA CCGCTACAGC TACGTGGGCC
721 AGGGCCAGGT CCTCCGGCTG AAGGGGCCTG ACCACCTGGC CTCCAGCTGC CTGTGGCACC
781 TGCAGGGCCC CAAGGACCTC ATGCTCAAAC TCCGGCTGGA GTGGACGCTG GCAGAGTGCC
841 GGGACCGACT GGCCATGTAT GACGTGGCCG GGCCCCTGGA GAAGAGGCTC ATCACCTCGG
901 TGTACGGCTG CAGCCGCCAG GAGCCCGTGG TGGAGGTTCT GGCGTCGGGG GCCATCATGG
961 CGGTCGTCTG GAAGAAGGGC CTGCACAGCT ACTACGACCC CTTCGTGCTC TCCGTGCAGC
1021 CGGTGGTCTT CCAGGCCTGT GAAGTGAACC TGACGCTGGA CAACAGGCTC GACTCCCAGG
1081 GCGTCCTCAG CACCCCGTAC TTCCCCAGCT ACTACTCGCC CCAAACCCAC TGCTCCTGGC
1141 ACCTCACGGT GCCCTCTCTG GACTACGGCT TGGCCCTCTG GTTTGATGCC TATGCACTGA
1201 GGAGGCAGAA GTATGATTTG CCGTGCACCC AGGGCCAGTG GACGATCCAG AACAGGAGGC
1261 TGTGTGGCTT GCGCATCCTG CAGCCCTACG CCGAGAGGAT CCCCGTGGTG GCCACGGCCG
 
1321 GGATCACCAT CAACTTCACC TCCCAGATCT CCCTCACCGG GCCCGGTGTG CGGGTGCACT
1381 ATGGCTTGTA CAACCAGTCG GACCCCTGCC CTGGAGAGTT CCTCTGTTCT GTGAATGGAC
1441 TCTGTGTCCC TGCCTGTGAT GGGGTCAAGG ACTGCCCCAA CGGCCTGGAT GAGAGAAACT
1501 GCGTTTGCAG AGCCACATTC CAGTGCAAAG AGGACAGCAC ATGCATCTCA CTGCCCAAGG
1561 TCTGTGATGG GCAGCCTGAT TGTCTCAACG GCAGCGACGA AGAGCAGTGC CAGGAAGGGG
1621 TGCCATGTGG GACATTCACC TTCCAGTGTG AGGACCGGAG CTGCGTGAAG AAGCCCAACC
1681 CGCAGTGTGA TGGGCGGCCC GACTGCAGGG ACGGCTCGGA TGAGGAGCAC TGTGACTGTG
1741 GCCTCCAGGG CCCCTCCAGC CGCATTGTTG GTGGAGCTGT GTCCTCCGAG GGTGAGTGGC
1801 CATGGCAGGC CAGCCTCCAG GTTCGGGGTC GACACATCTG TGGGGGGGCC CTCATCGCTG
1861 ACCGCTGGGT GATAACAGCT GCCCACTGCT TCCAGGAGGA CAGCATGGCC TCCACGGTGC
1921 TGTGGACCGT GTTCCTGGGC AAGGTGTGGC AGAACTCGCG CTGGCCTGGA GAGGTGTCCT
1981 TCAAGGTGAG CCGCCTGCTC CTGCACCCGT ACCACGAAGA GGACAGCCAT GACTACGACG
2041 TGGCGCTGCT GCAGCTCGAC CACCCGGTGG TGCGCTCGGC CGCCGTGCGC CCCGTCTGCC
2101 TGCCCGCGCG CTCCCACTTC TTCGAGCCCG GCCTGCACTG CTGGATTACG GGCTGGGGCG
2161 CCTTGCGCGA GGGCGCCCTA CGGGCGGATG CTGTGGCCCT ATTTTATGGA TGGAGAAACC
2221 AAGGCTCAGA GACATGTTGC TGCCCCATCA GCAACGCTCT GCAGAAAGTG GATGTGCAGT
2281 TGATCCCACA GGACCTGTGC AGCGAGGTCT ATCGCTACCA GGTGACGCCA CGCATGCTGT
2341 GTGCCGGCTA CCGCAAGGGC AAGAAGGATG CCTGTCAGGG TGACTCAGGT GGTCCGCTGG
2401 TGTGCAAGGC ACTCAGTGGC CGCTGGTTCC TGGCGGGGCT GGTCAGCTGG GGCCTGGGCT
2461 GTGGCCGGCC TAACTACTTC GGCGTCTACA CCCGCATCAC AGGTGTGATC AGCTGGATCC
2521 AGCAAGTGGT GACCTGAGGA ACTGCCCCCC TGCAAAGCAG GGCCCACCTC CTGGACTCAG
2581 AGAGCCCAGG GCAACTGCCA AGCAGGGGGA CAAGTATTCT GGCGGGGGGT GGGGGAGAGA
2641 GCAGGCCCTG TGGTGGCAGG AGGTGGCATC TTGTCTCGTC CCTGATGTCT GCTCCAGTGA
2701 TGGCAGGAGG ATGGAGAAGT GCCAGCAGCT GGGGGTCAAG ACGTCCCCTG AGGACCCAGG
2761 CCCACACCCA GCCCTTCTGC CTCCCAATTC TCTCTCCTCC GTCCCCTTCC TCCACTGCTG
2821 CCTAATGCAA GGCAGTGGCT CAGCAGCAAG AATGCTGGTT CTACATCCCG AGGAGTGTCT
2881 GAGGTGCGCC CCACTCTGTA CAGAGGCTGT TTGGGCAGCC TTGCCTCCAG AGAGCAGATT
2941 CCAGCTTCGG AAGCCCCTGG TCTAACTTGG GATCTGGGAA TGGAAGGTGC TCCCATCGGA
3001 GGGGACCCTC AGAGCCCTGG AGACTGCCAG GTGGGCCTGC TGCCACTGTA AGCCAAAAGG
3061 TGGGGAAGTC CTGACTCCAG GGTCCTTGCC CCACCCCTGC CTGCCACCTG GGCCCTCACA
3121 GCCCAGACCC TCACTGGGAG GTGAGCTCAG CTGCCCTTTG GAATAAAGCT GCCTGATCCA
3181 AAAAAAAAAA AAAAAA (SEQ ID NO:4)
 A *TMPRSS6* nucleic acid sequence having an A at nucleotide position 2321 is provided below:

1 GGACAAACAG AGGCTCCTGA GGCCTGTGTG CAGGCCCGGC ACCTATCTGC CGCTCCCAAA
61 GGATGCCCGT GGCCGAGGCC CCCCAGGTGG CTGGCGGGCA GGGGGACGGA GGTGATGGCG
121 AGGAAGCGGA GCCGGAGGGG ATGTTCAAGG CCTGTGAGGA CTCCAAGAGA AAAGCCCGGG
181 GCTACCTCCG CCTGGTGCCC CTGTTTGTGC TGCTGGCCCT GCTCGTGCTG GCTTCGGCGG
241 GGGTGCTACT CTGGTATTTC CTAGGGTACA AGGCGGAGGT GATGGTCAGC CAGGTGTACT
301 CAGGCAGTCT GCGTGTACTC AATCGCCACT TCTCCCAGGA TCTTACCCGC CGGGAATCTA
361 GTGCCTTCCG CAGTGAAACC GCCAAAGCCC AGAAGATGCT CAAGGAGCTC ATCACCAGCA

421 CCCGCCTGGG AACTTACTAC AACTCCAGCT CCGTCTATTC CTTTGGGGAG GGACCCCTCA
481 CCTGCTTCTT CTGGTTCATT CTCCAAATCC CCGAGCACCG CCGGCTGATG CTGAGCCCCG
541 AGGTGGTGCA GGCACTGCTG GTGGAGGAGC TGCTGTCCAC AGTCAACAGC TCGGCTGCCG
601 TCCCCTACAG GGCCGAGTAC GAAGTGGACC CCGAGGGCCT AGTGATCCTG GAAGCCAGTG
661 TGAAAGACAT AGCTGCATTG AATTCCACGC TGGGTTGTTA CCGCTACAGC TACGTGGGCC
721 AGGGCCAGGT CCTCCGGCTG AAGGGGCCTG ACCACCTGGC CTCCAGCTGC CTGTGGCACC
781 TGCAGGGCCC CAAGGACCTC ATGCTCAAAC TCCGGCTGGA GTGGACGCTG GCAGAGTGCC
841 GGGACCGACT GGCCATGTAT GACGTGGCCG GGCCCCTGGA GAAGAGGCTC ATCACCTCGG
901 TGTACGGCTG CAGCCGCCAG GAGCCCGTGG TGGAGGTTCT GGCGTCGGGG GCCATCATGG
961 CGGTCGTCTG GAAGAAGGGC CTGCACAGCT ACTACGACCC CTTCGTGCTC TCCGTGCAGC
1021 CGGTGGTCTT CCAGGCCTGT GAAGTGAACC TGACGCTGGA CAACAGGCTC GACTCCCAGG
1081 GCGTCCTCAG CACCCCGTAC TTCCCCAGCT ACTACTCGCC CCAAACCCAC TGCTCCTGGC
1141 ACCTCACGGT GCCCTCTCTG GACTACGGCT TGGCCCTCTG GTTTGATGCC TATGCACTGA
1201 GGAGGCAGAA GTATGATTTG CCGTGCACCC AGGGCCAGTG GACGATCCAG AACAGGAGGC
1261 TGTGTGGCTT GCGCATCCTG CAGCCCTACG CCGAGAGGAT CCCCGTGGTG GCCACGGCCG
1321 GGATCACCAT CAACTTCACC TCCCAGATCT CCCTCACCGG GCCCGGTGTG CGGGTGCACT
1381 ATGGCTTGTA CAACCAGTCG GACCCCTGCC CTGGAGAGTT CCTCTGTTCT GTGAATGGAC
1441 TCTGTGTCCC TGCCTGTGAT GGGGTCAAGG ACTGCCCCAA CGGCCTGGAT GAGAGAAACT
1501 GCGTTTGCAG AGCCACATTC CAGTGCAAAG AGGACAGCAC ATGCATCTCA CTGCCCAAGG
1561 TCTGTGATGG GCAGCCTGAT TGTCTCAACG GCAGCGACGA AGAGCAGTGC CAGGAAGGGG
1621 TGCCATGTGG GACATTCACC TTCCAGTGTG AGGACCGGAG CTGCGTGAAG AAGCCCAACC
1681 CGCAGTGTGA TGGGCGGCCC GACTGCAGGG ACGGCTCGGA TGAGGAGCAC TGTGACTGTG
1741 GCCTCCAGGG CCCCTCCAGC CGCATTGTTG GTGGAGCTGT GTCCTCCGAG GGTGAGTGGC
1801 CATGGCAGGC CAGCCTCCAG GTTCGGGGTC GACACATCTG TGGGGGGGCC CTCATCGCTG
1861 ACCGCTGGGT GATAACAGCT GCCCACTGCT TCCAGGAGGA CAGCATGGCC TCCACGGTGC
1921 TGTGGACCGT GTTCCTGGGC AAGGTGTGGC AGAACTCGCG CTGGCCTGGA GAGGTGTCCT
1981 TCAAGGTGAG CCGCCTGCTC CTGCACCCGT ACCACGAAGA GGACAGCCAT GACTACGACG
2041 TGGCGCTGCT GCAGCTCGAC CACCCGGTGG TGCGCTCGGC CGCCGTGCGC CCCGTCTGCC
2101 TGCCCGCGCG CTCCCACTTC TTCGAGCCCG GCCTGCACTG CTGGATTACG GGCTGGGGCG
2161 CCTTGCGCGA GGGCGCCCTA CGGGCGGATG CTGTGGCCCT ATTTTATGGA TGGAGAAACC
2221 AAGGCTCAGA GACATGTTGC TGCCCCATCA GCAACGCTCT GCAGAAAGTG GATGTGCAGT
2281 TGATCCCACA GGACCTGTGC AGCGAGGTCT ATCGCTACCA AGTGACGCCA CGCATGCTGT
2341 GTGCCGGCTA CCGCAAGGGC AAGAAGGATG CCTGTCAGGG TGACTCAGGT GGTCCGCTGG
2401 TGTGCAAGGC ACTCAGTGGC CGCTGGTTCC TGGCGGGGCT GGTCAGCTGG GGCCTGGGCT
2461 GTGGCCGGCC TAACTACTTC GGCGTCTACA CCCGCATCAC AGGTGTGATC AGCTGGATCC
2521 AGCAAGTGGT GACCTGAGGA ACTGCCCCCC TGCAAAGCAG GGCCCACCTC CTGGACTCAG
2581 AGAGCCCAGG GCAACTGCCA AGCAGGGGGA CAAGTATTCT GGCGGGGGGT GGGGGAGAGA
2641 GCAGGCCCTG TGGTGGCAGG AGGTGGCATC TTGTCTCGTC CCTGATGTCT GCTCCAGTGA
2701 TGGCAGGAGG ATGGAGAAGT GCCAGCAGCT GGGGGTCAAG ACGTCCCCTG AGGACCCAGG
2761 CCCACACCCA GCCCTTCTGC CTCCCAATTC TCTCTCCTCC GTCCCCTTCC TCCACTGCTG
2821 CCTAATGCAA GGCAGTGGCT CAGCAGCAAG AATGCTGGTT CTACATCCCG AGGAGTGTCT
2881 GAGGTGCGCC CCACTCTGTA CAGAGGCTGT TTGGGCAGCC TTGCCTCCAG AGAGCAGATT
2941 CCAGCTTCGG AAGCCCCTGG TCTAACTTGG GATCTGGGAA TGGAAGGTGC TCCCATCGGA

3001 GGGGACCCTC AGAGCCCTGG AGACTGCCAG GTGGGCCTGC TGCCACTGTA AGCCAAAAGG
3061 TGGGGAAGTC CTGACTCCAG GGTCCTTGCC CCACCCCTGC CTGCCACCTG GGCCCTCACA
3121 GCCCAGACCC TCACTGGGAG GTGAGCTCAG CTGCCCTTTG GAATAAAGCT GCCTGATCCA
3181 AAAAAAAAAA AAAAAA (SEQ ID NO:5)

By "**variant**" is meant a polynucleotide or polypeptide sequence that differs from a reference sequence by one or more nucleotides or one or more amino acids. An exemplary *TMPRSS6* variant is *TMPRSS6* (A736V), resulting from SNP rs855791 (G→A).

By "**single nucleotide polymorphism**" or "**SNP**" is meant a naturally occurring DNA sequence variant in which a single nucleotide in the genome differs between members of a biological species or between paired chromosomes in an individual. SNPs can be used as genetic markers for variant alleles. In one embodiment, the *TMPRSS6* SNP is rs855791.

By "**rs855791**" is meant a single nucleotide polymorphism (SNP) in the human *TMPRSS6* gene, 2321G→A, resulting in an alanine to valine substitution (A736V) in the catalytic domain of Matriptase-2 (MT2), which is encoded by the *TMPRSS6* gene. The allele with highest frequency in the human population (the major allele) is 2321G, encoding 736A. The allele with lowest frequency in the human population (minor allele) is 2321A, encoding 736V.

By "**heterozygous**" is meant that a chromosomal locus has two different alleles. In one embodiment of the methods described herein, heterozygous refers to a genotype in which one allele has a *TMPRSS6* nucleic acid sequence encoding a *TMPRSS6* polypeptide having an alanine at amino acid position 736 (e.g., having a G or C at nucleotide position 2321 of a *TMPRSS6* nucleic acid molecule) (rs855791 major allele), and the other allele has a variant *TMPRSS6* nucleic acid sequence encoding a *TMPRSS6* polypeptide comprising a valine at amino acid position 736 (e.g., having an A or T at nucleotide position 2321 of a *TMPRSS6* nucleic acid molecule) (rs855791 minor allele).

By "**homozygous**" is meant that a chromosomal locus has two identical alleles. In certain embodiments of the methods described herein, homozygous refers to a genotype in which both alleles have a *TMPRSS6* nucleic acid sequence encoding a *TMPRSS6* polypeptide comprising an alanine at amino acid position 736 (e.g., having a G or C at nucleotide position 2321 of a *TMPRSS6* nucleic acid molecule) (rs855791 homozygous major allele). In some embodiments, homozygous refers to a genotype in which both alleles have a *TMPRSS6* nucleic acid sequence encoding a *TMPRSS6* polypeptide comprising a valine at amino acid position 736 (e.g., having an A or T at nucleotide position 2321 of a *TMPRSS6* nucleic acid molecule) (rs855791 homozygous minor allele).

**"Determining that a patient has at least one copy of the *TMPRSS6* rs855791 major allele**" includes, but is not limited to, performing an assay to determine that a patient has at least one copy of the TMPRSS6 rs855791 major allele; ordering an assay to determine that a patient has at least one copy of the TMPRSS6 rs855791 major allele; prescribing an assay to determine that a patient has at least one copy of the TMPRSS6 rs855791 major allele; otherwise directing or controlling that an assay be performed to determine that a patient has at least one copy of the TMPRSS6 rs855791 major allele; and reviewing *TMRSS6* genotype assay data or protein or nucleic acid sequence data to determine that a patient has at least one copy of the TMPRSS6 rs855791 major allele.

By "**interleukin 6 (IL-6)**" or "**IL**-**6 polypeptide**" is meant a polypeptide or fragment thereof having at least about 85% or greater amino acid identity to the amino acid sequence provided at NCBI Accession No. NP_000591 and having IL-6 biological activity. IL-6 is a pleotropic cytokine with multiple biologic functions. Exemplary IL-6 biological activities include immunostimulatory and pro-inflammatory activities. An exemplary IL-6 amino acid sequence is provided below:

1 MCVGARRLGR GPCAALLLLG LGLSTVTGLH CVGDTYPSND RCCHECRPGN GMVSRCSRSQ
61 NTVCRPCGPG FYNDVVSSKP CKPCTWCNLR SGSERKQLCT ATQDTVCRCR AGTQPLDSYK
121 PGVDCAPCPP GHFSPGDNQA CKPWTNCTLA GKHTLQPASN SSDAICEDRD PPATQPQETQ
181 GPPARPITVQ PTEAWPRTSQ GPSTRPVEVP GGRAVAAILG LGLVLGLLGP LAILLALYLL
241 RRDQRLPPDA HKPPGGGSFR TPIQEEQADA HSTLAKI (SEQ ID NO:6)

By "**interleukin 6 (IL-6) nucleic acid**" is meant a polynucleotide encoding an interleukin 6 (IL-6) polypeptide. An exemplary interleukin 6 (IL-6) nucleic acid sequence is provided at NCBI Accession No. NM_000600. The exemplary sequence at NCBI Accession No. NM_000600 is provided below.

1 AATATTAGAG TCTCAACCCC CAATAAATAT AGGACTGGAG ATGTCTGAGG CTCATTCTGC
61 CCTCGAGCCC ACCGGGAACG AAAGAGAAGC TCTATCTCCC CTCCAGGAGC CCAGCTATGA
121 ACTCCTTCTC CACAAGCGCC TTCGGTCCAG TTGCCTTCTC CCTGGGGCTG CTCCTGGTGT
181 TGCCTGCTGC CTTCCCTGCC CCAGTACCCC CAGGAGAAGA TTCCAAAGAT GTAGCCGCCC
241 CACACAGACA GCCACTCACC TCTTCAGAAC GAATTGACAA ACAAATTCGG TACATCCTCG
301 ACGGCATCTC AGCCCTGAGA AAGGAGACAT GTAACAAGAG TAACATGTGT GAAAGCAGCA
361 AAGAGGCACT GGCAGAAAAC AACCTGAACC TTCCAAAGAT GGCTGAAAAA GATGGATGCT
421 TCCAATCTGG ATTCAATGAG GAGACTTGCC TGGTGAAAAT CATCACTGGT CTTTTGGAGT
481 TTGAGGTATA CCTAGAGTAC CTCCAGAACA GATTTGAGAG TAGTGAGGAA CAAGCCAGAG
541 CTGTGCAGAT GAGTACAAAA GTCCTGATCC AGTTCCTGCA GAAAAAGGCA AAGAATCTAG
601 ATGCAATAAC CACCCCTGAC CCAACCACAA ATGCCAGCCT GCTGACGAAG CTGCAGGCAC
661 AGAACCAGTG GCTGCAGGAC ATGACAACTC ATCTCATTCT GCGCAGCTTT AAGGAGTTCC

721 TGCAGTCCAG CCTGAGGGCT CTTCGGCAAA TGTAGCATGG GCACCTCAGA TTGTTGTTGT
781 TAATGGGCAT TCCTTCTTCT GGTCAGAAAC CTGTCCACTG GGCACAGAAC TTATGTTGTT
841 CTCTATGGAG AACTAAAAGT ATGAGCGTTA GGACACTATT TTAATTATTT TTAATTTATT
901 AATATTTAAA TATGTGAAGC TGAGTTAATT TATGTAAGTC ATATTTATAT TTTTAAGAAG
961 TACCACTTGA AACATTTTAT GTATTAGTTT TGAAATAATA ATGGAAAGTG GCTATGCAGT
1021 TTGAATATCC TTTGTTTCAG AGCCAGATCA TTTCTTGGAA AGTGTAGGCT TACCTCAAAT
1081 AAATGGCTAA CTTATACATA TTTTTAAAGA AATATTTATA TTGTATTTAT ATAATGTATA
1141 AATGGTTTTT ATACCAATAA ATGGCATTTT AAAAAATTCA GCAAAAAAAA AAAAAAAAAA
1201 A (SEQ ID NO:7)

By "**interleukin 6 receptor (IL-6R) complex**" is meant a protein complex comprising an IL-6 receptor subunit alpha (IL-6Rα) and interleukin 6 signal transducer Glycoprotein 130, also termed interleukin 6 receptor subunit β (IL-6Rβ).

By "**interleukin 6 receptor subunit α (IL-6Rα) polypeptide**" is meant a polypeptide or fragment thereof having at least about 85% or greater amino acid identity to the amino acid sequence provided at NCBI Accession No. NP_000556 or NP_852004 and having IL-6 receptor biological activity. Exemplary IL-6Rα biological activities include binding to IL-6, binding to glycoprotein 130 (gp130), and regulation of cell growth and differentiation. An exemplary IL-6R sequence is provided below:

1 MLAVGCALLA ALLAAPGAAL APRRCPAQEV ARGVLTSLPG DSVTLTCPGV EPEDNATVHW
61 VLRKPAAGSH PSRWAGMGRR LLLRSVQLHD SGNYSCYRAG RPAGTVHLLV DVPPEEPQLS
121 CFRKSPLSNV VCEWGPRSTP SLTTKAVLLV RKFQNSPAED FQEPCQYSQE SQKFSCQLAV
181 PEGDSSFYIV SMCVASSVGS KFSKTQTFQG CGILQPDPPA NITVTAVARN PRWLSVTWQD
241 PHSWNSSFYR LRFELRYRAE RSKTFTTWMV KDLQHHCVIH DAWSGLRHVV QLRAQEEFGQ
301 GEWSEWSPEA MGTPWTESRS PPAENEVSTP MQALTTNKDD DNILFRDSAN ATSLPVQDSS
361 SVPLPTFLVA GGSLAFGTLL CIAIVLRFKK TWKLRALKEG KTSMHPPYSL GQLVPERPRP
421 TPVLVPLISP PVSPSSLGSD NTSSHNRPDA RDPRSPYDIS NTDYFFPR (SEQ ID NO:8)

By "**interleukin 6 receptor subunit β (IL-6Rβ) polypeptide**" is meant a polypeptide or fragment thereof having at least about 85% or greater amino acid identity to the amino acid sequence provided at NCBI Accession No. NP_002175, NP_786943, or NP_001177910 and having IL-6 receptor biological activity. Exemplary IL-6Rβ biological activities include binding to IL-6Rα, IL-6 receptor signaling activity, and regulation of cell growth, differentiation, hepcidin expression etc. An exemplary IL-6Rβ sequence is provided below:

1 MLTLQTWLVQ ALFIFLTTES TGELLDPCGY ISPESPVVQL HSNFTAVCVL KEKCMDYFHV
61 NANYIVWKTN HFTIPKEQYT IINRTASSVT FTDIASLNIQ LTCNILTFGQ LEQNVYGITI
121 ISGLPPEKPK NLSCIVNEGK KMRCEWDGGR ETHLETNFTL KSEWATHKFA DCKAKRDTPT

181 SCTVDYSTVY FVNIEVWVEA ENALGKVTSD HINFDPVYKV KPNPPHNLSV INSEELSSIL

241 KLTWTNPSIK SVIILKYNIQ YRTKDASTWS QIPPEDTAST RSSFTVQDLK PFTEYVFRIR

301 CMKEDGKGYW SDWSEEASGI TYEDRPSKAP SFWYKIDPSH TQGYRTVQLV WKTLPPFEAN

361 GKILDYEVTL TRWKSHLQNY TVNATKLTVN LTNDRYLATL TVRNLVGKSD AAVLTIPACD

421 FQATHPVMDL KAFPKDNMLW VEWTTPRESV KKYILEWCVL SDKAPCITDW QQEDGTVHRT

481 YLRGNLAESK CYLITVTPVY ADGPGSPESI KAYLKQAPPS KGPTVRTKKV GKNEAVLEWD

541 QLPVDVQNGF IRNYTIFYRT IIGNETAVNV DSSHTEYTLS SLTSDTLYMV RMAAYTDEGG

601 KDGPEFTFTT PKFAQGEIEA IVVPVCLAFL LTTLLGVLFC FNKRDLIKKH IWPNVPDPSK

661 SHIAQWSPHT PPRHNFNSKD QMYSDGNFTD VSVVEIEAND KKPFPEDLKS LDLFKKEKIN

721 TEGHSSGIGG SSCMSSSRPS ISSSDENESS QNTSSTVQYS TVVHSGYRHQ VPSVQVFSRS

781 ESTQPLLDSE ERPEDLQLVD HVDGGDGILP RQQYFKQNCS QHESSPDISH FERSKQVSSV

841 NEEDFVRLKQ QISDHISQSC GSGQMKMFQE VSAADAFGPG TEGQVERFET VGMEAATDEG

901 MPKSYLPQTV RQGGYMPQ (SEQ ID NO:9)

By "**IL-6 antagonist**" is meant an agent that is capable of decreasing the biological activity of IL-6. IL-6 antagonists include agents that decrease the level of IL-6 polypeptide in serum, including agents that decrease the expression of an IL-6 polypeptide or nucleic acid; agents that decrease the ability of IL-6 to bind to the IL-6R; agents that decrease the expression of the IL-6R; and agents that decrease signal transduction by the IL-6R receptor when bound by IL-6. In preferred embodiments, the IL-6 antagonist decreases IL-6 biological activity by at least about 10%, 20%, 30%, 50%, 70%, 80%, 90%, 95%, or even 100%. As further described in Section 5.7 below, IL-6 antagonists include IL-6 binding polypeptides, such as anti-IL-6 antibodies and antigen binding fragments or derivatives thereof; IL-6R binding polypeptides, such as anti-IL-6R antibodies and antigen binding fragments or derivatives thereof; and synthetic chemical molecules, such as JAK1 and JAK3 inhibitors.

By "**IL-6 antibody**" or "**anti-IL-6 antibody**" is meant an antibody that specifically binds IL-6. Anti-IL-6 antibodies include monoclonal and polyclonal antibodies that are specific for IL-6, and antigen-binding fragments or derivatives thereof. IL-6 antibodies are described in greater detail in Section 5.7.1 below.

By "**IL-6 mediated inflammatory disorder**" is meant any disorder in which IL-6 is known or suspected to contribute to the etiology of the disease or any of its symptoms.

By "**erythropoietin (EPO)**" is meant a polypeptide or fragment thereof having at least about 85% or greater amino acid identity to the amino acid sequence provided at NCBI Accession No. NP_000790 and having EPO biological activity. Exemplary EPO biological activities include binding to the erythropoietin receptor and resultant proliferation and terminal differentiation of erythroid precursor cells and/or increasing erythropoiesis (red blood cell production). An exemplary EPO amino acid sequence is provided below:

1 MGVHECPAWL WLLLSLLSLP LGLPVLGAPP RLICDSRVLE RYLLEAKEAE NITTGCAEHC
61 SLNENITVPD TKVNFYAWKR MEVGQQAVEV WQGLALLSEA VLRGQALLVN SSQPWEPLQL
121 HVDKAVSGLR SLTTLLRALG AQKEAISPPD AASAAPLRTI TADTFRKLFR VYSNFLRGKL
181 KLYTGEACRT GDR (SEQ ID NO:10)

By "**erythropoiesis stimulating agent (ESA)**" is meant an agent that stimulates erythropoiesis. ESAs include, but are not limited to, EPO; darbepoetin (Aranesp); epoetin beta (NeoRecormon); epoetin delta (Dynepo); epoetin omega (Epomax); epoetin zeta.

By "**erythropoietic factor**" is meant an agent that increases the growth or proliferation of a red blood cell or progenitor thereof (e.g., a hematopoietic stem cell) and/or decrease cell death in a red blood cell or progenitor thereof. In various embodiments, erythropoietic factors include erythropoiesis stimulating agents, HIF stabilizers, and supplemental iron.

By "**C-reactive protein (CRP) polypeptide**" is meant a polypeptide or fragment thereof having at least about 85% or greater amino acid identity to the amino acid sequence provided at NCBI Accession No. NP_000558 and having complement activating activity. CRP levels increase in response to inflammation. An exemplary CRP sequence is provided below:

1 MEKLLCFLVL TSLSHAFGQT DMSRKAFVFP KESDTSYVSL KAPLTKPLKA FTVCLHFYTE
61 LSSTRGYSIF SYATKRQDNE ILIFWSKDIG YSFTVGGSEI LFEVPEVTVA PVHICTSWES
121 ASGIVEFWVD GKPRVRKSLK KGYTVGAEAS IILGQEQDSF GGNFEGSQSL VGDIGNVNMW
181 DFVLSPDEIN TIYLGGPFSP NVLNWRALKY EVQGEVFTKP QLWP (SEQ ID NO:11)

By "**agent**" is meant any compound or composition suitable to be administered in therapy, and explicitly includes chemical compounds; proteins, including antibodies or antigen-binding fragments thereof; peptides; and nucleic acid molecules.

By "**subject**" is meant a human or non-human mammal, including, but not limited to, bovine, equine, canine, ovine, feline, and rodent, including murine and rattus, subjects. A "**patient**" is a human subject.

As used herein, the terms "**treat**," **treating**," "**treatment**," and the like refer to reducing or ameliorating a disorder, and/or signs or symptoms associated therewith, or slowing or halting the progression thereof. It will be appreciated that, although not precluded, treating a disorder or condition does not require that the disorder, condition or symptoms associated therewith be completely eliminated.

"**Pre-treatment**" means prior to the first administration of an IL-6 antagonist according the methods described herein. Pre-treatment does not exclude, and often includes, the prior administration of treatments other than an IL-6 antagonist.

In this disclosure, "**comprises**," "**comprising**," "**containing**," "**having**," "**includes**," "**including**," and linguistic variants thereof have the meaning ascribed to them in U.S. Patent law, permitting the presence of additional components beyond those explicitly recited.

By "**biological sample**" is meant any tissue, cell, fluid, or other material derived from an organism (e.g., human subject). In certain embodiments, the biological sample is serum or blood.

By "**angiotensin converting enzyme (ACE) inhibitor**" is meant an agent that inhibits an angiotensin converting enzyme's biological function of converting angiotensin I to angiotensin II. ACE inhibitors include, without limitation, quinapril, perindopril, ramipril, captopril, benazepril, trandolapril, fosinopril, lisinopril, moexipril, and enalapril. In various embodiments, the ACE inhibitor is perindopril.

### 5.1. Other interpretational conventions

Unless otherwise specified, antibody constant region residue numbering is according to the EU index as in Kabat.

Ranges provided herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

Unless specifically stated or apparent from context, as used herein the term "or" is understood to be inclusive. Unless specifically stated or apparent from context, as used herein, the terms "a", "an", and "the" are understood to be singular or plural.

Unless specifically stated or otherwise apparent from context, as used herein the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

### 5.2. Methods Of Treating Hepcidin-Mediated Disorders

In a first aspect, methods of treating a hepcidin-mediated disorder are provided.

The methods comprise administering a therapeutically effective amount of an IL-6 antagonist to a subject, typically a human patient, who has a hepcidin-mediated disorder, wherein the subject has been determined to have at least one copy of the *TMPRSS6* rs855791 major allele. In a first series of embodiments, the subject has previously been determined to have at least one copy of the *TMPRSS6* rs855791 major allele. In another series of embodiments, the method further comprises the earlier step of determining that the subject has at least one copy of the *TMPRSS6* rs855791 major allele. Typically, the methods affirmatively exclude treatment of subjects who are homozygous for the *TMPRSS6* rs855791 minor allele. Typically, the patient has elevated pre-treatment serum levels of IL-6.

### 5.2.1. Hepcidin-Mediated Disorders

### 5.2.1.1. Anemia of Chronic Disease / Chronic Inflammation

In various embodiments, the hepcidin-mediated disorder treated by the methods described herein is an anemia of chronic disease, also known as anemia of chronic inflammation.

In various embodiments, the patient is male and has a pre-treatment hemoglobin (Hb) content of less than 14 g/dl. In some embodiments, the male patient has a pre-treatment Hb level of 13.0 - 13.9 g/dl, 12.0 - 12.9 g/dl, 11.0 - 11.9 g/dl, 10.0 - 10.9 g/dl, or less than 10 g/dl. In various embodiments, the patient is female and has a pre-treatment Hb content of less than 12 g/dl. In some embodiments, the female patient has a pre-treatment Hb level of 11.0 - 11.9 g/dl, 10.0 - 10.9 g/dl, 9.0 - 9.9 g/dl, 8.0 - 8.9 g/dl, or less than 8 g/dl. In some of these embodiments, prior the patient has been treated with an ESA. In some embodiments, the patient has been treated with iron supplementation. In some embodiments, the patient has been treated with transfusion of blood or packed red blood cells.

In various embodiments, the patient is male and has a pre-treatment hematocrit of less than 40%. In some embodiments, the male patient has a pre-treatment hematocrit less than 39%, less than 38%, less than 37%, less than 36%, or less than 35%. In certain embodiments, the male patient has a pre-treatment hematocrit of 39%, 38%, 37%, 36%, 35%, 34%, 33%, 32%, 31% or 30%. In various embodiments, the patient is female, and has a pre-treatment hematocrit of less than 36%. In some embodiments, the female patient has a pre-treatment hematocrit of less than 35%, 34%, 33%, 32%, 31%, 30%, 29%, 28%, 27%, or 26%. In certain embodiments, the female patient has a pre-treatment hematocrit of 35%, 34%, 33%, 32%, 31%, 30%, 29%, 28%, 27%, or 26%. In some of these embodiments, the patient has been treated with an ESA. In some embodiments, the patient has been treated with iron supplementation. In some embodiments, the patient has been treated with transfusion of blood or packed red blood cells.

In some embodiments, the patient has been treated with an ESA and has a normal pre-treatment Hb content and/or normal pre-treatment hematocrit. In certain embodiments, the patient is male and has a pre-treatment hemoglobin (Hb) content of at least 14 g/dl and/or a pre-treatment hematocrit of at least 40%. In certain embodiments, the patient is female, and has a pre-treatment Hb content of at least 12 g/dl and/or a hematocrit of at least 36%. In specific embodiments, the ESA is EPO. In specific embodiments, the ESA is darbepoetin alfa.

In some embodiments, the patient has been treated with iron supplementation, and has a normal pre-treatment Hb content and/or normal pre-treatment hematocrit. In certain embodiments, the patient is male and has a pre-treatment hemoglobin (Hb) content of at least 14 g/dl and/or a pre-treatment hematocrit of at least 40%. In certain embodiments, the patient is female, and has a pre-treatment Hb content of at least 12 g/dl and/or a hematocrit of at least 36%.

In some embodiments, the patient has been treated with transfusion of whole blood or packed red blood cells, and has a normal pre-treatment Hb content and/or normal pre-treatment hematocrit. In certain embodiments, the patient is male and has a pre-treatment hemoglobin (Hb) content of at least 14 g/dl and/or a pre-treatment hematocrit of at least 40%. In certain embodiments, the patient is female, and has a pre-treatment Hb content of at least 12 g/dl and/or a hematocrit of at least 36%.

In some embodiments, the IL-6 antagonist is administered at a dose, on a schedule, and for a period sufficient to increase the patient's Hb levels above pre-treatment levels. In some embodiments, the IL-6 antagonist is administered at a dose, on a schedule, and for a period sufficient to increase the patient's hematocrit above pre-treatment levels. In some embodiments, the IL-6 antagonist is administered at a dose, on a schedule, and for a period sufficient to increase both Hb levels and hematocrit above pre-treatment levels.

In some embodiments, the IL-6 antagonist is administered at a dose, on a schedule, and for a period sufficient to allow reduction in the patient's dose of ESA without reduction in the patient's Hb levels below pre-treatment levels. In some embodiments, the IL-6 antagonist is administered at a dose, on a schedule, and for a period sufficient to allow reduction in the patient's dose of ESA without reduction in the patient's hematocrit below pre-treatment levels. In some embodiments, the IL-6 antagonist is administered at a dose, on a schedule, and for a period sufficient to allow reduction in the patient's dose of ESA without reduction in the patient's Hb levels and hematocrit.

In some embodiments, the IL-6 antagonist is administered at a dose, on a schedule, and for a period sufficient to allow at least a 10% reduction in the patient's dose of ESA as compared to pre-treatment ESA dose. In certain embodiments, the IL-6 antagonist is administered at a dose, on a schedule, and for a period sufficient to allow at least a 20%, 30%, 40%, or 50% reduction in the patient's dose of ESA as compared to pre-treatment ESA dose. In particular embodiments, the IL-6 antagonist is administered at a dose, on a schedule, and for a period sufficient to allow at least 60%, or even at least 75% reduction in patient's dose of ESA as compared to pre-treatment ESA dose.

In some embodiments, the IL-6 antagonist is administered at a dose, on a schedule, and for a period sufficient to reverse functional iron deficiency.

### 5.2.1.1.1. Chronic Kidney Disease

In various embodiments, the chronic disease is chronic kidney disease (CKD).

In some embodiments, the patient has KDOQI stage 1 chronic kidney disease. In certain embodiments, the patient has KDOQI stage 2 chronic kidney disease, KDOQI stage 3 chronic kidney disease, KDOQI stage 4 chronic kidney disease, or KDOQI stage 5 chronic kidney disease.

In some embodiments, the patient has cardiorenal syndrome (CRS). In certain embodiments, the patient has CRS Type 4.

In some embodiments, the patient has been treated with dialysis.

In some embodiments, the IL-6 antagonist is administered at a dose, on a schedule, and for a period sufficient to reduce cardiovascular (CV) mortality as compared to age-matched and disease-matched historical cohorts.

### 5.2.1.1.2. Chronic Inflammatory Diseases

In various embodiments, the chronic disease is a chronic inflammatory disease.

In some embodiments, the chronic inflammatory disease is rheumatoid arthritis (RA).

In specific embodiments, the patient has a pre-treatment DAS28 score of greater than 5.1. In some embodiments, the patient has a pre-treatment DAS28 score of 3.2 to 5.1. In some embodiments, the patient has a pre-treatment DAS28 score of less than 2.6. In various embodiments, the patient's pre-treatment RA is severely active. In some embodiments, the patient's pre-treatment RA is moderately active.

In certain embodiments, the patient has been treated with methotrexate. In some embodiments, methotrexate is discontinued when treatment with an IL-6 antagonist is initiated. In some embodiments, treatment with methotrexate is continued when treatment with an IL-6 antagonist is initiated.

In certain embodiments, the patient has been treated with an anti-TNFa agent. In particular embodiments, the anti-TNFa agent is selected from etanercept, adalimumab, infliximab, certolizumab, and golimumab. In particular embodiments, the anti-TNFa agent is discontinued when treatment with an IL-6 antagonist is initiated.

In certain embodiments, the patient has been treated with an IL-1 receptor antagonist. In specific embodiments, the IL-1 receptor antagonist is anakinra. In particular embodiments, the IL-1 receptor antagonist is discontinued when treatment with an IL-6 antagonist is initiated.

In certain embodiments, the patient has been treated with abatacept. In particular embodiments, abatacept is discontinued when treatment with an IL-6 antagonist is initiated.

In certain embodiments, the patient has been treated with an IL-6 antagonist, and the method further comprises continuing to administer an IL-6 antagonist only to those patients newly determined to have at least one copy of the *TMPRSS6* rs855791 major allele. In specific embodiments, the IL-6 antagonist is tocilizumab. In specific embodiments, the IL-6 antagonist is tofacitinib.

In various embodiments, the chronic inflammatory disease is selected from the group consisting of juvenile idiopathic arthritis, ankylosing spondylitis, plaque psoriasis, psoriatic arthritis, inflammatory bowel disease, Crohn's disease, and ulcerative colitis.

### 5.2.1.1.3. Cancer

In various embodiments, the chronic disease is cancer.

In some embodiments, the cancer is selected from the group consisting of: solid tumors, small cell lung cancer, non-small cell lung cancer, hematological cancer, multiple myeloma, leukemias, chronic lymphocytic leukemia (CLL), chronic myeloid leukemia (CML), lymphomas, and Hodgkin's lymphoma.

### 5.2.1.1.4. Chronic Infection

In various embodiments, the chronic disease is a chronic infection.

### 5.2.1.1.5. Congestive Heart Failure

In various embodiments, the chronic disease is congestive heart failure (CHF).

### 5.2.1.2. Iron-refractory Iron-deficiency Anemia (IRIDA)

In various embodiments, the hepcidin-mediated disorder is iron-refractory iron-deficiency anemia (IRIDA).

### 5.2.1.3. Anemia Associated With Hepcidin-Producing Hepatic Adenomas

In various embodiments, the hepcidin-mediated disorder is anemia associated with a hepcidin-producing hepatic adenoma.

### 5.2.1.4. Acute Coronary Syndrome

The data presented in Examples 2, 3, and 5 below demonstrate that IL-6 antagonists are effective in reducing risk of heart failure and death, and in increasing cardiac function and reducing fibrosis, after acute myocardial infarction. Accordingly, in various embodiments, the hepcidin-mediated disorder is acute coronary syndrome.

In certain embodiments, the patient has suffered a myocardial infarction within the 60 days prior to first administration of an IL-6 antagonist. In particular embodiments, the patient has suffered a myocardial infarction within the 30 days, 14 days, 7 days, 48 hours, or 24 hours prior to first administration of an IL-6 antagonist.

In some embodiments, the IL-6 antagonist is administered at a dose, on a schedule, and for a period sufficient to improve myocardial contractility as compared to pre-treatment levels. In certain embodiments, the IL-6 antagonist is administered at dose, on a schedule, and for a period sufficient to improve cardiac ejection fraction as compared to pre-treatment levels. In certain embodiments, the IL-6 antagonist is administered at dose, on a schedule, and for a period sufficient to reduce cardiac fibrosis as compared to pre-treatment levels.

### 5.2.1.5. Castleman's Disease

In various embodiments, the hepcidin-mediated disorder is Castleman's Disease.

### 5.3. Methods Of Improving Treatment of Hepcidin-Mediated Disorders

In another aspect, methods are provided for improving treatment of a hepcidin-mediated disorder by discontinuing therapy that is ineffective, thereby reducing side effects and reducing cost without loss of treatment efficacy. The methods comprise discontinuing administration of an IL-6 antagonist to a patient with a hepcidin-mediated disorder, wherein the patient has been determined to be homozygous for the *TMPRSS6* rs855791 minor allele. In one series of embodiments, the patient has previously been determined to be homozygous for the *TMPRSS6* rs855791 minor allele. In another series of embodiments, the method further comprises the earlier step of determining that the patient is homozygous for the *TMPRSS6* rs855791 minor allele. In typical embodiments, the patient has elevated pre-treatment serum levels of IL-6. In various embodiments, the patient has elevated pre-treatment serum levels of CRP.

In various embodiments, the patient has a hepcidin-mediated disorder selected from those described in Section 5.2.1 above. In certain embodiments, the patient has anemia of chronic disease.

### 5.4. Methods of Treating IL-6 Mediated Inflammatory Disorders

The data presented in Examples 2, 3 and 5 below demonstrate that IL-6 antagonists provide therapeutic benefit in subjects having elevated pre-treatment IL-6 levels and at least one copy of the *TMPRSS6* major allele, even in the absence of anemia. Accordingly, in another aspect, methods are provided for treating IL-6 mediated inflammatory disorders in patients without anemia of chronic inflammation.

The methods comprise administering a therapeutically effective amount of an IL-6 antagonist to a subject, typically a human patient, who has an IL-6 mediated inflammatory disorder, wherein the patient does not have anemia, and wherein the subject has been determined to have at least one copy of the *TMPRSS6* rs855791 major allele. In a first series of embodiments, the subject has previously been determined to have at least one copy of the *TMPRSS6* rs855791 major allele. In another series of embodiments, the method further comprises the earlier step of determining that the subject has at least one copy of the *TMPRSS6* rs855791 major allele. Typically, the methods affirmatively exclude treatment of subjects who are homozygous for the *TMPRSS6* rs855791 minor allele. Typically, the patient has elevated pre-treatment serum levels of IL-6.

In some embodiments, the IL-6 mediated disorder is rheumatoid arthritis (RA).

In specific embodiments, the patient has a pre-treatment DAS28 score of greater than 5.1. In some embodiments, the patient has a pre-treatment DAS28 score of 3.2 to 5.1. In some embodiments, the patient has a pre-treatment DAS28 score of less than 2.6. In various embodiments, the patient's pre-treatment RA is severely active. In some embodiments, the patient's pre-treatment RA is moderately active.

In certain embodiments, the patient has been treated with methotrexate. In some embodiments, methotrexate is discontinued when treatment with an IL-6 antagonist is initiated. In some embodiments, methotrexate is continued when treatment with an IL-6 antagonist is initiated.

In certain embodiments, the patient has been treated with an anti-TNFa agent. In particular embodiments, the anti-TNFa agent is selected from etanercept, adalimumab, infliximab, certolizumab, and golimumab. In particular embodiments, the anti-TNFa agent is discontinued when treatment with an IL-6 antagonist is initiated.

In certain embodiments, the patient has been treated with abatacept. In particular embodiments, abatacept is discontinued when treatment with an IL-6 antagonist is initiated.

In various embodiments, the IL-6 mediated disorder is selected from the group consisting of juvenile idiopathic arthritis, ankylosing spondylitis, plaque psoriasis, psoriatic arthritis, inflammatory bowel disease, Crohn's disease, and ulcerative colitis.

### 5.5. Pre-Treatment Serum IL-6 And CRP Levels

In typical embodiments of the methods described herein, the patient has elevated pre-treatment serum levels of IL-6.

In some embodiments, the patient has a pre-treatment serum IL-6 level of greater than 2.5 pg/ml. In various embodiments, the patient has a pre-treatment serum IL-6 level of greater than 5 pg/ml, greater than 7.5 pg/ml, greater than 10 pg/ml, greater than 12.5 pg/ml, or greater than 15 pg/ml.

In some embodiments, the IL-6 antagonist is administered at a dose, on a schedule, and for a period sufficient to reduce the patient's serum IL-6 levels below pre-treatment levels. In certain embodiments, the IL-6 antagonist is administered at a dose, on a schedule, and for a period sufficient to reduce the patient's serum IL-6 levels by at least 10%, 20%, 30%, 40%, or 50% as compared to pre-treatment levels.

In various embodiments, the patient has elevated pre-treatment levels of C-reactive protein (CRP). In some embodiments, the patient has a pre-treatment CRP level greater than 2 mg/L, 2.5 mg/L, 3 mg/L, 3.5 mg/L, 4 mg/L, 4.5 mg/L, or 5 mg/L. In some embodiments, the patient has pre-treatment CRP levels greater than 7.5 mg/L, 10 mg/L, 12.5 mg/L, or 15 mg/L.

In some embodiments, the IL-6 antagonist is administered at a dose, on a schedule, and for a period sufficient to reduce the patient's CRP levels below pre-treatment levels. In certain embodiments, the IL-6 antagonist is administered at a dose, on a schedule, and for a period sufficient to reduce the patient's CRP levels by at least 10%, 20%, 30%, 40%, or 50% as compared to pre-treatment levels.

### 5.6. TMPRSS6 rs855791 Genotyping

Methods described herein comprise administering a therapeutically effective amount of an IL-6 antagonist to a subject who has been determined to have at least one copy of the *TMPRSS6* rs855791 major allele. Preferably, both alleles corresponding to a gene of interest are identified, thus permitting identification and discrimination of patients who are homozygous for the *TMPRSS6* rs855791 major allele, heterozygous for the major and minor *TMPRSS6* rs855791 alleles, and homozygous for the *TMPRSS6* rs855791 minor allele. *TMPRSS6* rs855791 major allele. Preferably, both alleles corresponding to a gene of interest are identified, thus permitting identification and discrimination of patients who are homozygous for the *TMPRSS6* rs855791 major allele, heterozygous for the major and minor *TMPRSS6* rs855791 alleles, and homozygous for the *TMPRSS6* rs855791 minor allele.

The absence (major allele) or presence (minor allele) of SNP rs855791 (2321G→A) in the *TMPRSS6* gene is determined using standard techniques.

Typically, PCR is used to amplify a biological sample obtained from the patient.

In some embodiments, the absence or presence of polymorphism is detected concurrently with amplification using real-time PCR (RT-PCR). In certain embodiments, the RT-PCR assay employs 5' nuclease (TaqMan® probes), molecular beacons, and/or FRET hybridization probes. *Reviewed in* Espy et al., Clin. Microbiol. Rev. 2006 January; 19(1): 165-256. In typical embodiments, a commercially available assay is used. In select embodiments, the commercially available assay is selected from the group consisting of TaqMan™ SNP Genotyping Assays (ThermoFisher); PCR SNP Genotyping Assay (Qiagen); Novallele Genotyping Assays (Canon); and SNP Type™ assays (formerly SNPtype) (Fluidigm).

In some embodiments, the absence or presence of polymorphism is detected following amplification using hybridization with a probe specific for SNP rs855791, restriction endonuclease digestion, nucleic acid sequencing, primer extension, microarray or gene chip analysis, mass spectrometry, and/or a DNAse protection assay. In some embodiments, the allelic variants are called by sequencing. In certain embodiments, Sanger sequencing is used. In certain embodiments, one of a variety of next-generation sequencing techniques is used, including for example a sequencing technique selected from the group consisting of microarray sequencing, Solexa sequencing (Illumina), Ion Torrent (Life Technologies), SOliD (Applied Biosystems), pyrosequencing, single-molecule real-time sequencing (Pacific Bio), nanopore sequencing and tunneling currents sequencing.

### 5.7. IL-6 Antagonists

The IL-6 antagonist used in the methods described herein is capable of decreasing the biological activity of IL-6.

### 5.7.1. Anti-IL-6 Antibodies

In various embodiments, the IL-6 antagonist is an anti-IL-6 antibody or antigen-binding fragment or derivative thereof.

In some embodiments, the IL-6 antagonist is a full-length anti-IL-6 monoclonal antibody. In particular embodiments, the full-length monoclonal antibody is an IgG antibody. In certain embodiments, the full-length monoclonal antibody is an IgG1, IgG2, IgG3, or IgG4 antibody. In some embodiments, the IL-6 antagonist is a polyclonal composition comprising a plurality of species of full-length anti-IL-6 antibodies, each of the plurality having unique CDRs. In some embodiments, the IL-6 antagonist is an antibody fragment selected from Fab, Fab', and F(ab')2 fragments. In some embodiments, the IL-6 antagonist is a scFv, a disulfide-linked Fv (dsFv), or a single domain antibody, such as a camelid-derived VHH single domain Nanobody. In some embodiments, the IL-6 antagonist is immunoconjugate or fusion comprising an IL-6 antigen-binding fragment. In some embodiments, the antibody is bispecific or multispecific, with at least one of the antigen-binding portions having specificity for IL-6.

In some embodiments, the antibody is fully human. In some embodiments, the antibody is humanized. In some embodiments, the antibody is chimeric and has non-human V regions and human C region domains. In some embodiments, the antibody is murine.

In typical embodiments, the anti-IL-6 antibody has a K_{D} for binding human IL-6 of less than 100 nM. In some embodiments, the anti-IL-6 antibody has a K_{D} for binding human IL-6 of less than 75 nM, 50 nM, 25 nM, 20 nM, 15 nM, or 10 nM. In particular embodiments, the anti-IL-6 antibody has a K_{D} for binding human IL-6 of less than 5 nM, 4 nM, 3 nM, or 2 nM. In selected embodiments, the anti-IL-6 antibody has a K_{D} for binding human IL-6 of less than 1 nM, 750 pM, or 500 pM. In specific embodiments, the anti-IL-6 antibody has a K_{D} for binding human IL-6 of no more than 500 pM, 400 pM, 300 pM, 200 pM, or 100 pM.

In typical embodiments, the anti-IL-6 antibody neutralizes the biological activity of IL-6. In some embodiments, the neutralizing antibody prevents binding of IL-6 to the IL-6 receptor.

In typical embodiments, the anti-IL-6 antibody has an elimination half-life following intravenous administration of at least 7 days. In certain embodiments, the anti-IL-6 antibody has an elimination half-life of at least 14 days, at least 21 days, or at least 30 days.

In some embodiments, the anti-IL-6 antibody has a human IgG constant region with at least one amino acid substitution that extends serum half-life as compared to the unsubstituted human IgG constant domain.

In certain embodiments, the IgG constant domain comprises substitutions at residues 252, 254, and 256, wherein the amino acid substitution at amino acid residue 252 is a substitution with tyrosine, the amino acid substitution at amino acid residue 254 is a substitution with threonine, and the amino acid substitution at amino acid residue 256 is a substitution with glutamic acid ("YTE"). *See* U.S. Pat. No. 7,083,784. In certain extended half-life embodiments, the IgG constant domain comprises substitutions selected from T250Q/M428L (Hinton et al., J. Immunology 176:346-356 (2006)); N434A (Yeung et al., J. Immunology 182:7663-7671 (2009)); or T307A/E380A/N434A (Petkova et al., International Immunology, 18: 1759-1769 (2006)).

In some embodiments, the elimination half-life of the anti-IL-6 antibody is increased by utilizing the FcRN-binding properties of human serum albumin. In certain embodiments, the antibody is conjugated to albumin (Smith et al., Bioconjug. Chem., 12: 750-756 (2001)). In some embodiments, the anti-IL-6 antibody is fused to bacterial albumin-binding domains (Stork et al., Prot. Eng. Design Science 20: 569-76 (2007)). In some embodiments, the anti-IL-6 antibody is fused to an albumin-binding peptide (Nguygen et al., Prot Eng Design Sel 19: 291-297 (2006)). In some embodiments, the anti-IL-antibody is bispecific, with one specificity being to IL-6, and one specificity being to human serum albumin (Ablynx, WO 2006/122825 (bispecific Nanobody)).

In some embodiments, the elimination half-life of the anti-IL-6 antibody is increased by PEGylation (Melmed et al., Nature Reviews Drug Discovery 7: 641-642 (2008)); by HPMA copolymer conjugation (Lu et al., Nature Biotechnology 17: 1101-1104 (1999)); by dextran conjugation (Nuclear Medicine Communications, 16: 362-369 (1995)); by conjugation with homo-amino-acid polymers (HAPs; HAPylation) (Schlapschy et al., Prot Eng Design Sel 20: 273-284 (2007)); or by polysialylation (Constantinou et al., Bioconjug. Chem. 20: 924-931 (2009)).

### 5.7.1.1.1. MED5117 and Derivatives

In certain embodiments, the anti-IL-6 antibody or antigen-binding portion thereof comprises all six CDRs of MEDI5117. In particular embodiments, the antibody or antigen-binding portion thereof comprises the MEDI5117 heavy chain V region and light chain V region. In specific embodiments, the antibody is the full-length MEDI5117 antibody. The MEDI5117 antibody is described in WO 2010/088444 and US 2012/0034212. The MEDI5117 antibody has the following CDR and heavy and light chain sequences:
MEDI5117 VH CDR1
   SNYMI (SEQ ID NO:12)
MEDI5117 VH CDR2
   DLYYYAGDTYYADSVKG (SEQ ID NO:13)
MEDI5117 VH CDR3
   WADDHPPWIDL (SEQ ID NO:14)
MEDI5117 VL CDR1
   RASQGISSWLA (SEQ ID NO:15)
MEDI5117 VL CDR2
   KASTLES (SEQ ID NO:16)
MEDI5117 VL CDR3
   QQSWLGGS (SEQ ID NO:17)
MEDI5117 Heavy chain 
EVQLVESGGGLVQPGGSLRLSCAASGFTISSNYMIWVRQAPGKGLEWVSDLYYYAGDTYY
ADSVKGRFTMSRDISKNTVYLQMNSLRAEDTAVYYCARWADDHPPWIDLWGRGTLVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLYITREPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO:18)
MEDI5117 Light chain

DIQMTQSPSTLSASVGDRVTITCRASQGISSWLAWYQQKPGKAPKVLIYKASTLESGVPS
RFSGSGSGTEFTLTISSLQPDDFATYYCQQSWLGGSFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC (SEQ ID NO:19)

In various embodiments, the anti-IL-6 antibody is a derivative of MED5117.

In some embodiments, the MED5117 derivative includes one or more amino acid substitutions in the MED5117 heavy and/or light chain V regions.

In certain embodiments, the derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the MEDI5117 anti-IL-6 antibody, while retaining specificity for human IL-6.

In certain embodiments, the MED5117 derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the VH and VL domain of MEDI5117. The percent sequence identity is determined using BLAST algorithms using default parameters.

In certain embodiments, the MED5117 derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of MEDI5117. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 5.7.1.1.2. Other Anti-IL-6 Antibodies

In various embodiments, the anti-IL-6 antibody comprises the six CDRs from an antibody selected from the group consisting of siltuximab, gerilimzumab, sirukumab, clazakizumab, olokizumab, elsilimomab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb). In certain embodiments, the anti-IL-6 antibody comprises the heavy chain V region and light chain V region from an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb). In particular embodiments, the anti-IL-6 antibody is an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

In some embodiments, the anti-IL-6 antibody comprises the six CDRs from an antibody selected from those described in US 2016/0168243, US 2016/0130340, US 2015/0337036, US 2015/0203574, US 2015/0140011, US 2015/0125468, US 2014/0302058, US 2014/0141013, US 2013/0280266, US 2013/0017575, US 2010/0215654, US 2008/0075726, US Pat. No. 5,856,135, US 2006/0240012, US 2006/0257407, or U.S. Pat. No. 7291721.

### 5.7.2. Anti-IL-6 Receptor Antibodies

In various embodiments, the IL-6 antagonist is an anti-IL-6 receptor antibody or antigen-binding fragment or derivative thereof

In some embodiments, the IL-6 antagonist is a full-length anti-IL-6 receptor monoclonal antibody. In particular embodiments, the full-length monoclonal antibody is an IgG antibody. In certain embodiments, the full-length monoclonal antibody is an IgG1, IgG2, IgG3, or IgG4 antibody. In some embodiments, the IL-6 antagonist is a polyclonal composition comprising a plurality of species of full-length anti-IL-6 receptor antibodies, each of the plurality having unique CDRs. In some embodiments, the IL-6 antagonist is an antibody fragment selected from Fab and Fab' fragments. In some embodiments, the IL-6 antagonist is a scFv, a single domain antibody, including a camelid-derived VHH single domain Nanobody. In some embodiments, the antibody is bispecific or multispecific, with at least one of the antigen-binding portions having specificity for IL-6R.

In some embodiments, the antibody is fully human. In some embodiments, the antibody is humanized. In some embodiments, the antibody is chimeric and has non-human V regions and human C region domains. In some embodiments, the antibody is murine.

In typical embodiments, the anti-IL-6 receptor antibody has a K_{D} for binding human IL-6R of less than 100 nM. In some embodiments, the anti-IL-6R antibody has a K_{D} for binding human IL-6R of less than 75 nM, 50 nM, 25 nM, 20 nM, 15 nM, or 10 nM. In particular embodiments, the anti-IL-6 receptor antibody has a K_{D} for binding human IL-6R of less than 5 nM, 4 nM, 3 nM, or 2 nM. In selected embodiments, the anti-IL-6 receptor antibody has a K_{D} for binding human IL-6R of less than 1 nM, 750 pM, or 500 pM. In specific embodiments, the anti-IL-6 receptor antibody has a K_{D} for binding human IL-6R of no more than 500 pM, 400 pM, 300 pM, 200 pM, or 100 pM.

In typical embodiments, the anti-IL-6R reduces the biological activity of IL-6.

In typical embodiments, the anti-IL-6R antibody has an elimination half-life following intravenous administration of at least 7 days. In certain embodiments, the anti-IL-6R antibody has an elimination half-life of at least 14 days, at least 21 days, or at least 30 days.

In some embodiments, the anti-IL-6R antibody has a human IgG constant region with at least one amino acid substitution that extends serum half-life as compared to the unsubstituted human IgG constant domain.

In certain embodiments, the IgG constant domain comprises substitutions at residues 252, 254, and 256, wherein the amino acid substitution at amino acid residue 252 is a substitution with tyrosine, the amino acid substitution at amino acid residue 254 is a substitution with threonine, and the amino acid substitution at amino acid residue 256 is a substitution with glutamic acid ("YTE"). See U.S. Pat. No. 7,083,784. In certain extended half-life embodiments, the IgG constant domain comprises substitutions selected from T250Q/M428L (Hinton et al., J. Immunology 176:346-356 (2006)); N434A (Yeung et al., J. Immunology 182:7663-7671 (2009)); or T307A/E380A/N434A (Petkova et al., International Immunology, 18: 1759-1769 (2006)).

In some embodiments, the elimination half-life of the anti-IL-6R antibody is increased by utilizing the FcRN-binding properties of human serum albumin. In certain embodiments, the antibody is conjugated to albumin (Smith et al., Bioconjug. Chem., 12: 750-756 (2001)). In some embodiments, the anti-IL-6R antibody is fused to bacterial albumin-binding domains (Stork et al., Prot. Eng. Design Science 20: 569-76 (2007)). In some embodiments, the anti-IL-6 antibody is fused to an albumin-binding peptide (Nguygen et al., Prot Eng Design Sel 19: 291-297 (2006)). In some embodiments, the anti-IL-antibody is bispecific, with one specificity being to IL-6R, and one specificity being to human serum albumin (Ablynx, WO 2006/122825 (bispecific Nanobody)).

In some embodiments, the elimination half-life of the anti-IL-6R antibody is increased by PEGylation (Melmed et al., Nature Reviews Drug Discovery 7: 641-642 (2008)); by HPMA copolymer conjugation (Lu et al., Nature Biotechnology 17: 1101-1104 (1999)); by dextran conjugation (Nuclear Medicine Communications, 16: 362-369 (1995)); by conjugation with homo-amino-acid polymers (HAPs; HAPylation) (Schlapschy et al., Prot Eng Design Sel 20: 273-284 (2007)); or by polysialylation (Constantinou et al., Bioconjug. Chem. 20: 924-931 (2009)).

In certain embodiments, the anti-IL-6R antibody or antigen-binding portion thereof comprises all six CDRs of tocilizumab. In particular embodiments, the antibody or antigen-binding portion thereof comprises the tocilizumab heavy chain V region and light chain V region. In specific embodiments, the antibody is the full-length tocilizumab antibody.

In certain embodiments, the anti-IL-6R antibody or antigen-binding portion thereof comprises all six CDRs of sarilumab. In particular embodiments, the antibody or antigen-binding portion thereof comprises the sarilumab heavy chain V region and light chain V region. In specific embodiments, the antibody is the full-length sarilumab antibody.

In certain embodiments, the anti-IL-6R antibody or antigen-binding portion thereof comprises all six CDRs of VX30 (Vaccinex), ARGX-109 (arGEN-X), FM101 (Formatech), , SA237 (Roche), NI-1201 (NovImmune), or an antibody described in US 2012/0225060.

In certain embodiments, the anti-IL-6R antibody or antigen-binding portion thereof is a single domain antibody. In particular embodiments, the single domain antibody is a camelid VHH single domain antibody. In specific embodiments, the antibody is vobarilizumab (ALX-0061) (Ablynx NV).

### 5.7.3. Anti-IL-6:IL-6R Complex Antibodies

In various embodiments, the IL-6 antagonist is an antibody specific for the complex of IL-6 and IL-6R. In certain embodiments, the antibody has the six CDRs of an antibody selected from those described in US 2011/0002936.

### 5.7.4. JAK and STAT Inhibitors

IL-6 is known to signal via the JAK-STAT pathway.

In various embodiments, the IL-6 antagonist is an inhibitor of the JAK signaling pathway. In some embodiments, the JAK inhibitor is a JAK1-specific inhibitor. In some embodiments, the JAK inhibitor is a JAK3-specific inhibitor. In some embodiments, the JAK inhibitor is a pan-JAK inhibitor.

In certain embodiments, the JAK inhibitor is selected from the group consisting of tofacitinib (Xeljanz), decernotinib, ruxolitinib, upadacitinib, baricitinib, filgotinib, lestaurtinib, pacritinib, peficitinib, INCB-039110, ABT-494, INCB-047986 and AC-410.

In various embodiments, the IL-6 antagonist is a STAT3 inhibitor. In a specific embodiment, the inhibitor is AZD9150 (AstraZeneca, Isis Pharmaceuticals), a STAT3 antisense molecule.

### 5.7.5. Additional IL-6 Antagonists

In various embodiments, the IL-6 antagonist is an antagonist peptide.

In certain embodiments, the IL-6 antagonist is C326 (an IL-6 inhibitor by Avidia, also known as AMG220), or FE301, a recombinant protein inhibitor of IL-6 (Ferring International Center S.A., Conaris Research Institute AG). In some embodiments, the anti-IL-6 antagonist comprises soluble gp130, FE301 (Conaris/Ferring).

### 5.8. Dosage regimens

### 5.8.1. Antibodies, antigen-binding fragments, peptides

In typical embodiments, antibody, antigen-binding fragments, and peptide IL-6 antagonists are administered parenterally.

In some parenteral embodiments, the IL-6 antagonist is administered intravenously. In certain intravenous embodiments, the IL-6 antagonist is administered as a bolus. In certain intravenous embodiments, the IL-6 antagonist is administered as an infusion. In certain intravenous embodiments, the IL-6 antagonist is administered as a bolus followed by infusion. In some parenteral embodiments, the IL-6 antagonist is administered subcutaneously.

In various embodiments, the antibody, antigen-binding fragment, or peptide IL-6 antagonist is administered in a dose that is independent of patient weight or surface area (flat dose).

In some embodiments, the intravenous flat dose is 1 mg, 2 mg, 3 mg, 4 mg, 5 mg, 6 mg, 7 mg, 8 mg, 9 mg, or 10 mg. In some embodiments, the intravenous flat dose is 11 mg, 12 mg, 13 mg, 14 mg, 15 mg, 16 mg, 17 mg, 18 mg, 19 mg, or 20 mg. In some embodiments, the intravenous flat dose is 25 mg, 30 mg, 40 mg, or 50 mg. In some embodiments, the intravenous flat dose is 60 mg, 70 mg, 80 mg, 90 mg, or 100 mg. In some embodiments, the intravenous flat dose is 1 - 10 mg, 10 - 15 mg, 15 - 20 mg, 20 - 30 mg, 30 - 40 mg, or 40 - 50 mg. In some embodiments, the intravenous flat dose is 1 - 40 mg, or 50 - 100 mg.

In some embodiments, the subcutaneous flat dose is 10 mg, 20 mg, 30 mg, 40 mg, 50 mg, 60 mg, 70 mg, 80 mg, 90 mg, or 100 mg. In some embodiments, the subcutaneous flat dose is 110 mg, 120 mg, 130 mg, 140 mg, 150 mg, 160 mg, 170 mg, 180 mg, 190 mg, or 200 mg. In some embodiments, the subcutaneous flat dose is 210 mg, 220 mg, 230 mg, 240 mg, or 250 mg. In some embodiments, the subcutaneous flat dose is 10 - 100 mg, 100 - 200 mg, or 200 - 250 mg. In some embodiments, the subcutaneous flat dose is 10 - 20 mg, 20 - 30 mg, 30 - 40 mg, 40 - 50 mg, 50 - 60 mg, 60 -70 mg, 70 - 80 mg, 80 - 90 mg, or 90 - 100 mg. In some embodiments, the subcutaneous flat dose is 100 - 125 mg, 125 - 150 mg, 150 - 175 mg, 175 - 200 mg, or 200 - 250 mg.

In various embodiments, the antibody, antigen-binding fragment, or peptide IL-6 antagonist is administered as a patient weight-based dose.

In some embodiments, the antagonist is administered at an intravenous dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg. In some embodiments, the antagonist is administered at a dose of 1.5 mg/kg, 2 mg/kg, 2.5 mg/kg, 3 mg/kg, 3.5 mg/kg, 4 mg/kg, 4.5 mg/kg, or 5 mg/kg.

In some embodiments, the subcutaneous weight-based dose is 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg. In some embodiments, the antagonist is administered at a dose of 1.5 mg/kg, 2 mg/kg, 2.5 mg/kg, 3 mg/kg, 3.5 mg/kg, 4 mg/kg, 4.5 mg/kg, or 5 mg/kg.

In various intravenous embodiments, the IL-6 antagonist is administered once every 7 days, once every 14 days, once every 21 days, once every 28 days, or once a month. In various subcutaneous embodiments, the IL-6 antagonist is administered once every 14 days, once every 28 days, once a month, once every two months (every other month), or once every three months.

In certain preferred embodiments, the IL-6 antagonist is the MEDI5117 antibody. In various embodiments, MEDI5117 is administered in a flat dose of 1 - 30 mg IV once every week. In certain embodiments, the MEDI5117 antibody is administered in a flat dose of 1, 2, 3, 4, 5, 7.5, 10, 15, 20, 25, or 30 mg IV once every week. In some embodiments, the MEDI5117 antibody is administered in a flat dose of 25 - 250 mg s.c. once every month to once every three months. In particular embodiments, MEDI5117 is administered at a dose of 30 mg, 45 mg, 60 mg, 75 mg, 100 mg, 120 mg, 125 mg, 150 mg, 175 mg, 200 mg, 225 mg, 240 mg, or 250 mg s.c. once every month, once every two months, or once every 3 months.

In some embodiments, the IL-6 antagonist is tocilizumab. In various embodiments, tocilizumab is administered s.c. in a starting dose for patients ≥100 kg of 162 mg once every week. In some embodiments, tocilizumab is administered intravenously at a dose of 4 mg/kg once every 4 weeks followed by an increase to 8 mg/kg every 4 weeks based on clinical response.

### 5.8.2. JAK and STAT inhibitors

In typical embodiments, small molecule JAK inhibitors and STAT inhibitors are administered orally.

In various embodiments, the inhibitor is administered once or twice a day at an oral dose of 1 - 10 mg, 10 - 20 mg, 20 - 30 mg, 30 - 40 mg, or 40 - 50 mg. In some embodiments, the inhibitor is administered once or twice a day at a dose of 50 - 60 mg, 60 - 70 mg, 70 - 80 mg, 80 - 90 mg, or 90 - 100 mg. In some embodiments, the inhibitor is administered at a dose of 5, 10, 15, 20, 25, 30, 35, 40, 45, or 50 mg PO once or twice a day. In some embodiments, the inhibitor is administered at a dose of 75 mg PO QD or BID, 100 mg PO QD or BID.

In certain embodiments, the JAK inhibitor is tofacitinib, and is administered at a dose of 5 mg PO BID or 11 mg PO qDay,

In certain embodiments, the JAK inhibitor is decernotinib, and is administered at a dose of 25 mg, 50 mg, 100 mg, or 150 mg PO BID.

In certain embodiments, the inhibitor is ruxolitinib, and is administered at dose of 25 mg PO BID, 20 mg PO BID, 15 mg PO BID, 10 mg PO BID, or 5 mg PO BID.

### 5.9. Additional therapeutic agents

In various embodiments of the methods described herein, the method further comprises administration of a therapeutic agent additional to the IL-6 antagonist, wherein the second therapeutic agent is also capable of reducing hepcidin expression.

In some embodiments, the second therapeutic agent is a BMP antagonist. In certain embodiments, the BMP antagonist is an anti-BMP6 antibody. In particular embodiments, the anti-BMP6 antibody has the six CDRs of an antibody described in US 2016/0176956, or US 2016/0159896.

In certain embodiments, the second therapeutic agent is a hemojuvelin antagonist. In particular embodiments, the hemojuvelin antagonist is an anti-hemojuvelin antibody. In specific embodiments, the anti-hemojuvelin antibody has the six CDRs of the antibodies disclosed in Kovac et al., Haematologica (2016) doi: 10.3324/ haematol.2015.140772 [ePub ahead of print].

In certain embodiments, the second therapeutic agent is a hepcidin antagonist. In particular embodiments, the hepcidin antagonist is an anti-hepcidin antibody. In specific embodiments, the antibody has the six CDRs from an antibody described in US 2016/0017032.

### 5.10. Kits

In another aspect, kits are provided.

In typical embodiments, the kits provide reagents for determining, from a biological sample obtained from a patient, the patient's genotype at the location of the *TMPRSS6* SNP rs855791.

### 5.11. Additional Aspects And Embodiments

### 5.11.1. Methods Of Treating Inflammation In Chronic Kidney Disease Or Cardiovascular Disease

In other aspects and embodiments, compositions and methods are provided for characterizing and treating inflammation in chronic kidney disease or cardiovascular disease with an IL-6 antagonist, as well as methods for characterizing the responsiveness of a patient to treatment.

These aspects and embodiments are based, at least in part, on the discovery that inflammation in chronic kidney disease patients and cardiovascular disease patients having one or more alleles of *TMPRSS6* comprising G or C at nucleotide position 2321 (encoding a *TMPRSS6* polypeptide comprising an alanine at amino acid position 736) caused these patients to be at higher risk of death, and that such subjects could be treated with an IL-6 antagonist to reduce this risk. As reported in more detail below, chronic kidney disease patients were genotyped and serum levels of IL-6 and CRP were assayed and these diagnostic data were compared to EPO dosage administered and risk of death. Patients having one or more alleles of *TMPRSS6* comprising G or C at nucleotide position 2321 (encoding a *TMPRSS6* polypeptide comprising an alanine at amino acid position 736), and elevated IL-6 and/or CRP levels, required higher doses of EPO for therapy and had higher mortality. The nucleotide at this position has been shown to be important in identifying patients with iron deficiency anemia (see Finberg et al., Nat. Genet. 2008; 40(5): 569-571). These data strongly support identification of a subset of patients based on the *TMPRSS6* genotype that required higher EPO dose and/or are at higher risk of death, and would likely respond to IL-6 inhibition with or without standard therapy for treatment of anemia (e.g., associated with chronic kidney disease). By inhibiting inflammation, EPO dosage can be reduced, thereby avoiding adverse side effects of EPO (e.g., cardiovascular risk).

These aspects and embodiments are further based on the discovery that patients having one or more alleles of *TMPRSS6* comprising G or C at nucleotide position 2321 (encoding a *TMPRSS6* polypeptide comprising an alanine at amino acid position 736) are at higher risk of death associated with myocardial infarction or cardiovascular disease. These patients would also likely benefit from IL-6 inhibition, which would reduce inflammation and the increased risk.

Accordingly, therapeutic methods are provided for treating inflammation associated with cardiovascular disease or chronic kidney disease, including anemia of chronic kidney disease and/or reducing the risk of death associated with such conditions by inhibiting IL-6 biological activity, for example, in patients selected by genotyping *TMPRSS6* at SNP rs855791, either by blocking IL-6 or its receptor (gp80) from binding to each other, or its signaling or expression (e.g., by anti-IL-6 antibody or by anti-IL-6R antibody or JAK1/STAT3 inhibition). In one embodiment, treatment of chronic kidney disease is carried out with or without standard treatment for anemia, as well as methods for characterizing the responsiveness of a patient suffering from chronic kidney disease to treatment for anemia, for example, by genotyping *TMPRSS6* at SNP rs855791 and detecting levels of inflammatory markers (e.g., increased IL-6 and/or CRP serum levels).

Methods are provided for treating cardiovascular disease or anemia of chronic kidney disease and/or reducing death associated with chronic inflammation in such patients by administering an agent that inhibits IL-6 biological activity or expression.

In some aspects and embodiments, compositions and methods are provided for treating chronic inflammation that contributes to mortality in subjects having chronic kidney disease or cardiovascular disease, and for characterizing the responsiveness of patients to such therapies. In particular embodiments, methods are provided for characterizing and treating chronic inflammatory anemia and mortality (e.g., in chronic kidney disease), as well as for characterizing the responsiveness of a patient to treatment for anemia (e.g., administration of erythropoietin or erythropoiesis-stimulating agents). In one aspect, methods of treating chronic inflammation in a selected subject are provided, the method comprising administering to the subject an IL-6 antagonist, wherein the subject is selected for treatment by having one or more alleles encoding a *TMPRSS6* polypeptide comprising an alanine at amino acid position 736.

In another aspect, methods are provided for treating inflammation or chronic inflammation in a selected subject having cardiovascular disease or chronic kidney disease, the method involving administering to the subject an IL-6 antagonist (e.g., anti-IL-6 antibody), wherein the subject is selected for treatment by having one or more alleles encoding a *TMPRSS6* polypeptide comprising an alanine at amino acid position 736. In one embodiment, the method reduces the subject's risk of mortality. In one embodiment, the subject has a history of myocardial infarction or heart failure.

In another aspect, methods of reducing inflammation and risk of mortality in a selected subject with cardiovascular disease or kidney disease are provided, the method comprising administering to the subject an IL-6 antagonist (e.g., anti-IL-6 antibody), wherein the subject is selected as having one or more alleles encoding a *TMPRSS6* polypeptide comprising an alanine at amino acid position 736 and increased inflammation relative to a reference. In one embodiment, the subject has a history of myocardial infarction or heart failure.

In another aspect, methods are provided for reducing the risk of mortality in a subject having chronic kidney disease or heart failure, the method comprising administering to the subject an IL-6 antagonist, wherein the subject is identified as having one or more alleles encoding a *TMPRSS6* polypeptide comprising an alanine at amino acid position 736 and increased inflammation relative to a reference.

In another aspect, methods of treating anemia in a subject are provided, the method involving administering to the subject an IL-6 antagonist alone or in combination with a therapy for anemia, where the subject is identified as having one or more alleles encoding a *TMPRSS6* polypeptide (also termed Matriptase-2; MT2) comprising an alanine at amino acid position 736 (e.g., having a G or C at nucleotide position 2321 of a *TMPRSS6* nucleic acid molecule) and increased inflammation relative to a reference.

In another aspect, methods of treating anemia in a subject having increased inflammation are provided, the method involving administering an IL-6 antagonist (e.g., IL-6 antibody) alone or in combination with an erythropoietic factor in an amount effective to neutralize inflammation in a subject having one or more alleles encoding a *TMPRSS6* polypeptide comprising an alanine at amino acid position 736 (e.g., having a G or C at nucleotide position 2321 of a *TMPRSS6* nucleic acid molecule).

In still another aspect, methods of enhancing responsiveness to EPO in a subject identified as in need thereof are provided, the method comprising administering an IL-6 antagonist (e.g., IL-6 antibody) in an amount effective to neutralize inflammation in a subject having one or more alleles encoding a *TMPRSS6* polypeptide comprising an alanine at amino acid position 736 (e.g., having a G or C at nucleotide position 2321 of a *TMPRSS6* nucleic acid molecule), thereby decreasing the EPO dose.

In another aspect, methods of reducing mortality in a subject having increased inflammation are provided, the method involving administering an IL-6 antagonist in an amount effective to neutralize inflammation in a subject having one or more alleles encoding a *TMPRSS6* polypeptide comprising an alanine at amino acid position 736 (e.g., having a G or C at nucleotide position 2321 of a *TMPRSS6* nucleic acid molecule).

In yet another aspect, methods of selecting therapy for a subject identified as in need thereof are provided, the method involving: characterizing the subject having one or more alleles encoding a *TMPRSS6* polypeptide comprising an alanine at amino acid position 736 (e.g., having a G or C at nucleotide position 2321 of a *TMPRSS6* nucleic acid molecule); and detecting the level of one or more inflammatory markers IL-6 or CRP, where the characterization indicates that an IL-6 antagonist should be administered, alone or in combination with a therapy for anemia.

In yet another aspect, methods are provided for increasing the proliferation or survival of a red blood cell or progenitor thereof (e.g., hematopoietic stem cell, proerythroblast, erythroblast, or reticulocyte) in a subject identified as in need thereof, the method comprising administering to the subject an IL-6 antagonist and an erythropoietic factor, where the subject is identified as having one or more alleles encoding a *TMPRSS6* polypeptide comprising an alanine at amino acid position 736 (e.g., having a G or C at nucleotide position 2321 of a *TMPRSS6* nucleic acid molecule) and increased inflammation relative to a reference.

In various embodiments of any of the aspects delineated herein, the subject has or is identified as having anemia, including cancer anemia, anemia in chronic autoimmune diseases, anemia in chronic inflammatory diseases, anemia in cardiovascular diseases, anemia in metabolic syndromes, and the like. In various embodiments of any of the aspects delineated herein, the subject has or is identified as having chronic kidney disease. In various embodiments of any of the aspects delineated herein, the subject has or is identified as having inflammation. In various embodiments of any of the aspects delineated herein, the subject has or is identified as having an increased risk of death associated with chronic inflammation, chronic kidney disease, or cardiovascular disease. In various embodiments of any of the aspects delineated herein, the subject is identified as in need of treatment. In various embodiments of any of the aspects delineated herein, the subject has or is identified as having increased inflammation. In various embodiments of any of the aspects delineated herein, the subject has or is identified as having one or more alleles encoding a *TMPRSS6* polypeptide comprising an alanine at amino acid position 736 (e.g., having a G or C at nucleotide position 2321 of a *TMPRSS6* nucleic acid molecule) and increased inflammation relative to a reference. In various embodiments of any of the aspects delineated herein, the method comprises administering to the subject an IL-6 antagonist. In various embodiments of any of the aspects delineated herein, the method comprises administering to the subject an IL-6 antagonist and a therapy for anemia. In various embodiments of any of the aspects delineated herein, the subject is human.

In various embodiments of any of the aspects delineated herein, the therapy for anemia comprises administering an erythropoietic factor. In various embodiments, the erythropoietic factor is one or more of erythropoietin, erythropoiesis-stimulating agent, HIF stabilizer, and supplemental iron.

In various embodiments, increased inflammation is characterized by increased levels of IL-6 and/or CRP, relative to a reference (e.g., as measured by conventional CRP assays or high sensitivity assays (hsCRP), which both detect CRP, but differ in analytical performance). In various embodiments, increased inflammation is characterized as IL-6 greater than about 5 pg/ml. In various embodiments, increased inflammation is characterized as CRP greater than about 2 mg/L.

In various embodiments of any of the aspects delineated herein, the IL-6 antagonist is administered in an amount effective to neutralize inflammation. In various embodiments, the amount effective to neutralize inflammation reduces IL-6 to less than about 15 pg/ml, less than about 10 pg/ml, or less than about 5 pg/ml. In various embodiments, the amount effective to neutralize inflammation reduces CRP to less than about 2 mg/L or less than about 0.2 mg/L.

In various embodiments of any of the aspects delineated herein, administering an IL-6 antagonist or anti-IL-6 antibody reduces the dose of EPO. In certain embodiments, the dose of EPO is reduced about 40 IU/kg/week, about 50 IU/kg/week, about 80 IU/kg/week, about 100 IU/kg/week or more. In various embodiments, administering an IL-6 antagonist or anti-IL-6 antibody reduces a side effect of increased EPO dose.

In one embodiment, patients with chronic kidney disease are treated with or without standard treatment for anemia. In particular, an agent that inhibits IL-6 biological activity or expression is provided to a subject having anemia associated with chronic kidney disease with or without a treatment for anemia (e.g., administration of EPO, ESA, HIF stabilizers, supplemental iron, or red cell transfusion). Treatments for anemia work by stimulating erythropoiesis or red blood cell production. Thus, agents that increase the growth or proliferation and/or decrease cell death of a red blood cell or progenitor thereof. Red blood cell progenitors include for example, hematopoietic stem cells, common myeloid progenitors, proerythroblasts, erythroblasts, reticulocytes, or any cell capable of differentiating or maturing into a red blood cell.

An agent that inhibits IL-6 biological activity either by blocking IL-6 or its receptor (gp80) from binding to each other, or its signaling or expression may be provided to a subject having anemia associated with chronic kidney disease in a pharmaceutical composition, where the pharmaceutical composition comprises an effective amount of the agent, an agent for treating anemia (e.g., EPO, ESA, HIF prolyl-hydroxylase inhibitors, supplemental iron) and a suitable excipient. In one embodiment, the agent is an IL-6 antagonist or anti-IL-6 antibody that decreases the level or activity of an IL-6 polypeptide or nucleic acid molecule in a subject, or inhibits intracellular signaling triggered by IL-6 receptor activation. An anti-IL-6 antibody (e.g., MEDI5117) may be administered in combination with a treatment for anemia (e.g., administration of EPO, ESA, HIF stabilizer, supplemental iron). Methods of treatment for anemia vary depending on the *TMPRSS6* genotype of the patient and the inflammatory state of the patient. Patients homozygous or heterozygous for the major allele of *TMPRSS6* comprising G or C at nucleotide position 2321 (encoding a *TMPRSS6* polypeptide comprising an alanine at amino acid position 736) and having elevated levels of inflammatory markers (e.g., IL-6 and/or CRP) are administered an IL-6 antagonist or anti-IL-6 antibody that decreases the level or activity of an IL-6 polypeptide in the context of a treatment for anemia (e.g., administration of EPO, ESA, HIF stabilizer, supplemental iron). Patients homozygous for the minor allele of *TMPRSS6* comprising A or T at nucleotide position 2321 (encoding a *TMPRSS6* polypeptide comprising a valine at amino acid position 736) do not require an anti-IL-6 therapy to supplement treatment for anemia. Methods of treatment for anemia may vary depending on the stage of chronic kidney disease, the patient's age, health, and physical condition.

In another aspect, assays are provided that are useful for characterizing a subject having anemia associated with chronic inflammation (e.g., in chronic kidney disease). Inflammatory markers IL-6 and CRP can be detected by any suitable method. The methods described herein can be used individually or in combination for detection of an IL-6 or CRP biomarker and/or inflammatory condition. In one embodiment, inflammation is characterized by detecting the level of IL-6 and/or CRP polypeptide in a biological sample (e.g., serum) of the subject relative to the expression in a reference (e.g., serum from a healthy control subject), where an increase in IL-6 and/or CRP expression is indicative of inflammation. In another embodiment, an increase in IL-6 and/or CRP expression is indicative that a subject having anemia associated with chronic kidney disease will not be responsive to treatment for anemia and/or will be responsive to treatment for anemia when administered in combination with an IL-6 antagonist (e.g., an anti-IL-6 antibody).

In one embodiment, an IL-6 and/or CRP polypeptide level is measured by immunoassay. Immunoassay typically utilizes an antibody (or other agent that specifically binds the marker) to detect the presence or level of a biomarker in a sample. Antibodies can be produced by methods well known in the art, e.g., by immunizing animals with the biomarker or fragments thereof. Biomarkers can be isolated from samples based on their binding characteristics. Alternatively, if the amino acid sequence of a polypeptide biomarker is known, the polypeptide can be synthesized and used to generate antibodies by methods well known in the art.

In various embodiments, traditional immunoassays are used, including, for example, Western blot, sandwich immunoassays including ELISA and other enzyme immunoassays, fluorescence-based immunoassays, and chemiluminescence. Nephelometry is an assay done in liquid phase, in which antibodies are in solution. Binding of the antigen to the antibody results in changes in absorbance, which is measured. Other forms of immunoassay include magnetic immunoassay, radioimmunoassay, and real-time immunoquantitative PCR (iqPCR). Other methods of detection include liquid chromatography and mass spectrometry.

Immunoassays can be carried out on solid substrates (e.g., chips, beads, microfluidic platforms, membranes) or on any other forms that supports binding of the antibody to the marker and subsequent detection. A single marker may be detected at a time or a multiplex format may be used. Multiplex immunoanalysis may involve planar microarrays (protein chips) and bead-based microarrays (suspension arrays).

Chronic kidney disease patients having anemia identified as having increased IL-6 and/or CRP polypeptide levels are selected for treatment with an agent that reduces IL-6 expression or activity (e.g., anti-IL-6 antibody) in combination with a treatment for anemia. Patients treated may be monitored by detecting alterations in hemoglobin, hematocrit, erythropoietin dose, IL-6 and/or CRP expression following treatment. Patients showing a reduction in IL-6 and/or CRP expression and/or a reduction in inflammation are identified as responsive to IL-6 inhibition.

### 5.11.2. Methods For Treating Cardiorenal Syndrome

In other aspects and embodiments, compositions and methods for treating cardiorenal syndrome are provided.

These aspects and embodiments are based, at least in part, on the discovery that anti-IL-6 treatment of heart injury in a rodent model of cardiorenal syndrome had an equivalent effect as standard of care treatment. As reported in more detail below, rodent models of cardiorenal syndrome were treated with anti-IL-6 or standard of care therapy (ACE inhibitor, perindopril) following a myocardial infarction. Following treatment, ejection fraction, force of cardiac contractility, and percentage of fibrotic tissue in heart tissue were measured. Levels of ejection fraction in both subject groups treated with anti-IL-6 and subject groups treated with standard of care therapy were increased compared to levels in a subject group treated with a control treatment. Cardiac contractility in both groups treated with anti-IL-6 and treated with standard of care therapy were increased compared to levels in a subject group treated with a control treatment. The amounts of fibrotic tissue in both groups treated with anti-IL-6 and treated with standard of care therapy were decreased compared to the amount in a subject group treated with a control treatment. Further, levels of ejection fraction and amount of fibrotic tissue were similar in the subject group treated with anti-IL-6 and the subject group treated with standard of care therapy. The results demonstrate that anti-IL-6 therapy had equivalent efficacy as standard of care therapy in treating cardiorenal syndrome in a rodent model.

These aspects and embodiments are further based, at least in part, on the discovery that patients identified as having cardiorenal syndrome following myocardial infarction and having elevated levels of IL-6 had particularly increased risk of cardiovascular death, including heart failure. Without being bound by theory, IL-6 may play a causal role in the development and/or progression of cardiorenal syndrome. Thus, patients having elevated levels of IL-6 following a myocardial infarction or patients having cardiorenal syndrome and elevated levels of IL-6 will likely benefit from IL-6 inhibition.

Accordingly, therapeutic methods are provided for treating a heart and/or kidney injury in a subject having a cardiorenal syndrome, involving administering to the subject an IL-6 antagonist. In some embodiments, treatment of a heart and/or kidney injury in a subject having a cardiorenal syndrome is carried out with or without standard treatment for cardiorenal syndrome. Methods are also provided for characterizing the risk of cardiovascular death in a patient following a myocardial infarction, the method involving detecting an increased level of IL-6 in a biological sample obtained from the patient.

In one aspect, methods of treating a heart and/or kidney injury in a subject having cardiorenal syndrome are provided, the method involving administering to the subject an IL-6 antagonist.

In another aspect, methods of increasing cardiac function in a subject having cardiorenal syndrome are provided, the method involving administering to the subject an IL-6 antagonist.

In still another aspect, methods of reducing fibrosis in a subject having cardiorenal syndrome are provided, the method involving administering to the subject an IL-6 antagonist.

In various embodiments of any of the aspects delineated herein, the method further involves administering a standard of care therapy to the subject. In various embodiments, the standard of care therapy is an angiotensin converting enzyme (ACE) inhibitor.

In various embodiments of any of the aspects delineated herein, the increase in cardiac function is characterized by an increase in the subject's ejection fraction and/or force of cardiac contractility, relative to a reference. In various embodiments of any of the aspects delineated herein, the reduction in fibrosis is characterized by a decrease in percentage of fibrotic tissue in a tissue sample from the subject, relative to a reference. In various embodiments, the fibrosis is in heart tissue.

In various embodiments of any of the aspects delineated herein, the subject has heart and/or kidney injury. In various embodiments of any of the aspects delineated herein, the subject has a heart injury followed by a kidney injury.

In another aspect, the disclosure provides a method of identifying an increased risk of cardiovascular death (e.g., heart failure) in a subject after a myocardial infarction in the subject, the method involving measuring a level of one or more of an IL-6 polynucleotide or polypeptide in a sample from the subject relative to a reference, where an increased level of one or more of an IL-6 polynucleotide or polypeptide indicates an increased risk of cardiovascular death.

In still another aspect, the disclosure provides a method of characterizing risk of cardiovascular death (e.g., heart failure) in a subject after a myocardial infarction in the subject, the method involving measuring a level of one or more of an IL-6 polynucleotide or polypeptide in a sample from the subject relative to a reference, where an increased level of one or more of an IL-6 polynucleotide or polypeptide indicates an increased risk of cardiovascular death.

In various embodiments of any of the aspects delineated herein, the subject has cardiorenal syndrome, heart failure, chronic kidney disease, or no cardiorenal pathology. In various embodiments of any of the aspects delineated herein, the subject is identified as having cardiorenal syndrome, heart failure, chronic kidney disease, or no cardiorenal pathology about one month after the myocardial infarction.

In another aspect, the disclosure provides a method of treating a heart and/or kidney injury in a selected subject having cardiorenal syndrome, the method involving administering to the subject an IL-6 antagonist, where the subject is selected for treatment by detecting an increased level of one or more of an IL-6 polynucleotide or polypeptide in a biological sample from the subject relative to a reference.

In still another aspect, the disclosure provides a method of decreasing risk of cardiovascular death (e.g., heart failure) in a selected subject having cardiorenal syndrome, the method involving administering to the subject an IL-6 antagonist, where the subject is selected by detecting an increased level of one or more of an IL-6 polynucleotide or polypeptide in a biological sample from the subject relative to a reference. In various embodiments of any of the aspects delineated herein, the subject has had a myocardial infarction.

In various embodiments of any of the aspects delineated herein, the IL-6 antagonist is an anti-IL-6 antibody. In various embodiments, the anti-IL-6 antibody is MEDI5117.

In various embodiments of any of the aspects delineated herein, the biological sample is a plasma sample or serum sample. In various embodiments of any of the aspects delineated herein, the subject is human.

In another aspect, methods are provided for treating cardiorenal syndrome in patients and/or reducing risk of death or heart failure in such patients by administering an agent that inhibits IL-6 biological activity or expression. In one embodiment, patients with cardiorenal syndrome are treated with or without standard treatment for cardiorenal syndrome (e.g., angiotensin converting enzyme (ACE) inhibitors). In particular, an agent that inhibits IL-6 biological activity or expression is provided to a subject having cardiorenal syndrome (e.g., administration of anti-IL-6 antibody).

In another aspect, methods of increasing cardiac function and methods of reducing fibrosis in subjects having cardiorenal syndrome are provided. The methods comprise administering an agent that inhibits IL-6 biological activity or expression to the subject. In some embodiments, the increase in cardiac function is characterized by an increase in the subject's ejection fraction relative to a reference (e.g., ejection fraction of a healthy control subject) or cardiac contractility (e.g., dP/dtₘₐₓ) relative to a reference (e.g., cardiac contractility of a healthy control subject). In some embodiments, the reduction in fibrosis is characterized by a decrease in percentage of fibrotic tissue in a tissue sample from the subject, relative to a reference (e.g., tissue sample obtained from a healthy control subject). In one embodiment, the fibrosis is in heart tissue.

An agent that inhibits IL-6 biological activity either by blocking IL-6 or its receptor (gp80) from binding to each other, or its signaling or expression may be provided to a subject having cardiorenal syndrome in a pharmaceutical composition, where the pharmaceutical composition comprises an effective amount of the agent and a suitable excipient. In one embodiment, the agent is an IL-6 antagonist or anti-IL-6 antibody that decreases the level or activity of an IL-6 polypeptide or polynucleotide in a subject, or inhibits intracellular signaling triggered by IL-6 receptor activation. An anti-IL-6 antibody (e.g., MEDI5117) may be administered. Methods of treatment for cardiorenal syndrome may vary depending on the stage of cardiorenal syndrome, the patient's age, health, and physical condition.

In various embodiments, subjects having cardiorenal syndrome are treated with an IL-6 antagonist. Further, subjects having increased risk of cardiovascular death and/or heart failure following a myocardial infarction may be identified by characterizing the plasma level of IL-6 in the subject. Subjects having elevated IL-6 levels have increased risk of cardiovascular death and/or heart failure. Such subjects may be selected for treatment with an IL-6 antagonist. Additionally, subjects having cardiorenal syndrome and increased IL-6 levels, including such subjects that have suffered a myocardial infarction, may be selected for treatment. Once selected for treatment, such subjects may be administered virtually any IL-6 antagonist known in the art. Suitable IL-6 antagonists include, for example, known IL-6 antagonists, commercially available IL-6 antagonists, IL-6 antagonists developed using methods well known in the art, and antagonists to the intracellular signaling systems associated with IL-6R.

In another aspect, assays are provided for characterizing the risk of cardiovascular death, heart failure, and/or mortality in a subject following a myocardial infarction. The assays feature detection of IL-6 in a biological sample obtained from the subject. IL-6 can be detected by any suitable method. In one embodiment, risk of cardiovascular death or heart failure is characterized by detecting the level of an IL-6 polypeptide in a biological sample (e.g., serum or plasma) of the subject relative to the expression in a reference (e.g., serum or plasma from a healthy control subject or from a control subject with no cardio-renal pathology), where an increase in IL-6 is indicative of increased risk of cardiovascular death or heart failure. Subjects identified as having increased risk of cardiovascular death, heart failure, or mortality may be selected for treatment. In another embodiment, a subject having cardiorenal syndrome and increased IL-6 levels is selected for treatment with an IL-6 antagonist (e.g., an anti-IL-6 antibody).

In one embodiment, an IL-6 polynucleotide level is measured. Levels of IL-6 polynucleotides may be measured by standard methods, such as quantitative PCR, Northern Blot, microarray, mass spectrometry, and in situ hybridization.

In one embodiment, an IL-6 polypeptide level is measured. Levels of IL-6 polypeptides may be measured by standard methods, such as by immunoassay. Immunoassay typically utilizes an antibody (or other agent that specifically binds the marker) to detect the presence or level of a biomarker in a sample. Antibodies can be produced by methods well known in the art, e.g., by immunizing animals with the biomarker or fragments thereof. Biomarkers can be isolated from samples based on their binding characteristics. Alternatively, if the amino acid sequence of a polypeptide biomarker is known, the polypeptide can be synthesized and used to generate antibodies by methods well known in the art.

In various embodiments, the assay employs traditional immunoassays including, for example, Western blot, sandwich immunoassays including ELISA and other enzyme immunoassays, fluorescence-based immunoassays, and chemiluminescence. Nephelometry is an assay done in liquid phase, in which antibodies are in solution. Binding of the antigen to the antibody results in changes in absorbance, which is measured. Other forms of immunoassay include magnetic immunoassay, radioimmunoassay, and real-time immunoquantitative PCR (iqPCR). Other methods of detection include liquid chromatography and mass spectrometry.

Immunoassays can be carried out on solid substrates (e.g., chips, beads, microfluidic platforms, membranes) or on any other forms that supports binding of the antibody to the marker and subsequent detection. A single marker may be detected at a time or a multiplex format may be used. Multiplex immunoanalysis may involve planar microarrays (protein chips) and bead-based microarrays (suspension arrays).

Cardiorenal syndrome patients identified as having increased IL-6 polypeptide levels are selected for treatment with an agent that reduces IL-6 expression or activity (e.g., anti-IL-6 antibody). The treatment may be administered in combination with a standard treatment for cardiorenal syndrome (e.g., an ACE inhibitor). Patients treated with a method of the disclosure may be monitored by detecting alterations in IL-6 following treatment.

### 5.12. Examples

The following examples are provided by way of illustration, not limitation.

### 5.12.1. Example 1: EPO dosage and overall survival in chronic kidney disease patients is correlated with serum IL-6 and CRP levels only in patients with at least one copy of the TMPRSS6SNP rs855791 major allele

The peptide hormone, hepcidin, plays a central role in systemic iron homeostasis. Hentze et al., Cell 142:24-38 (2010). Hepcidin expression is known to be influenced by the product of the *TMPRSS6* gene, matriptase-2, a type II transmembrane serine protease. Common variants in the *TMPRSS6* gene have been shown to correlate with iron status, Benyamin et al., Nature Genetics 41(11):1173-1175 (2009), and certain mutations in the *TMPRSS6* gene have been shown to cause iron-refractory iron deficiency anemia (IRIDA), Finberg et al., Nature Genetics 40(5):569-571 (2008). SNP rs855791 (2321G→A; A736V) is a naturally occurring variation in the *TMPRSS6* gene that has been associated with naturally occurring variations in hepcidin expression and blood hemoglobin levels.

To determine whether the genotype at the *TMPRSS6* rs855791 SNP predicted extent of anemia in end stage renal disease, data previously collected in clinical studies of patients with chronic kidney disease were analyzed in conjunction with newly determined SNP genotyping. Because hepcidin expression is also regulated by IL-6, Casanovas et al., PLOS Computational Biol. 10(1): e1003421 (2014), the data were also analyzed to determine whether serum IL-6 levels could predict extent of anemia in end stage renal disease.

### Methods

Data from N=257 patients enrolled in the MIMICK1, MIMICK2 (Mapping of Inflammatory Markers in Chronic Kidney Disease) and MIA (malnutrition, inflammation and atherosclerosis) cohorts who were recruited during the period of October 2003-September 2004 in six dialysis units in the Stockholm-Uppsala (Sweden) region was curated to N=208 based on criteria of prevalent dialysis, ferritin >100 ng/ml, and Hb >10 mg/dL to select patients who were stable on hemodialysis without iron deficiency and without marked anemia, thereby excluding patients with factors that might divorce iron handling from hemoglobin levels.

All patient clinical data, including erythropoietin (EPO) dose in IU/kg/week, IL-6 serum level in pg/ml, CRP serum level in mg/L, survival in months, and *TMPRSS6* genotype at SNP rs855791, was collated and analyzed using statistics analytics software (SPSS Statistics Desktop; IBM). The *TMPRSS6* alleles studied and their nucleotide and amino acid are indicated at Table 1.

| **Table 1** | | |
|---|---|---|
| *TMPRSS6* alleles | | |
| *TMPRSS6* allele | Nucleotide at position 2321 | Amino acid at position 736 |
| Major | Gor C | Ala |
| Minor | A or T | Val |

The cohort was separated into rs855791 subgroups (homozygous AA, heterozygous AG, and homozygous GG), and each genotype group was separated into tertiles or quartiles of serum IL-6 level (e.g., IL-6 < 5pg/ml vs >10 pg/ml and IL-6 < 5pg/ml vs > 15pg/ml) or serum CRP level (CRP < 2 mg/L vs >2 mg/L). Comparisons were made between EPO dose in the top and bottom tertiles and quartiles. Statistician analysis within genotype groups by Students T-Test and between groups by ANOVA were conducted.

### Results

Because each patient's EPO dose had been titrated by the treating physicians to achieve normal hemoglobin levels, EPO dose could be used as a proxy for the underlying degree of anemia. EPO dose in subjects homozygous for the minor allele (A/A) were found to be relatively insensitive to variations in IL-6 (FIG. 1A; left panel). However, EPO dose in subjects with at least one copy of the major allele - patients heterozygous (A/G) or homozygous (G/G) for the major allele (G) - were sensitive to their IL-6 level (FIG. 1B; right panel). In these latter subjects, increased levels of serum IL-6 (e.g., >5 pg/ml) were associated with increased EPO dose.

Without being bound to a particular theory, homozygosity at the minor allele removed the influence of IL-6 on iron handling. Thus, EPO dose in these patients (A/A) was approximately the same regardless of the IL-6 level.

Subjects homozygous for the *TMPRSS6* rs855791 minor allele (A) showed similar mortality regardless of IL-6 levels (FIG. 2A). However, survival in subjects with at least one copy of the major allele - patients heterozygous or homozygous for the major allele (G) - varied according to IL-6 level (FIG. 2B). In fact, the G allele of *TMPRSS6* conferred a higher all-cause mortality in response to elevated IL-6 levels in chronic kidney disease stage 5 dialysis subjects. In subjects having at least one copy of the major allele (G), IL-6 levels ≥ 5pg/ml (i.e., middle and highest tertile IL-6) were associated with increased mortality compared to IL-6 levels < 5pg/ml (i.e., low tertile IL-6) (Figure 2B).

The level of the acute phase reactant, CRP, a marker of inflammation, also correlated with increased EPO dosage in subjects heterozygous or homozygous for the major allele (G), but not in patients homozygous for the minor allele (FIG. 3).

### Discussion

As shown in FIG. 1, the extent of underlying anemia - measured as the clinically-titrated EPO dose - correlated with IL-6 levels only in patients having at least one copy of the major allele at the *TMPRSS6* rs855791 SNP. In these patients, the higher the serum IL-6 level, the higher the required EPO dose (FIG. 1B). In contrast, the degree of anemia in patients having two copies of the minor allele was not correlated with serum IL-6 levels (FIG. 1A).

Analogously, overall survival correlated with IL-6 levels only in patients with at least one copy of the major allele at the *TMPRSS6* SNP rs855791. In subjects having at least one copy of the *TMPRSS6* rs855791 major allele, survival was inversely correlated with serum IL-6 level, with patients in the highest tertile of serum IL-6 levels having statistically significantly worse survival than those in the lowest tertile of IL-6 levels (FIG. 2B). In contrast, the overall survival of patients homozygous for the minor allele at rs855791 was unaffected by IL-6 levels (FIG. 2A).

Without intending to be bound by theory, in patients having at least one copy of the *TMPRSS6* major allele, increases in serum IL-6 may drive increased hepcidin expression, thereby increasing anemia. The increased mortality risk is a consequence of the dysregulated iron metabolism, the resulting anemia, and/or the increased dose of erythropoiesis stimulating agent, such as EPO. If these correlations reflect a causal relationship, they raise the possibility that reducing IL-6 levels or IL-6 signaling could reduce anemia, reduce required EPO dose, and increase survival in patients with chronic kidney disease, but only in those patients having at least one copy of the *TMPRSS6* rs855791 major allele, and with greatest effect in those patients having elevated serum levels of IL-6.

### 5.12.2. Example 2: Risk of death and risk of heart failure after acute myocardial infarction are correlated with IL-6 serum levels only in patients with at least one copy of the TMPRSS6SNP rs855791 major allele

To determine whether *TMPRSS6* rs855791 genotype affected IL-6 sensitivity in patients with acute rather than chronic disease, data previously collected in clinical studies of patients hospitalized for acute coronary syndrome were analyzed in conjunction with newly determined SNP genotyping.

### Methods

Data were analyzed from subjects previously enrolled in a multi-center Study of Platelet Inhibition and Patient Outcomes (PLATO). Patients were eligible for enrollment in PLATO if they had been hospitalized for an acute coronary syndrome with an onset of symptoms during the previous 24 hours. Mortality and presence of heart failure was measured in these subjects beginning thirty days following a myocardial infarction.

### Results

The mortality of subjects homozygous for the *TMPRSS6* rs855791 SNP minor allele (A) did not correlate with variations in IL-6 (FIG. 4A). However, one or two copies of the major allele (G) conferred a higher all-cause mortality in response to elevated IL-6 levels in subjects following myocardial infarction (FIG. 4B). Thus, *TMPRSS6* modulated IL-6 mediated risk of death following myocardial infarction.

The effect of *TMPRSS6* genotype on IL-6 mediated risk of heart failure was also measured in subjects enrolled in PLATO beginning thirty days post-myocardial infarction. Heart failure in subjects homozygous for the minor allele (A) did not correlate with variations in IL-6 (FIG. 5A). However, the G allele of *TMPRSS6* conferred a higher heart failure rate in response to elevated IL-6 levels in subjects following a myocardial infarction (FIG. 5B). Thus, *TMPRSS6* modulated IL-6 mediated risk of heart failure following myocardial infarction.

### Discussion

These data demonstrate that the correlation between *TMPRSS6* genotype, IL-6 levels, and adverse clinical outcomes is not limited to patients with chronic kidney disease. Without intending to be bound by theory, in patients having at least one copy of the *TMPRSS6* major allele, increases in serum IL-6 drive may drive increased hepcidin expression, with consequent increased sequestration of iron in cardiomyocytes, followed by iron-mediated cellular toxicity. If these correlations reflect a causal relationship, they raise the possibility that reducing IL-6 levels or IL-6 signaling could reduce heart failure and mortality in patients with acute coronary syndrome, but only in those patients having at least one copy of the *TMPRSS6* rs855791 major allele, and with greatest effect in those patients with elevated serum levels of IL-6.

### 5.12.3. Example 3: In vitro studies on iPS-derived human cardiomyocytes confirm a causal relationship between TMPRSS6 genotype and IL-6-mediated cytotoxicity

Although the correlations observed in Examples 1 and 2 imply that reducing IL-6 mediated signaling should provide clinical benefit in patients having at least one copy of the *TMPRSS6* rs855791 major allele, elevated levels of IL-6, and either anemia or a hepcidin-mediated cellular toxicity, the observed correlations fall short of proving a causal relationship. Accordingly, experiments were conducted in human induced pluripotent cell derived cardiac myocytes (iPS-CMs) transfected with variants of *TMPRSS6* to interrogate the effect of BMP and BMP plus IL-6 on hepcidin expression and cellular susceptibility to ischemic injury.

### 5.12.3.1. Methods

*Culturing of human iPS derived cardiomyocytes* - iCell cardiomyocytes (Cellular Dynamics International, CDI Inc.) were plated on 0.1% gelatin coated 6-well or 96-well cell culture plate with iCell Cardiomyocytes plating medium (CDI Inc.). Forty-eight hours after plating, plating medium was replaced with Maintenance Medium (CDI Inc.). Maintenance Medium was replaced every other day up to the day the experiment was performed.

*Simulated Ischemia*/*Reoxygenation Protocol* - The iPS cardiomyocytes were subjected to simulated ischemia (SI) for 90 min by replacing the cell medium with an "ischemia buffer" that contained 118 mm NaCl, 24 mm NaHCO₃, 1.0 mm NaH₂PO₄, 2.5 mm CaCl₂-2H₂O, 1.2 mm MgCl₂, 20 mm sodium lactate, 16 mm KCl, 10 mm 2-deoxyglucose (pH adjusted to 6.2) as reported previously (Das, A., Xi, L., and Kukreja, K. C. (2005) J. Biol. Chem. 280: 12944-12955; Das A, Smolenski A, Lohmann SM, Kukreja RC. (2006) J. Biol Chem. 281(50):38644-52). The cells were incubated at 37°C in tri-gas incubator adjusting 1-2% O₂ and 5% CO₂ during the entire SI period. Reoxygenation (RO) was accomplished by replacing the ischemic buffer with normal cell medium under normoxic conditions. Cell necrosis after 2 or 18 h of reoxygenation, respectively. iCells were subjected to 4 hr SI and 24 hr RO as above.

*Evaluation of Cell Viability and Apoptosis* - Trypan blue exclusion assay was performed to assess cell necrosis as reported previously (Das, A., Xi, L., and Kukreja, K. C. (2005) J. Biol. Chem. 280, 12944-12955; Das A, Smolenski A, Lohmann SM, Kukreja RC. (2006) J. Biol. Chem. 281(50):38644-52).

*Transfection of iCell Cardiomyocytes* - At day 8 post-plating, the medium was replaced with fresh Maintenance Medium and cells were incubated for 4 hrs. Cells were transfected with pCMV6-XL5 TMPRSS6 (K523) or pCMV6-XL5 TMPRSS6 (K523) V763A using ViaFect™ Transfection Reagent according to the manufacture's instruction (Promega Corp., Madison, WI). After 48 hr of transfection, cells were subjected to further experiments.

*Western Blot Analysis* - Western blots were performed as described previously (Das, A., Xi, L., and Kukreja, K. C. (2005) J. Biol. Chem. 280, 12944-12955; Das A, Smolenski A, Lohmann SM, Kukreja RC. (2006) J. Biol. Chem. 281(50):38644-52). Total soluble protein was extracted from the cells with lysis buffer (Cell Signaling, MA). The homogenate was centrifuged at 10,000 × g for 5 min at 4°C, and the supernatant was recovered. Protein (50 µg of from each sample) was separated by 12% acrylamide gels and transferred to nitrocellulose membrane and then blocked with 5% nonfat dry milk in TBST (10 mm Tris-HCl, pH 7.4, 100 mm NaCl, and 0.1% Tween 20) for 1 h. The membrane was then incubated overnight with rabbit monoclonal/polyclonal or goat polyclonal primary antibody at a dilution of 1:1000 for each of the respective proteins, *i.e.* Phospho-Beclin-1 (Ser93) (D9A5G) Rabbit mAb, Beclin-1, SQSTM1/p62, LC3A/B (D3U4C) XP® Rabbit mAb, Phospho-Akt (Ser473) (D9E) XP® Rabbit mAb, Akt (pan) (C67E7) Rabbit mAb, Phospho-S6 Ribosomal Protein (Ser240/244) (D68F8) XP® Rabbit mAb, S6 Ribosomal Protein (5G10) Rabbit mAb from Cell Signaling, MA, Anti-Matriptase 2 (TMPRSS6) and Anti- SLC40A1(Ferroportin) from Abcam Company, MA and goat polyclonal Actin-HRP (Santa Cruz Biotechnology, TX). The membranes were then incubated with anti-rabbit horseradish peroxidase-conjugated secondary antibody (1:2000 dilution; Amersham Biosciences) for 2 h. The blots were developed using a chemiluminescent system, and the bands were scanned and quantified by densitometry analysis.

*Real time PCR- Taqman assay* - Total RNA including small RNA was isolated using miRNeasy mini kit according to manufacturer's protocol (QIAGEN Sciences, MD, USA). Concentration and purity of the isolated RNA was measured using Nanodrop ND-1000 spectrophotometer (Agilent technologies, CA, USA). Briefly 1 µg of total RNA was converted to cDNA with random hexamer using high capacity cDNA synthesis kit (Applied Biosystems, CA, USA). Reverse transcription reaction was carried out using the following PCR conditions: 25°C for 10 min; 37°C for 120 min and 85°C for 5 min. Real-time PCR was performed using Taqman amplicon specific probes (Applied Biosystems, CA, USA) Hamp (CGGCTCTGCAGCCTTG) (SEQ ID NO:20) under the following PCR cycle condition: 95°C for 10 minutes; 95°C for 15 seconds and 60°C for 60 seconds. The expression of Hamp was normalized to GAPDH (CTTCCAGGAGCGAGATCCCGCTAA) (SEQ ID NO:21) housekeeping gene. The relative gene expression was analyzed using the 2-ΔΔCt method.

*TMPRSS6 mutagenesis and transfection of iPS cells* - pCMV6-XL5 TMPRSS6 was purchased from Origene Technologies (Rockville, MD), catalog number SC306623 corresponding to GenBank accession number NM_153609. This clone contains a mutation resulting in an amino acid change, K253A. Site directed mutagenesis was performed to revert the amino acid at position 253 to the canonical lysine (K). Once the reversion was confirmed, site directed mutagenesis was performed to introduce the V736A mutation. All mutagenesis reactions were carried out using Agilent Technologies QuikChange II XL Site-Directed Mutagenesis Kit (Santa Clara, CA; catalog number 200521). All vectors were sequenced to confirm. The primer sequences used were: antisense (as) TMPRSS6 E253K GCATGAGGTCCTTGGGGCCCTGCAG (SEQ ID NO:22); sense (s) TMPRSS6 E253K CTGCAGGGCCCCAAGGACCTCATGC (SEQ ID NO:23); antisense (as) TMPRSS6 V736A CCTGGTAGCGATAGGCCTCGCTGCACAGG (SEQ ID NO:24); sense (s) TMPRSS6 V736A CCTGTGCAGCGAGGCCTATCGCTACCAGG (SEQ ID NO:25).

### 5.12.3.2. Results

Human iPS-CMs only minimally express matriptase-2 at baseline. Cells were transfected with a construct driving constitutive expression of matriptase-2 736A, encoded by the *TMPRSS6* rs855791SNP major allele, or matriptase-2 736V, encoded by the minor allele, mimicking homozygous major allele and homozygous minor allele cardiomyocytes, respectively.

Hepcidin expression is regulated by both the BMP6/SMAD and IL-6/STAT signaling pathways, with both BMP and IL-6 acting through their respective receptors to drive increased hepcidin expression. Casanovas et al., PLOS Comp. Biol. 10(1):e1003421 (2014). The major allele and minor allele iPS cardiomyocytes were treated *in vitro* with agonists of both signaling pathways - recombinant BMP2 and IL-6 - or with BMP2 alone to model clinical interventions in which IL-6 levels (or signaling) are reduced. Control iPS cells were not treated with either agonist. Cell mortality was measured under normal oxygen tension (normoxia), and also under conditions that simulate hypoxia followed by reoxygenation (reperfusion).

FIG. 6A shows the results when the cells were treated under normal oxygen levels. iPS cardiomyocytes expressing only the *TMPRSS6* rs855791 minor allele ("736V minor allele") are not significantly affected ("n.s.") by elimination of IL-6 signaling: cell mortality measured as percent Trypan Blue positive cells is insignificantly reduced when the cells are treated with BMP2 alone, as compared to treatment with BMP2+IL-6. In contrast, iPS cardiomyocytes expressing the *TMPRSS6* rs855791 major allele show statistically significantly lower cell death when IL-6 signaling is eliminated.

FIG. 6B shows the results when the cells were subjected to hypoxia followed by reoxygenation. As compared to normoxic conditions, hypoxia/reoxygenation is significantly toxic to the iPS cardiomyocytes, with about 40 percent of major and minor allele control cells killed, as compared to about 20% of the control cells under normoxic conditions (compare FIG. 6B to FIG. 6A). Against this increased background toxicity, minor allele iPS cardiomyocytes are not significantly affected by elimination of IL-6 signaling: cell mortality is insignificantly reduced when the cells are treated with BMP2 alone, as compared to treatment with BMP2+IL-6. In contrast, the iPS cardiomyocytes expressing the *TMPRSS6* rs855791 major allele show statistically significantly lower cell death when IL-6 signaling is eliminated.

### 5.12.3.3. Discussion

These data strengthen the inferences drawn from the post hoc analysis of clinical trial data in Example 1 and Example 2: reduction in IL-6 signaling can be effective to reduce IL-6 mediated toxicity in cardiomyocytes expressing the *TMPRSS6* rs855791 major allele, but not in cardiomyocytes expressing only the minor allele. Without intending to be bound by theory, the IL-6 driven increase in toxicity in the major allele iPS cardiomyocytes may result from IL-6 mediated increase in hepcidin expression, with consequent increased sequestration of iron in the cells, followed by iron-mediated cellular toxicity.

### 5.12.4. Example 4: Anti-IL-6 therapy is as effective as current standard of care in a model of cardiorenal syndrome in rats genotypically analogous to human TMPRSS6 rs855791 major allele homozygotes

Patients with chronic kidney disease, such as those enrolled in the MIMICK studies analyzed in Example 1, often develop impaired cardiac function, which is a major contributor to mortality rates. This secondary cardiac injury following primary chronic kidney disease is termed cardiorenal syndrome type 4 (CRS type 4).

To test whether anti-IL-6 therapy would be effective as a treatment in CRS4 patients having at least one copy of the *TMPRSS6* rs855791 major allele, as suggested by the data in Examples 1 and 3, we used a model of CRS4 in rats genotypically analogous to human beings homozygous for the *TMPRSS6* rs855791 major allele.

FIG. 7 outlines the study design.

At week 0, myocardial infarction was induced in the CRS animals. At week 2, nephrectomy was performed. A control group was subjected to sham operations instead. Prior to nephrectomy, various assessments of the subjects were conducted. The assessments included measurements of serum creatinine, glomerular filtration rate, 24 hour protein levels in urine, echocardiography, tail cuff blood pressure, and biomarkers in plasma and urine.

Treatment was commenced on day 1 after the nephrectomy. Animals were divided into three groups: (i) control treatment, (ii) anti-IL-6 therapy, and (iii) standard of care therapy. The anti-IL-6 therapy was an anti-IL-6 antibody suitable for use in rodents. The standard of care therapy was administration of perindopril, an ACE (angiotensin-converting enzyme) inhibitor. At the commencement of treatment, assessments of the subjects in all groups were conducted. The assessments included measurement of serum creatinine, glomerular filtration rate, 24 hour protein levels, and biomarkers in plasma.

At day 3 and day 7 after the nephrectomy, assessments of the subjects in all groups were conducted. The assessments included measurement of serum creatinine and biomarkers in plasma on day 3 and measurement of serum creatinine, glomerular filtration rate, 24 hour protein levels, echocardiography, blood pressure, and biomarkers in plasma on day 7.

At week 6, the subjects were sacrificed. Prior to sacrifice, various assessments of the subjects in all groups were conducted. The assessments included measurement of serum creatinine, glomerular filtration rate, 24 hour protein levels, blood pressure, biomarkers in plasma, echocardiography, and pressure-volume loop analysis. After sacrifice, tissue was also harvested from subjects in all groups for histology evaluation (i.e., Sirius red staining of cardiac tissue).

FIGS. 8A-8D shows the cardiac ejection fraction of rats without CRS ("Sham"), CRS animals treated with a pharmacologically irrelevant isotype-control antibody ("isotype"), CRS animals treated with anti-IL-6 antibody ("IL-6 ab"), and CRS animals treated with standard of care ACE inhibitor ("Peri") in the cardiorenal syndrome model summarized in FIG. 7.

FIG. 8A shows baseline ejection fraction levels for all groups two weeks after myocardial infarction, but before nephrectomy, and before treatment, demonstrating that the experimentally induced myocardial infarction caused a significant decrease in cardiac ejection fraction. Figure 8B is a plot showing ejection fraction levels for all groups one week after nephrectomy, after 1 week of treatment. Figure 8C is a plot showing ejection fraction levels for all groups two weeks after nephrectomy, after 2 weeks of treatment. FIG. 8D is a plot showing ejection fraction levels for all groups four weeks after nephrectomy, after 4 weeks of treatment. Results are expressed as mean +/- SEM.

After 4 weeks of treatment, both of the treatment groups - the group treated with anti-IL-6 and the group treated with standard of care ACE inhibitor therapy - showed statistically significantly increased ejection fraction levels compared to the isotype control group (FIG. 8D) (p<0.001). Similar ejection fraction levels in the anti-IL-6 and standard of care groups measured after week 4 of treatment showed that anti-IL-6 therapy had equivalent efficacy to the ACE inhibitor perindopril (standard of care therapy), demonstrating that anti-IL-6 therapy had therapeutic efficacy in preserving cardiac function in the cardiorenal syndrome model equivalent to standard of care therapy, as measured by changes in cardiac ejection fraction.

Measurement of cardiac contractility (FIG. 9) showed that anti-IL-6 therapy also had an effect equivalent to standard of care therapy with an ACE inhibitor. After 4 weeks of treatment, cardiac contractility in groups treated with anti-IL-6 and standard of care therapy were significantly increased over the cardiac contractility of the control, isotype group. Similar cardiac contractility in the anti-IL-6 and standard of care groups demonstrates that anti-IL-6 therapy had efficacy in preserving cardiac function in the cardiorenal syndrome model equivalent to the ACE inhibitor perindopril (standard of care therapy), as measured by contractility.

Measurement of fibrosis in heart tissue harvested from animals in all the groups also demonstrated that anti-IL-6 therapy had an equivalent effect as standard of care therapy (FIGS. 10A-10C). Fibrosis in heart tissue was quantified by measuring the percentage area of fibrotic tissue in two regions: the "Normal" region and "Fibrosis margin" region. An example "Normal" region is indicated by the delineated portion of the tissue slice shown in the micrograph in FIG. 10A. The inset in the micrograph shows a magnified view of the "Normal" region, showing that small portions of the "Normal" region has fibrotic tissue. The "Fibrosis Margin" region is a region of tissue in the "Normal" region peripheral to the fibrotic tissue.

Plots in FIGS. 10B and 10C show that heart tissue from subjects in groups treated with anti-IL-6 or standard of care therapy had significantly decreased percentage area of fibrotic tissue compared to the isotype control group, both when measured in the "Normal" region (FIG. 10B) or in the "Fibrosis margin" region (FIG. 10C). In addition, the percentage areas of fibrotic tissue measured in the anti-IL-6 and standard of care therapy groups were similar (both in the "Normal" region and "Fibrosis margin" region), indicating that anti-IL-6 had an equivalent anti-fibrotic effect as the ACE inhibitor perindopril (standard of care therapy).

These data demonstrate that treatment with an anti-IL-6 agent is effective to reduce cardiac injury and restore function in an *in vivo* model of cardiorenal syndrome in animals that are genotypically analogous to human beings homozygous for the *TMPRSS6* rs855791 major allele.

### 5.12.5. Example 5: Anti-IL-6 therapy is effective in preserving cardiac function in a model of acute myocardial infarction in mice genotypically analogous to human TMPRSS6 rs855791 major allele homozygotes

The data in Examples 2 and 3 suggested that reducing IL-6 levels or IL-6 signaling could reduce heart failure and mortality in patients with acute coronary syndrome, but only in those patients having at least one copy of the *TMPRSS6* rs855791 major allele, and with greatest effect in those patients with elevated serum levels of IL-6.

A rodent study was performed to determine the effect of anti-IL-6 therapy after acute myocardial infarction in mice genotypically analogous to human beings homozygous for the *TMPRSS6* rs855791 major allele.

FIGS. 11A and 11B show data from an *in vivo* model in which myocardial infarction was induced in mice genotypically analogous to human beings homozygous for the *TMPRSS6* rs855791 major allele. The control group received no therapy. The experimental group was treated with an anti-murine-IL-6 antibody. FIG. 11A shows that treatment with anti-IL-6 provides statistically significant improvement in ejection fraction. FIG. 11B shows that treatment with anti-IL-6 provides statistically significant improvement in contractility, measured as cardiac fractional shortening. The data demonstrate that anti-IL-6 therapy given immediately after myocardial infarction improves functional recovery of the left ventricle in rodents that are genotypically analogous to human patients having the *TMPRSS6* rs855791 major allele.

## Claims

1. An IL-6 antagonist for use in a method of treating hepcidin-mediated anemia of chronic inflammation in a patient, the method comprising administering a therapeutically effective amount of said IL-6 antagonist to said patient,
wherein the patient has been determined to have at least one copy of the *TMPRSS6* rs855791 major allele, optionally wherein this has been determined previously or wherein said method comprises an earlier step of determining whether said patient has at least one copy of the *TMPRSS6* rs855791 major allele; and wherein the IL-6 antagonist is:
(a) an anti-IL-6 antibody, or antigen-binding fragment or derivative thereof; or
(b) an anti-IL-6R antibody, or antigen-binding fragment or derivative thereof, optionally tocilizumab or vobarilizumab.

2. The IL-6 antagonist for use of claim 1, wherein the hepcidin-mediated anemia of chronic inflammation is an anemia of:
(a) chronic kidney disease (CKD), optionally KDOQI stage 1 chronic kidney disease, KDOQI stage 2 chronic kidney disease, KDOQI stage 3 chronic kidney disease, KDOQI stage 4 chronic kidney disease, or KDOQI stage 5 chronic kidney disease; optionally wherein the patient has cardiorenal syndrome (CRS) optionally of Type 4; and optionally has received at least one pre-treatment dialysis treatment;
(b) a chronic inflammatory disease, optionally rheumatoid arthritis (RA), juvenile idiopathic arthritis, ankylosing spondylitis, plaque psoriasis, psoriatic arthritis, inflammatory bowel disease, Crohn's disease, or ulcerative colitis;
(c) cancer, optionally solid tumors, small cell lung cancer, non-small cell lung cancer, hematological cancer, multiple myeloma, leukemias, chronic lymphocytic leukemia (CLL), chronic myeloid leukemia (CML), lymphomas, Hodgkin's lymphoma or hepatic adenoma;
(d) a chronic infection; or
(e) congestive heart failure (CHF).

3. The IL-6 antagonist for use of claim 2 wherein the patient is:
(a) male and has:
(i) pre-treatment hemoglobin (Hb) level of less than 14 g/dl, less than 13 g/dl, less than 12 g/dl, or less than 11 g/dl; and/or
(ii) a pre-treatment hematocrit of less than 40%, less than 35%, or of 30 - 34%;
(b) female and has:
(i) a pre-treatment Hb level of less than 12 g/dl, less than 11 g/dl, less than 10 g/dl, or less than 9 g/dl; and/or
(ii) a pre-treatment hematocrit of less than 36%, less than 30%, or of 26 - 29%.

4. The IL-6 antagonist for use of any one of the preceding claims, wherein:
the patient has elevated pre-treatment serum levels of IL-6, optionally wherein said pre-treatment level of IL-6 is greater than 2.5 pg/ml, 5 pg/ml, 7.5 pg/ml, 10 pg/ml or 12.5 pg/ml; and/or
the patient has elevated pre-treatment serum levels of CRP, optionally wherein said pre-treatment level of CRP is greater than 2 mg/L, 3 mg/L, 5 mg/L, 7.5 mg/L or 10 mg/L.

5. The IL-6 antagonist for use of any one of the preceding claims, wherein the patient has been determined to have at least one copy of the *TMPRSS6* rs855791 major allele using a real-time PCR assay.

6. The IL-6 antagonist for use of any one of the preceding claims, wherein the patient has received at least one pre-treatment administration of an erythropoiesis stimulating agent (ESA), an iron supplement, or a transfusion of blood or packed red blood cells, and optionally has a normal Hb level or normal hematocrit.

7. The IL-6 antagonist for use of any one of the preceding claims, wherein the IL-6 antagonist is administered at a dose, on a schedule, and for a period sufficient to:
(a) increase the patient's Hb levels or hematocrit above pre-treatment levels; and/or
(b) allow reduction in the patient's dose of ESA without reduction in the patient's Hb levels or hematocrit below levels present immediately pre-treatment, optionally wherein said reduction in the patient's dose of ESA as compared to pre-treatment ESA dose is of at least 10%, 20% or 50%; and/or
(c) reverse functional iron deficiency; and/or
(d) reduce cardiovascular (CV) mortality in patients with CKD as compared to age-matched and disease-matched historical controls; and/or
(e) reduce the free IL-6 levels in the patient's serum below pre-treatment levels, optionally by at least 10%, 20% or 50% compared to pre-treatment levels; and/or
(f) reduce the patient's CRP levels below pre-treatment levels, optionally, by at least 50% as compared to pre-treatment levels.

8. The IL-6 antagonist for use of any one of the preceding claims, wherein the anti-IL-6 antibody or antigen-binding fragment or derivative:
(a) has a K_{D} for binding human IL-6 of less than 100 nM, 50 nM, 10 nM or 1 nM; and/or
(b) has an elimination half-life following intravenous administration of at least 7 days, 14 days, 21 days or 30 days.

9. The IL-6 antagonist for use of any one of the preceding claims, wherein the anti-IL-6 antibody or antigen-binding fragment or derivative:
is fully human or is humanized, and/or is a full-length monoclonal anti-IL-6 antibody, optionally an IgG1 or IgG4 antibody; or
is a single domain antibody, a VHH Nanobody, an Fab, or a scFv.

10. The IL-6 antagonist for use of any one of the preceding claims, wherein the anti-IL-6 antibody or antigen-binding fragment or derivative comprises all six variable region CDRs of MEDI5117, or comprises the VH and VL of MEDI5117, or wherein the antibody is MEDI5117.

11. The IL-6 antagonist for use of any one of claims 1 to 9, wherein the anti-IL-6 antibody or antigen-binding fragment or derivative comprises all six variable region CDRs, or comprises the heavy chain V region and light chain V region, of an antibody selected from the group consisting of siltuximab, gerilimzumab, sirukumab, clazakizumab, olokizumab, elsilimomab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

12. The IL-6 antagonist for use of claim 11, wherein the anti-IL-6 antibody or antigen-binding fragment or derivative is an antibody selected from the group consisting of siltuximab, gerilimzumab, sirukumab, clazakizumab, olokizumab, VX30 (VOP-R003; Vaccinex), EB-007 (EBI-029; Eleven Bio), ARGX-109 (ArGEN-X), FM101 (Femta Pharmaceuticals, Lonza) and ALD518/BMS-945429 (Alder Biopharmaceuticals, Bristol-Myers Squibb).

13. The IL-6 antagonist for use of any one of the preceding claims, wherein the antibody or antigen-binding fragment or derivative is administered parenterally, optionally subcutaneously or intravenously.

14. A method for determining whether an IL-6 antagonist, as defined in claim 1, will be ineffective in a patient with hepcidin-mediated anemia of chronic inflammation, the method comprising determining in a biological sample from the patient whether or not the patient is homozygous for the *TMPRSS6* rs855791 minor allele, optionally using a real-time PCR assay.

15. The method according to claim 14, further comprising the step of determining to discontinue administration of an IL-6 antagonist to a patient who is determined to be homozygous.

## Patentansprüche

1. IIL-6-Antagonist für die Verwendung in einem Verfahren zum Behandeln von Hepcidin-vermittelter Anämie bei chronischer Entzündung bei einem Patienten, wobei das Verfahren das Verabreichen einer therapeutisch wirksamen Menge des IL-6-Antagonisten an den Patienten umfasst,
wobei bei dem Patienten bestimmt wurde, dass er mindestens eine Kopie des *TMPRSS6* rs855791-Hauptallels aufweist, optional wobei dies zuvor bestimmt worden ist oder wobei das Verfahren einen früheren Schritt des Bestimmens umfasst, ob der Patient mindestens eine Kopie des *TMPRSS6* rs855791-Hauptallel besitzt; und wobei der IL-6-Antagonist ist:
(a) ein Anti-IL-6-Antikörper, oder ein Antigen-bindendes Fragment oder Derivat davon; oder
(b) ein Anti-IL-6R-Antikörper, oder ein Antigen-bindendes Fragment oder Derivat davon, optional Tocilizumab oder Vobarilizumab.

2. IL-6-Antagonist für die Verwendung nach Anspruch 1, wobei die Hepcidin-vermittelte Anämie der chronischen Entzündung eine Anämie ist von:
(a) einer chronischen Nierenerkrankung (CKD), optional einer chronischen Nierenerkrankung im KDOQI-Stadium 1, einer chronischen Nierenerkrankung im KDOQI-Stadium 2, einer chronischen Nierenerkrankung im KDOQI-Stadium 3, einer chronischen Nierenerkrankung im KDOQI-Stadium 4 oder einer chronischen Nierenerkrankung im KDOQI-Stadium 5; optional wobei der Patient ein kardiorenales Syndrom (CRS), optional des Typs 4, aufweist; und optional mindestens eine Dialysebehandlung vor der Behandlung erhalten hat;
(b) einer chronischen entzündlichen Erkrankung, optional rheumatoider Arthritis (RA), juveniler idiopathischer Arthritis, Spondylitis ankylosans, Plaque-Psoriasis, Psoriasis-Arthritis, entzündlicher Darmerkrankung, Morbus Crohn oder Colitis ulcerosa;
(c) Krebs, optional soliden Tumoren, kleinzelligem Lungenkrebs, nichtkleinzelligem Lungenkrebs, hämatologischem Krebs, multiplem Myelom, Leukämien, chronischer lymphatischer Leukämie (CLL), chronischer myeloischer Leukämie (CML), Lymphomen, Hodgkin-Lymphom oder hepatischem Adenom;
(d) einer chronischen Infektion; oder
(e) kongestiver Herzinsuffizienz (CHF).

3. IL-6-Antagonist für die Verwendung nach Anspruch 2, wobei der Patient ist:
(a) männlich und aufweist:
(i) einen Hämoglobin- (Hb-) Spiegel vor der Behandlung von weniger als 14 g/dl, weniger als 13 g/dl, weniger als 12 g/dl oder weniger als 11 g/dl; und/oder
(ii) einen Hämatokrit vor der Behandlung von weniger als 40 %, weniger als 35% oder von 30 bis 34 %;
(b) weiblich und aufweist:
(i) einen Hb-Spiegel vor der Behandlung von weniger als 12 g/dl, weniger als 11 g/dl, weniger als 10 g/dl oder weniger als 9 g/dl; und/oder
(ii) einen Hämatokrit vor der Behandlung von weniger als 36 %, weniger als 30 % oder von 26 bis 29 %.

4. IL-6-Antagonist für die Verwendung nach einem der vorhergehenden Ansprüche, wobei:
der Patient vor der Behandlung erhöhte Serumspiegel von IL-6 aufweist, optional wobei der Spiegel von IL-6 vor der Behandlung größer als 2,5 pg/ml, 5 pg/ml, 7,5 pg/ml, 10 pg/ml oder 12,5 pg/ml ist; und/oder
der Patient erhöhte Serumspiegel von CRP vor der Behandlung aufweist, wobei der Spiegel von CRP vor der Behandlung größer als 2 mg/L, 3 mg/L, 5 mg/L, 7,5 mg/L oder 10 mg/L ist.

5. IL-6-Antagonist für die Verwendung nach einem der vorhergehenden Ansprüche, wobei unter Verwendung eines Echtzeit-PCR-Assays bestimmt wurde, dass der Patient mindestens eine Kopie des *TMPRSS6* rs855791-Hauptallels aufweist.

6. IL-6-Antagonist für die Verwendung nach einem der vorhergehenden Ansprüche, wobei der Patient vor der Behandlung mindestens eine Verabreichung eines Erythropoese-stimulierenden Mittels (ESA), eines Eisenpräparats oder einer Transfusion von Blut oder gepackten roten Blutkörperchen erhalten hat, und optional einen normalen Hb-Spiegel oder einen normalen Hämatokrit aufweist.

7. IL-6-Antagonist für die Verwendung nach einem der vorhergehenden Ansprüche, wobei der IL-6-Antagonist in einer Dosis, nach einem Zeitplan und für eine Dauer verabreicht wird, die ausreicht, um:
(a) die Hb-Spiegel oder den Hämatokrit des Patienten über die Spiegel vor der Behandlung zu erhöhen; und/oder
(b) eine Reduktion der ESA-Dosis des Patienten zu ermöglichen, ohne dass der Hb-Spiegel oder der Hämatokrit des Patienten unter die Werte sinkt, die unmittelbar vor der Behandlung vorhanden waren, optional wobei die Reduktion der ESA-Dosis des Patienten im Vergleich zu der ESA-Dosis vor der Behandlung mindestens 10 %, 20 % oder 50 % beträgt; und/oder
(c) einen funktionellen Eisenmangel umzukehren; und/oder
(d) die kardiovaskuläre (CV) Sterblichkeit bei Patienten mit CKD im Vergleich zu alters- und krankheitsangepassten historischen Kontrollen zu reduzieren; und/oder
(e) die freien IL-6-Spiegel in dem Serum des Patienten unter die Spiegel vor der Behandlung zu reduzieren, optional um mindestens 10 %, 20 % oder 50 % im Vergleich zu den Spiegeln vor der Behandlung; und/oder
(f) die CRP-Spiegel des Patienten unter die Werte vor der Behandlung zu reduzieren, optional um mindestens 50 % im Vergleich zu den Spiegeln vor der Behandlung.

8. IL-6-Antagonist für die Verwendung nach einem der vorhergehenden Ansprüche, wobei der Anti-IL-6-Antikörper oder das Antigen-bindende Fragment oder Derivat:
(a) einen K_{D} für die Bindung von humanem IL-6 von weniger als 100 nM, 50 nM, 10 nM oder 1 nM aufweist; und/oder
(b) eine Eliminationshalbwertszeit nach intravenöser Verabreichung von mindestens 7 Tagen, 14 Tagen, 21 Tagen oder 30 Tagen aufweist.

9. IL-6-Antagonist für die Verwendung nach einem der vorhergehenden Ansprüche, wobei der Anti-IL-6-Antikörper oder das Antigen-bindende Fragment oder Derivat:
vollständig human ist oder humanisiert ist und/oder ein monoklonaler Anti-IL-6-Antikörper in voller Länge ist, optional ein IgG1- oder IgG4-Antikörper; oder
ein Einzeldomänenantikörper, ein VHH-Nanokörper, ein Fab oder ein scFv ist.

10. IL-6-Antagonist für die Verwendung nach einem der vorhergehenden Ansprüche, wobei der Anti-IL-6-Antikörper oder das Antigen-bindende Fragment oder Derivat alle sechs CDRs der variablen Region von MEDI5117 umfasst, oder die VH und VL von MEDI5117 umfasst, oder wobei der Antikörper MEDI5117 ist.

11. IL-6-Antagonist für die Verwendung nach einem der Ansprüche 1 bis 9, wobei der Anti-IL-6-Antikörper oder das Antigen-bindende Fragment oder Derivat alle sechs CDRs der variablen Region umfasst, oder die V-Region der schweren Kette und die V-Region der leichten Kette eines Antikörpers, ausgewählt aus der Gruppe bestehend aus Siltuximab, Gerilimzumab, Sirukumab, Clazakizumab, Olokizumab, Elsilimomab, VX30 (VOP-R003; Vaccinex), EB-007 (EBI-029; Eleven Bio), ARGX-109 (ArGEN-X), FM101 (Femta Pharmaceuticals, Lonza) und ALD518/BMS-945429 (Alder Biopharmaceuticals, Bristol-Myers Squibb) umfasst.

12. IL-6-Antagonist für die Verwendung nach Anspruch 11, wobei der Anti-IL-6-Antikörper oder das Antigen-bindende Fragment oder Derivat ein Antikörper ist, ausgewählt aus der Gruppe bestehend aus Siltuximab, Gerilimzumab, Sirukumab, Clazakizumab, Olokizumab, VX30 (VOP-R003; Vaccinex), EB-007 (EBI-029; Eleven Bio), ARGX-109 (ArGEN-X), FM101 (Femta Pharmaceuticals, Lonza) und ALD518/BMS-945429 (Alder Biopharmaceuticals, Bristol-Myers Squibb).

13. IL-6-Antagonist für die Verwendung nach einem der vorhergehenden Ansprüche, wobei der Antikörper oder das Antigen-bindende Fragment oder Derivat parenteral verabreicht wird, optional subkutan oder intravenös.

14. Verfahren für die Bestimmung, ob ein IL-6-Antagonist, wie in Anspruch 1 definiert, bei einem Patienten mit Hepcidin-vermittelter Anämie einer chronischen Entzündung unwirksam ist, wobei das Verfahren das Bestimmen in einer biologischen Probe des Patienten umfasst, ob der Patient für das untergeordnete *TMPRSS6* rs855791-Allel homozygot ist, optional unter Verwendung eines Echtzeit-PCR-Assays.

15. Verfahren nach Anspruch 14, umfassend ferner den Schritt des Bestimmens, die der Verabreichung eines IL-6-Antagonisten an einen Patienten zu unterbrechen, bei dem bestimmt wird, dass er homozygot ist.

## Revendications

1. Antagoniste d'IL-6 pour l'utilisation dans un procédé de traitement d'anémie à médiation par hepcidine d'inflammation chronique chez un patient, le procédé comprenant l'administration d'une quantité thérapeutiquement efficace dudit antagoniste d'IL-6 audit patient,
dans lequel il a été déterminé que le patient a au moins une copie de l'allèle majeur rs855791 de *TMPRSS6*, optionnellement dans lequel ceci a été déterminé auparavant ou dans lequel ledit procédé comprend une étape précédente de la détermination du fait que ledit patient a ou n'a pas au moins une copie de l'allèle majeur rs855791 de *TMPRSS6* ; et dans lequel l'antagoniste d'IL-6 est :
(a) un anticorps anti-IL-6, ou fragment de liaison à l'antigène ou dérivé de celui-ci ; ou
(b) un anticorps anti-IL-6R, ou fragment de liaison à l'antigène ou dérivé de celui-ci, optionnellement tocilizumab ou vobarilizumab.

2. Antagoniste d'IL-6 pour l'utilisation selon la revendication 1, dans lequel l'anémie à médiation par hepcidine d'inflammation chronique est une anémie de :
(a) maladie rénale chronique (MRC), optionnellement maladie rénale chronique de stade 1 KDOQI, maladie rénale chronique de stade 2 KDOQI, maladie rénale chronique de stade 3 KDOQI, maladie rénale chronique de stade 4 KDOQI, ou maladie rénale chronique de stade 5 KDOQI ; optionnellement dans lequel le patient présente un syndrome cardio-rénal (SCR) optionnellement de type 4 ; et optionnellement a reçu au moins un traitement par dialyse en prétraitement ;
(b) une maladie inflammatoire chronique, optionnellement arthrite rhumatoïde (RA), arthrite idiopathique juvénile, spondylite ankylosante, psoriasis en plaques, arthrite psoriasique, maladie intestinale inflammatoire, maladie de Crohn, ou colite ulcérative ;
(c) cancer, optionnellement tumeurs solides, cancer du poumon à petites cellules, cancer du poumon non à petites cellules, cancer hématologique, myélome multiple, leucémies, leucémie lymphoïde chronique (LLC), leucémie myéloïde chronique (LMC), lymphomes, lymphome de Hodgkin ou adénome hépatique ;
(d) une infection chronique ; ou
(e) insuffisance cardiaque congestive (ICC).

3. Antagoniste d'IL-6 pour l'utilisation selon la revendication 2, dans lequel le patient est :
(a) mâle et a :
(i) un niveau d'hémoglobine (Hb) en prétraitement inférieur à 14 g/dl, inférieur à 13 g/dl, inférieur à 12 g/dl, ou inférieur à 11 g/dl ; et/ou
(ii) un hématocrite en prétraitement inférieur à 40 %, inférieur à 35 %, ou de 30 à 34 % ;
(b) femelle et a :
(i) un niveau de Hb en prétraitement inférieur à 12 g/dl, inférieur à 11 g/dl, inférieur à 10 g/dl, ou inférieur à 9 g/dl ; et/ou
(ii) un hématocrite en prétraitement inférieur à 36 %, inférieur à 30 %, ou de 26 à 29 %.

4. Antagoniste d'IL-6 pour l'utilisation selon l'une quelconque des revendications précédentes, dans lequel :
le patient a des niveaux d'IL-6 sériques élevés en prétraitement, optionnellement dans lequel ledit niveau d'IL-6 en prétraitement est supérieur à 2,5 pg/ml, 5 pg/ml, 7,5 pg/ml, 10 pg/ml ou 12,5 pg/ml ; et/ou
le patient a des niveaux de CRP sériques élevés en prétraitement, optionnellement dans lequel ledit niveau de CRP en prétraitement est supérieur à 2 mg/L, 3 mg/L, 5 mg/L, 7,5 mg/L ou 10 mg/L.

5. Antagoniste d'IL-6 pour l'utilisation selon l'une quelconque des revendications précédentes, dans lequel il a été déterminé que le patient a au moins un copie de l'allèle majeur rs855791 de TMPRSS6 en utilisant une analyse PCR en temps réel.

6. Antagoniste d'IL-6 pour l'utilisation selon l'une quelconque des revendications précédentes, dans lequel le patient a reçu au moins une administration, en prétraitement, d'un agent stimulant l'érythropoïèse (ESA), d'un complément en fer, ou une transfusion de sang ou de concentré de globules rouges, et optionnellement a un niveau de Hb normal ou un hématocrite normal.

7. Antagoniste d'IL-6 pour l'utilisation selon l'une quelconque des revendications précédentes, dans lequel l'antagoniste d'IL-6 est administré, à une dose, selon un programme, et pendant une période suffisante pour :
(a) augmenter les niveaux de Hb ou l'hématocrite du patient jusqu'à des valeurs supérieures aux niveaux en prétraitement ; et/ou
(b) permettre la réduction de la dose d'ESA du patient sans réduction des niveaux de Hb ou de l'hématocrite du patient jusqu'à des valeurs inférieures à des niveaux présents immédiatement prétraitement, optionnellement dans lequel ladite réduction de la dose d'ESA du patient par rapport à la dose d'ESA en prétraitement est d'au moins 10 %, 20 % ou 50 % ; et/ou
(c) inverser la carence martiale fonctionnelle ; et/ou
(d) réduire la mortalité cardiovasculaire (CV) chez des patients souffrant de MRC par rapport à des témoins historiques appariés en âge et appariés en maladie ; et/ou
(e) réduire les niveaux d'IL-6 libres dans le sérum du patient jusqu'à des valeurs inférieures à des niveaux en prétraitement, optionnellement d'au moins 10 %, 20 % ou 50 % par rapport à des niveaux en prétraitement ; et/ou
(f) réduire les niveaux de CRP du patient jusqu'à des valeurs inférieures à des niveaux en prétraitement, optionnellement, d'au moins 50 % par rapport à des niveaux en prétraitement.

8. Antagoniste d'IL-6 pour l'utilisation selon l'une quelconque des revendications précédentes, dans lequel l'anticorps anti-IL-6 ou le fragment de liaison à l'antigène ou le dérivé :
(a) a une valeur K_{D} pour la liaison d'IL-6 humaine inférieure à 100 nM, 50 nM, 10 nM ou 1 nM ; et/ou
(b) a une demi-vie d'élimination suivant l'administration intraveineuse d'au moins 7 jours, 14 jours, 21 jours ou 30 jours.

9. Antagoniste d'IL-6 pour l'utilisation selon l'une quelconque des revendications précédentes, dans lequel l'anticorps anti-IL-6 ou le fragment de liaison à l'antigène ou le dérivé :
est entièrement humain ou est humanisé, et/ou est un anticorps anti-IL-6 monoclonal entier, optionnellement un anticorps IgG1 ou IgG4 ; ou
est un anticorps à domaine unique, un nanocorps VHH, un Fab, ou un scFv.

10. Antagoniste d'IL-6 pour l'utilisation selon l'une quelconque des revendications précédentes, dans lequel l'anticorps anti-IL-6 ou le fragment de liaison à l'antigène ou le dérivé comprend toutes les six CDRs de région variable de MEDI5117, ou comprend les VH et VL de MEDI5117, ou dans lequel l'anticorps est MEDI5117.

11. Antagoniste d'IL-6 pour l'utilisation selon l'une quelconque des revendications 1 à 9, dans lequel l'anticorps anti-IL-6 ou le fragment de liaison à l'antigène ou le dérivé comprend toutes les six CDRs de région variable, ou comprend la région V de chaîne lourde et la région V de chaîne légère, d'un anticorps sélectionné parmi le groupe constitué de : siltuximab, gerilimzumab, sirukumab, clazakizumab, olokizumab, elsilimomab, VX30 (VOP-R003 ; Vaccinex), EB-007 (EBI-029 ; Eleven Bio), ARGX-109 (ArGEN-X), FM101 (Femta Pharmaceuticals, Lonza) et ALD518/BMS-945429 (Alder Biopharmaceuticals, Bristol-Myers Squibb).

12. Antagoniste d'IL-6 pour l'utilisation selon la revendication 11, dans lequel l'anticorps anti-IL-6 ou le fragment de liaison à l'antigène ou le dérivé est un anticorps sélectionné parmi le groupe constitué de : siltuximab, gerilimzumab, sirukumab, clazakizumab, olokizumab, VX30 (VOP-R003 ; Vaccinex), EB-007 (EBI-029 ; Eleven Bio), ARGX-109 (ArGEN-X), FM101 (Femta Pharmaceuticals, Lonza) et ALD518/BMS-945429 (Alder Biopharmaceuticals, Bristol-Myers Squibb).

13. Antagoniste d'IL-6 pour l'utilisation selon l'une quelconque des revendications précédentes, dans lequel l'anticorps ou le fragment de liaison à l'antigène ou le dérivé est administré de façon parentérale, optionnellement de façon sous-cutanée ou intraveineuse.

14. Procédé pour la détermination du fait qu'un antagoniste d'IL-6, selon la revendication 1, sera ou ne sera pas inefficace chez un patient souffrant d'anémie à médiation par hepcidine d'inflammation chronique, le procédé comprenant la détermination, dans un échantillon biologique du patient, du fait que le patient est ou n'est pas homozygote pour l'allèle mineur rs855791 de *TMPRSS6,* optionnellement en utilisant une analyse PCR en temps réel.

15. Procédé selon la revendication 14, comprenant en outre l'étape de la détermination de discontinuer l'administration d'un antagoniste d'IL-6 à un patient déterminé comme étant homozygote.
